# EUROPEAN PATENT SPECIFICATION

(11) **EP 2 419 520 B1**
(45) Date of publication and mention of the grant of the patent: **26.06.2019**
(21) Application number: 10765129.1
(22) Date of filing: 14.04.2010
(51) Int. Cl.: C12P 7/64, C12N 1/00

(54) **METHODS OF MICROBIAL OIL EXTRACTION AND SEPARATION**
VERFAHREN ZUR EXTRAKTION UND ABSCHEIDUNG VON MIKROBIELLEM ÖL
PROCÉDÉS D'EXTRACTION ET DE SÉPARATION DE LIPIDES MICROBIENS

(30) Priority: 14.04.2009 US 169271 P; 14.10.2009 WO PCT/US2009/060692; 30.11.2009 WO PCT/US2009/066142; 30.11.2009 WO PCT/US2009/066141; 28.01.2010 US 299250 P
(43) Date of publication of application: 22.02.2012
(73) Proprietor: Corbion Biotech, Inc., South San Francisco, CA 94080 (US)
(72) Inventor: WITTENBERG, Jon, South San Francisco California 94080 (US); ARANA, Felipe, South San Francisco California 94080 (US)
(74) Representative: Marshall, Cameron John
(86) International application number: PCT/US2010/031108
(87) International publication number: WO 2010/120939

(56) References cited:
- WO-A1-91/11918
- WO-A1-03/049832
- WO-A2-97/37032
- WO-A2-2006/046943
- WO-A2-2006/047445
- US-A1- 2004 049 062
- US-A1- 2006 122 410
- US-A1- 2008 179 434
- US-A1- 2008 233 175
- US-A1- 2009 061 493
- US-B1- 6 176 176
- ZA-A- 9 702 702
- DATABASE GENBANK [Online] 21 November 2001 TURMEL ET AL., XP008141931 Database accession no. L42851
- DATABASE GENEBANK [Online] 11 July 2001 CASAREGOLA ET AL., XP008150667 Database accession no. AL411154

## Description

### REFERENCE TO A SEQUENCE LISTING

This application includes a sequence listing as shown in pages 1-21, appended hereto.

### FIELD OF INVENTION

This invention generally relates to the production and extraction of oil from microorganisms. In particular, the invention provides methods for extracting, recovering, isolating, and obtaining oil from a microorganism and compositions comprising the oil. The invention accordingly relates to the fields of biology, microbiology, fermentation technology and oil and fuel production technology.

### BACKGROUND OF THE INVENTION

Fossil fuel is a general term for combustible geologic deposits of organic materials formed from decayed plants and animals that have been converted to crude oil, coal, natural gas, or heavy oils by exposure to heat and pressure in the earth's crust over hundreds of millions of years.

Fossil fuels are a finite, non-renewable resource. With global modernization in the 20^{th} and 21^{st} centuries, the demand for energy from fossil fuels, especially gasoline derived from oil, is growing and has been the cause of major regional and global conflicts. Increased demand for energy has also increased the cost of hydrocarbon fuels. Aside from energy, many industries, including the plastics and chemical manufacturing industries, are dependent on the availability of hydrocarbons as a feedstock for manufacturing. Alternatives to current sources of supply would help mitigate the upward pressure on these raw material costs.

Lipids for use in biofuels can be produced in microorganisms, such as algae, fungi, and bacteria. Typically, manufacturing a lipid in a microorganism involves growing microorganisms, such as algae, fungi, or bacteria, which are capable of producing a desired lipid in a fermentor or bioreactor, isolating the microbial biomass, drying it, and extracting the intracellular lipids, which are a form of oil. However, these processes are generally considered to be inefficient and expensive, particularly when one considers the scale on which they must be conducted to produce meaningful supplies of fuel. One significant problem with these processes is the extraction of the lipid or oil from a microorganism.

WO2006/046943 A2 discloses methods of preparing polyunsaturated fatty acid-containing lipids from a lipid-containing material that include enzymatic treatment of components of the material and/or pressure disruption of the material. The lipid-containing materials include biomass, such as microorganisms.

There is a need for a process for extracting oil from microorganisms that mitigates the problems of low efficiency and high cost of current methods for lipid extraction from microorganisms. The present invention provides such a process.

### SUMMARY OF THE INVENTION

The present invention is as described in the appended claims. The present invention provides a method for extracting lipids from microbial biomass comprising
a. drying the microbial biomass to produce dried microbial biomass having a moisture content of from 3% to 15%, less than 6%, less than 5%, less than 4%, from 0.1% to 5%, from 0.1% to 3%, or from 0.5% to 3.5% by weight and constituting at least 20% oil by weight;
b. conditioning the dried microbial biomass to produce conditioned feedstock by heating the dry microbial biomass to a temperature in the range of 70°C to 150 °C, the conditioned feedstock having a moisture content of from 0.5% to 2.5% by weight of the microbial biomass; and
c. subjecting the conditioned feedstock to pressure sufficient to separate at least 5% of the oil in the biomass from other components, leaving spent biomass of reduced oil content relative to the conditioned feedstock.

The present invention provides a method for extracting lipids/oil from microbial biomass. In one embodiment, the present invention provides a method for extracting oil from microbial biomass, said method comprising the steps of subjecting dry microbial biomass having a moisture content of less than 6% by weight and constituting at least 20% oil by weight and heat conditioned to a temperature in the range of 70°C to 150 °C (160°F to 300°F) to pressure sufficient to extract more than 5% of the oil by weight from the biomass so that extracted oil and spent biomass of reduced oil content is produced. In various embodiments, more than 75% of the oil by weight in the dry microbial biomass is extracted from the biomass in the pressing step.

Thus, this method comprises drying and then conditioning the microbial biomass to produce conditioned feedstock that is then subjected to pressure. Conditioning changes the physical and/or physiochemical properties of the biomass but does not cause the release of more than 5% of the oil in the biomass. The conditioning step comprises heating the dry microbial biomass to a temperature in the range of 70°C to 150 °C (160°F to 300°F), thereby altering its moisture content. In various embodiments, a "bulking agent" or "press-aid" is added either to the microbial biomass or to conditioned feedstock prior to the application of pressure during the pressing step. During the pressing step, the conditioned feedstock is subjected to pressure sufficient to separate at least 5% of the oil in the biomass or conditioned feedstock from other components. These other components are contained in the "spent biomass", which may include residual oil but in any event has reduced oil content relative to the conditioned feedstock. In one embodiment, the pressure is exerted by an expeller press.

In various embodiments of this and other aspects of the invention, the biomass is prepared by fermentation of a microbe selected from the group consisting of microalgae, oleaginous bacteria, oleaginous yeast, and fungi. In various embodiments, the microalgae is a species of a genus selected from Chlorella, Parachlorella, or Prototheca, or is one of the other species in Table 1, below. In various embodiments, the oleaginous bacteria is a species of the genus Rhodococcus. In various embodiments, the oleaginous yeast is Rhodotorula glutinis or another species listed in Table 2, below. In various embodiments, the fungi is a species listed in Table 3, below.

In various embodiments of this and other aspects of the invention, the biomass is prepared by fermentation of a microbe that contains 18:1 fatty acid. In various embodiments, the microbe has a fatty acid profile of less than 2% C14:0; about 13-16% C16:0; about 1-4% C18:0; about 64-71% C18:1; about 10-15% C18:2; about 0.5-2% w/w C18:3; and less than 1% carbon chain length 20 or longer. In various embodiments, the microbe has a fatty acid profile of about 1-2% C14:0; about 20% C16:0; about 4% C18:0; about 64% C18:1; and about 7-8% C18:2. In some embodiments, the microbe has a fatty acid profile of about C14:0 (1.65); C16:0 (28.0); C18:0 (2.90); C18:1 (53.80); C18:2 (10.95); and C18:3alpha (0.80). In other embodiments, the microbe has a fatty acid profile of C14:0 (2.33); C15:0 (9.08); C16:0 (24.56); C16:1 (11.07); C17:0 (10.50); C18:0 (2.49); C18:1 (17.41); C18:2 (0.05). In still other embodiments, the microbe has a fatty acid profile of C12 (less than 1%); C14:0 (2.18-3.36); C15:0 (0.12-0.25); C16:0 (29.94-33.26); C16:1 (0.49-0.76); C17:0; C18:0 (6.88-8.17); C18:1 (42.68-48.12); C18:2 (7.88-9.28) C18:3 alpha (0.84-1.33); and greater than C:20 (1.1-1.45). In various embodiments, the microbe has less than 0.5% DHA. In these and other embodiments, the microbe is, in some instances, a microalgae.

In various embodiments of this and other aspects of the invention, the microbial biomass (dry or hydrated) or conditioned feedstock contains at least 25% oil (lipids) by weight. In various embodiments, the dry microbial biomass or conditioned feedstock contains at least 25% oil by dry cell weight. In various embodiments, the dry microbial biomass or conditioned feedstock contains at least 40%, at least 50%, or at least 75% oil by dry cell weight. In various embodiments, the dry microbial biomass or conditioned feedstock contains at least 15% carbohydrate by dry cell weight.

In various embodiments of this and other aspects of the invention, the conditioning step involves the application of heat and/or pressure to the biomass. In various embodiments, the conditioning step comprises heating the biomass at a temperature in the range of 70°C to 150 °C (160°F to 300°F). In various embodiments, the heating is performed using a vertical stacked shaker. In various embodiments, the conditioning step comprises treating the dry biomass with an expander or extruder to shape and/or homogenize the biomass. In various embodiments, the dry biomass has a moisture content of less than 5% by weight. In various embodiments, the dry biomass has a moisture content in the range of 0.1% and 5% by weight. In various embodiments, the dry biomass has a moisture content of less than 4% by weight. In various embodiments, the dry biomass has a moisture content in the range of 0.5% and 3.5% by weight. In various embodiments, the dry biomass has a moisture content in the range of 0.1% and 3% by weight.

In various embodiments of this and other aspects of the invention, a bulking agent is added to the microbial biomass, which may be either dry or hydrated (i.e., biomass that has not been dried or that contains significant, i.e., more than 6% by weight, moisture, including biomass in fermentation broth that has not been subjected to any process to remove or separate water) microbial biomass or conditioned feedstock prior to the pressing step. In various embodiments, the bulking agent has an average particle size of less than 1.5 mm. In various embodiments, the bulking agent is selected from the group consisting of cellulose, corn stover, dried rosemary, soybean hulls, spent biomass (biomass of reduced lipid content relative to the biomass from which it was prepared), sugar cane bagasse, and switchgrass. In various embodiments, the bulking agent is spent biomass that contains between 50% and 80% polysaccharide by weight and/or less than 10% oil by weight. In various embodiments, the polysaccharide in the spent biomass used as a bulking agent contains 20-30 mole percent galactose, 55-65 mole percent glucose, and/or 5-15 mole percent mannose.

The invention provides various methods relating to the extraction of oil from microbial biomass that employ the bulking agents described above. In one method, hydrated microbial biomass suitable for oil extraction is prepared by adding a bulking agent to the biomass and drying the mixture obtained thereby to a moisture content less than 6% by weight, thereby forming a dried bulking agent/biomass mixture. In another method, oil is extracted from microbial biomass by co-drying hydrated microbial biomass containing at least 20% oil by weight and a bulking agent to form a dried bulking agent/biomass mixture; reducing the moisture content in the mixture to less than 4% by weight; and pressing the reduced moisture content mixture to extract oil therefrom, thereby forming spent biomass of reduced lipid content. In another method, increased yields of oil are obtained from microbial biomass containing at least 20% lipid by weight by co-drying the microbial biomass with a bulking agent, because the co-dried mixture will, upon pressing, release more oil than can be obtained from the biomass under the same conditions in the absence of a bulking agent. In various embodiments of these and other methods of the invention, the hydrated microbial biomass is contained in fermentation broth that has not been subjected to processes to separate or remove water from the biomass prior to adding the bulking agent to the biomass. Typically, the admixture of bulking agent and biomass is conditioned by heating to a temperature in the range of 70°C to 150 °C (160°F to 300°F) immediately prior to the pressing step.

In various embodiments of the different aspects of the invention, dry microbial biomass, hydrated microbial biomass admixed with a bulking agent, or conditioned feedstock, optionally comprising a bulking agent, is subjected to pressure in a pressing step to extract oil, producing oil separated from the spent biomass. The pressing step involves subjecting pressure sufficient to extract oil from the conditioned feedstock. Cell lysis will occur during this step, if the biomass or feedstock has not been subjected to conditions that lyse some or all of the cells prior to the pressing step. In various embodiments of the different aspects of the invention, the pressing step will involve subjecting the conditioned feedstock to at least 689 bar (10,000 psi) of pressure. In various embodiments, the pressing step involves the application of pressure for a first period of time, a reduction in pressure for a second period of time, and then application of a pressure higher than during the first period of time for a third period of time. This process may be repeated one or more times ("oscillating pressure"). In various embodiments, more than 5 cycles of oscillating pressure are applied. In various embodiments, one or more of the subsequent cycles may exert an average pressure that is higher than the average pressure exerted in one or more earlier cycles. For example and without limitation, the average pressure in the last cycle can be at least 2-fold higher than the average pressure in the first or any earlier cycle. In various embodiments, moisture
content of the conditioned feedstock is controlled during the pressing step.

In various embodiments, the pressing step is conducted with an expeller press. In various embodiments, the pressing step is conducted in a continuous flow mode. In various embodiments, the oiling rate is at least 500 g/min. to no more than 1000 g/min. In various continuous flow embodiments, the expeller press is a device comprising a continuously rotating worm shaft within a cage having a feeder at one end and a choke at the opposite end, having openings within the cage is utilized. The conditioned feedstock enters the cage through the feeder, and rotation of the worm shaft advances the feedstock along the cage and applies pressure to the feedstock disposed between the cage and the choke, the pressure releasing oil through the openings of cage and extruding spent biomass from the choke end of the cage. In various embodiments, the cage has an internal length that is between at least ten times to at least twenty times its internal diameter. In various embodiments, the cage comprises a plurality of elongated bars with at least some of the elongated bars separated by one or more spacers, the bars resting on a frame, wherein the one or more spacers between the bars form the openings, and oil is released through the openings to a collecting vessel fluidly coupled with the cage. In various embodiments, the spacers between the elongated bars are of different thicknesses thereby allowing variation of the space between each elongated bar. In various embodiments, either the spacers or the gaps between the bars are from 0.13 to 0.76 mm (0.005 to 0.030 inches) thick.

In various embodiments, the pressure increases by a factor of between 10 and 20 from the feeder end to the choke end of the cage. In various embodiments, the pressure along the cage does not increase by more than 100% of the pressure at the feeder end of the cage per linear foot of the cage between the feeder and choke ends of the cage. In various embodiments, the power consumed by the device does not increase by more than 10% when fully loaded with conditioned feedstock relative to running empty. In various embodiments, the residence time of feedstock in the barrel of the device is no longer than 5-10 min. In various embodiments, either the temperature of the expeller device or the pressure exerted by the expeller device or both are monitored and/or controlled.

In various embodiments, pressure is controlled by adjusting rotational velocity of a worm shaft. In various embodiments, including those in which pressure is not controlled, an expeller (screw) press comprising a worm shaft and a barrel can be used. In various embodiments, the barrel has a length and a channel having a diameter sized to receive the worm shaft, and wherein the barrel length is at least 10 to 15 times greater than the channel diameter. In various embodiments, the barrel of the press has an entrance and an exit and the diameter of the worm shaft increases from the entrance to the exit, and the pressing comprises increasing the pressure from the entrance to the exit of the barrel; in various embodiments, the pressure at the exit is 12 to 16, or even up to 20 times higher than the pressure at the entrance. In various embodiments, the expeller press comprises a worm shaft and a barrel having a first channel and a second channel, both channels concentric and sized to receive the worm shaft, wherein the first channel has a first diameter and the second channel has a second diameter different than the first diameter. In various embodiments, the conditioned feedstock remains resident in the barrel of the expeller press for 5 to 10 minutes.

In various embodiments, the expeller press comprises a worm shaft disposed in a barrel lined with a plurality of elongate bars separated by one or more spacers therebetween, the spacers creating a gap between the elongate bars. In such a press, pressure can be controlled by adjusting the gap by changing the size or number of spacers between the elongate bars, and/or if the press has a space between an outer surface of the worm shaft and an inner surface of the elongate bars, pressure can be controlled by replacing at least some of the elongate bars with different sized bars so as to change the space. In various embodiments, the press comprises an output aperture and an adjustable choke coupled therewith, and pressure is controlled by adjusting the choke to increase or decrease the pressure. In various embodiments, the press comprises a worm shaft disposed in a barrel, and pressure is controlled by adjusting a gap between an outer surface of the worm shaft and an inside surface of the barrel.

After the pressing step, the method results in the extraction of oil and the production of spent biomass. In various embodiments, the released oil contains solid particles of biomass or conditioned feedstock, and the method further comprises separating the released oil from the solid particles. Optionally, the separated solid particles can be subjected to pressure to extract any remaining oil therefrom. In various embodiments, the extracted oil contains no more than 8 ppm chloride, no more than 2 ppm phosphorus, no more than 26 ppm potassium, no more than 12 ppm sodium, and/or no more than 5 ppm sulfur. The oil produced by the process is useful in a variety of applications, including but not limited to the production of fuels such as biodiesel and renewable diesel and the production of food.

In various embodiments, the oil content in the spent biomass of reduced oil content is at least 45 percent less than the oil content of the microbial biomass before the pressing step. In various embodiments, the spent biomass of reduced oil content remaining after the pressing step is pelletized or extruded as a cake. The spent biomass, which may be subjected to additional processes, including additional conditioning and pressing or solvent-based or other extraction methods to extract residual oil, is similarly useful in a variety of applications, including but not limited to use as food, particularly for animals, and as a bulking agent. In various embodiments, remaining oil is extracted from the spent biomass of reduced oil content; in various embodiments the extracting is performed by subjecting the spent biomass to pressure or by extracting the oil with an organic solvent.

In view of the foregoing, the present invention is directed to a method for extracting lipids from microbial biomass. In one embodiment, the method comprises subjecting microbial biomass constituting at least 20% lipids by weight and having a moisture content of less than 6% by weight to pressure, whereby cells of the biomass are lysed, releasing more than 5% of the lipids and leaving spent biomass of reduced lipid content, wherein the extracted lipids and spent biomass are separated from each other.

In some cases, the microbial biomass is subjected to a lower pressure for a first period of time followed by a higher pressure for a second period of time. In some cases, the microbial biomass is subjected to more than 5 cycles of oscillating pressure, and the average pressure exerted on the biomass during the course of the last cycle is at least 2 fold higher than the average pressure exerted on the biomass during the course of the first cycle. In some cases, the microbial biomass is subject to pressure by a method comprising continuous flow through a device applying the pressure. In one embodiment, the device is an expeller press. In some cases, the microbial biomass is subjected to at least 689 bar (10,000 PSI) of pressure.

In some embodiments, the microbial biomass is subject to pressure by a method comprising continuous flow through a device applying the pressure, wherein the device is a continuously rotating worm shaft within a cage having a feeder at one end and a choke at an end opposite thereof, and having openings within the cage, wherein the biomass enters the cage through the feeder, and rotation of the worm shaft advances the biomass along the cage and applies pressure to the biomass disposed between the cage and the choke, the pressure lysing cells of the biomass and releasing oil through the openings of the cage such that spent biomass of reduced oil content is extruded from the choke end of the cage. In some cases, the cage comprises a plurality of elongated bars with at least some of the elongated bars separated by one or more spacers, and the bars resting on a frame, wherein the one or more spacers between the bars form the openings, and lipids are released through the openings to a collecting vessel fluidly coupled with the cage. In some cases, the spacers between the elongated bars are of different thicknesses thereby allowing variation of the space between each elongated bar. In some embodiments, either the spacers or the gaps between the bars are from 0.13 to 0.76 mm (0.005 to 0.030 inches) thick. In some cases, the pressure increases by a factor of between 10 and 20 from the feeder end to the choke end of the cage. In some cases, the residence time of biomass in the barrel of the device is between 5-10 minutes. In some embodiments, the cage has an internal length that is between at least ten times to at least 20 times its internal diameter. In some cases, the power consumed by a device does not increase by more than 10% when fully loaded with microbial biomass relative to running empty. In some cases, the pressure along the cage does not increase by more than 100% of the pressure at the feeder end of the cage per linear foot of the cage between the feeder and choke ends of the cage.

In some embodiments, the method further comprises pelletizing the spent biomass of reduced oil content or extruding the spent biomass of reduced oil content as a cake. In some embodiments, the method further comprises extracting lipids from the spent biomass of reduced oil content. In some cases, the lipid content in the spent biomass of reduced oil content is at least 45 percent less than the lipid content of the microbial biomass before subjecting it to pressure. In some embodiments, the method further comprises extracting lipids from the spent biomass of reduced oil content with an organic solvent.

In some cases, the method comprises adjusting the moisture content of the microbial biomass to between 1.0% and 2.0% by weight before subjecting the microbial biomass to pressure. In some embodiments, the adjustment is achieved by conditioning the biomass with heat. In some cases, the conditioning with heat is performed using a vertical stacked conditioner.

In some embodiments, the method further comprises conditioning the biomass to change its physical or physiochemical properties without releasing more than 5% of the lipids to facilitate release of lipids in a subsequent step wherein the biomass is subjected to pressure. In some cases, the conditioning step comprises heating the biomass at 66-149 °C (150-300° F). In some cases, the conditioning step comprises heating the biomass at 93-132 °C (200-270° F). In some cases, the conditioning step comprises heating the biomass at 99-127 °C (210-260° F). In some embodiments, the conditioning step comprises heating the biomass for a period of time between 20 and 60 minutes. In some embodiments, the conditioning step comprises subjecting the biomass to a first pressure that does not release more than 5% of the lipids in the biomass.

In some embodiments, the method further comprises treating the biomass with an expander or extruder without releasing more than 5% of the lipids in the biomass before the step of subjecting the biomass to pressure sufficient to release more than 5% of the lipids.

In some embodiments, the method further comprises adding a bulking agent to the microbial biomass to facilitate release of the lipids when the microbial biomass is subjected to pressure. In some cases, the bulking agent is selected from the group consisting of switchgrass, soybean hulls, dried rosemary, corn stover, cellulose, spent biomass of reduced lipid content, and sugar cane bagasse. In some cases, the bulking agent is spent microbial biomass of reduced lipid content that comprises between 40% and 90% polysaccharide and less than 10% oil. In some cases, the bulking agent is spent microbial biomass of reduced lipid content that comprises between 60% and 80% polysaccharide and less than 10% oil. In some cases, the bulking agent is spent microbial biomass of the same strain as the microbial biomass. In some embodiments, the polysaccharide is of 20-30 mole percent galactose; 55-65% mole percent glucose; and 5-15 mole percent mannose. In some cases, the spent biomass of reduced lipid content is from microalgae from the genus *Chlorella*, *Parachlorella* or *Prototheca.* In some embodiments, the bulking agent has an average particle size of less than 1.5mm. In some cases, the bulking agent has an average particle size of between 150-350 microns. In some cases, the bulking agent is added to the microbial biomass prior to a step of dehydrating the microbial biomass to a moisture content of less than 6%.

In some embodiments of the present invention, the microbial biomass is microalgae. In some cases, the microalgae is selected from the species listed in Table 1. In some cases, the microalgae is of the genus *Chlorella, Parachlorella* or *Prototheca.* In some embodiments, the microalgae has a 23S rRNA genomic sequence with at least 75%, 85% or 95% nucleotide identity to one or more of SEQ ID NOs: 1-23 or 26-34.

In some embodiments of the present invention, the microbial biomass is bacteria. In some cases, the bacteria is from the genus *Rhodococcus.*

In some embodiments of the present invention, the microbial biomass is oleaginous yeast. In some cases, the oleaginous yeast is selected from the species listed in Table 2. In some cases, the oleaginous yeast is *Rhodotorula glutinis.* In some embodiments, the oleaginous yeast has a fungal 18S and 26S rRNA genomic sequence with at least 75%, 85%, or 95% nucleotide identity to one or more of SEQ ID NOs: 37-76. In some embodiments, the microbial biomass is an oleaginous yeast of the genus *Torulaspora* or *Yarrowia.*

In some embodiments of the present invention, the microbial biomass is non-yeast oleaginous fungi. In some cases, the non-yeast oleaginous fungi is selected from the species listed in Table 3.

In some embodiments, the microbial biomass contains at least 45% lipids by dry cell weight. In some cases, the microbial biomass has at least 15% carbohydrate by dry weight. In some cases, the microbial biomass is derived from microalgae having a fatty acid profile of: less than 2% C14:0; about 13-16% C16:0; about 1-4% C18:0; about 64-71% C18:1; about 10-15% C18:2; about 0.5-2% C18:3; and less than 1% carbon chain length 20 or longer. In some cases, the microalgae has a fatty acid lipid profile comprising of at least 15% C:16 fatty acids, at least 50% C18:1 fatty acids, at least 7% C18:2 fatty acids, and less than 3% C10:0-C14:0 fatty acids. In some cases, the microalgae has a fatty acid profile of: about 1-2% C14:0; about 16-26% C16:0; about 2-6% C18:0; about 58-68% C18:1; and about 7-11% C18:2. In some embodiments, the microalgae has a lipid profile comprising at least 4% C8-C14 and contains an exogenous gene encoding a thioesterase with a preference for one or more fatty acid chain lengths of 8, 10, 12 and 14 carbon atoms. In some cases, the microalgae has a lipid profile comprising between 10 and 40% C8-C14. In some cases, the microbial biomass has a lipid profile comprising at least 10% 16:1. In some embodiments, the microbial biomass contains at least 30% lipids by weight. In some cases, the microbial biomass contains at least 40% lipids by weight. In some cases, the microbial biomass contains at least 50% lipids by weight. In some cases, the microbial biomass contains between 60-70% lipids by weight.

In some embodiments, the extracted lipid has less than 0.01 milligram of chlorophyll per kilogram of lipid. In some cases, the extracted lipid has between 0.2 and 0.3 micrograms of carotenoids per milliliter of lipid.

In some embodiments, the microbial biomass contains an exogenous gene encoding a sucrose invertase.

In some embodiments, the microbial biomass has been subjected to a pneumatic drying step prior to application of pressure.

In some embodiments, the extracted lipids comprise one or more of the following: no more than 8 ppm chloride, no more than 2 ppm phosphorus, no more than 26 ppm potassium, no more than 12 ppm sodium, and no more than 5 ppm sulfur.

In another aspect, the present invention is directed to a method of preparing hydrated microbial biomass for oil extraction. In one embodiment, the method comprises adding a bulking agent to the biomass, and drying the bulking agent and the biomass together to a moisture content of less than 6%, thereby forming a dried bulking agent-biomass mixture. In some embodiments, the hydrated microbial biomass is contained in a fermentation broth that has not been subjected to separation or water removal processes. In some cases, the bulking agent is selected from the group consisting of switchgrass, soybean hulls, dried rosemary, corn stover, cellulose, spent biomass of reduced lipid content, and sugar cane bagasse. In some cases, the bulking agent is spent biomass of reduced lipid content that comprises between 40% and 90% polysaccharide and less than 10% oil.

In yet another aspect, the present invention is directed to a method for extracting lipids from microbial biomass. In one embodiment, the method comprises (a) co-drying hydrated microbial biomass constituting at least 20% lipids by weight and a bulking agent, therby forming a dried bulking agent-biomass mixture, (b) conditioning the dried bulking agent-biomass mixture so that the moisture content is from 0.5% to 2.5% by weight, and (c) subjecting the conditioned dried bulking agent-biomass mixture to pressure, whereby cells of the biomass are lysed, releasing more than 5% of the lipids and leaving spent biomass of reduced lipid content. In some embodiments, the hydrated microbial biomass is contained in a fermentation broth that has not been subjected to separation or water removal processes. In some cases, the bulking agent is selected from the group consisting of switchgrass, soybean hulls, dried rosemary, corn stover, cellulose, spent biomass of reduced lipid content and sugar cane bagasse. In some cases, the bulking agent is spent biomass of reduced lipid content that comprises between 40% and 90% polysaccharide and less than 10% oil.

In still another aspect, the present invention is directed to a method of increasing yield in lipid extraction from microbial biomass constituting at least 20% lipids by weight. In one embodiment, the method comprises co-drying the microbial biomass with a bulking agent, whereby the amount of oil extracted from the co-dried microbial biomass and bulking agent when subjected to pressure is greater than without the addition of the bulking agent. In some cases, the microbial biomass is derived from a culture that was cultivated through a process selected from the group consisting of a heterotrophic process, a photoautotrophic process, and a mixotrophic process.

These and other aspects and embodiments of the invention are described in the accompanying drawings, a brief description of which immediately follows, and in the detailed description of the invention below, and are exemplified in the examples below. Any or all of the features discussed above and throughout the application can be combined in various embodiments of the present invention.

### BRIEF DESCRIPTION OF THE DRAWINGS

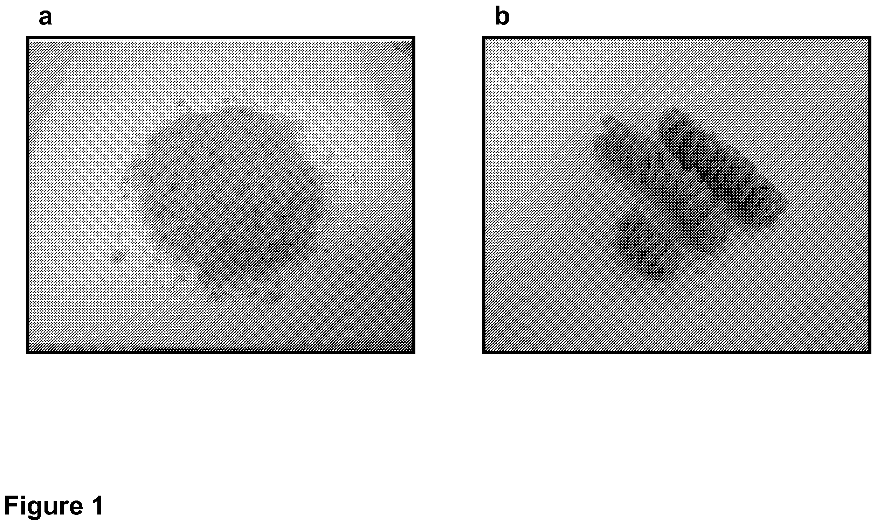

Figure 1a shows microalgal biomass after the surface moisture has been removed through drum drying.
Figure 1b shows microalgal biomass after being conditioned using a low pressure "pre-press" to form collets.
Figure 2a shows spent pressed cake from microbial biomass that is of poor quality for subsequent solvent extraction.
Figure 2b shows spent pressed cake from microbial biomass that is of good quality for subsequent solvent extraction.

### DETAILED DESCRIPTION OF THE INVENTION

The present invention provides methods for extracting lipids from microorganisms. This detailed description of the invention is divided into sections for the convenience of the reader, beginning with section I, which provides definitions of various terms used in describing the invention. Section II describes the methods of the invention for extracting oil from microorganisms, for preparing microbial biomass for the extraction of oil, and for further processing spent biomass. Section III describes microorganisms useful in generating oil-containing microbial biomass and methods for culturing them to produce oil. Section V provides illustrative examples of how to practice the methods of the invention.

### I. DEFINITIONS

Unless defined otherwise, all technical and scientific terms used herein have the meaning commonly understood by a person skilled in the art to which this invention belongs. The following references provide one of skill in the art to which this invention pertains with general definitions of many of the terms used in this disclosure: Singleton et al., Dictionary of Microbiology and Molecular Biology (2nd ed. 1994); The Cambridge Dictionary of Science and Technology (Walker ed., 1988); Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them, unless specified otherwise.

"Area Percent" refers to the area of peaks observed using FAME GC/FID detection methods in which every fatty acid in the sample is converted into a fatty acid methyl ester (FAME) prior to detection. For example, a separate peak is observed for a fatty acid of 14 carbon atoms with no unsaturation (C14:0) compared to any other fatty acid such as C14:1. The peak area for each class of FAME is directly proportional to its percent composition in the mixture and is calculated based on the sum of all peaks present in the sample (i.e. [area under specific peak/ total area of all measured peaks] X 100). When referring to lipid profiles of oils and cells of the invention, "at least 4% C8-C14" means that at least 4% of the total fatty acids in the cell or in the extracted glycerolipid composition have a chain length that includes 8, 10, 12 or 14 carbon atoms.

"Axenic" refers to a culture of an organism that is free from contamination by other living organisms.

"Biomass" refers to material produced by growth and/or propagation of cells. Biomass may contain cells and/or intracellular contents as well as extracellular material. Extracellular material includes, but is not limited to, compounds secreted by a cell.

"Bioreactor" refers to an enclosure or partial enclosure in which cells, *e*.*g*., microorganisms, are cultured, optionally in suspension.

"Bulking agent" and "press-aid" are used interchangeably herein and refer to material that is suitable to add to feedstock (such as dried and/or conditioned biomass) to increase the fiber content of the feedstock. Bulking agents include, but are not limited to, switchgrass, soybean hulls, spent biomass, and sugar cane bagasse. Bulking agents facilitate release of lipids (oil) from biomass, perhaps by increasing the uniformity with which pressure can be applied to the component cells of the biomass. In some cases, a press aid can also act as a filter aid, clarifying or reducing the amount of foots that is extracted with the oil. An example of a press aid that also acts as a filter aid is cellulose.

"Cellulosic material" means the products of digestion of cellulose, including glucose, xylose, disaccharides, oligosaccharides, lignin, and other molecules.

"Conditioned feedstock" is dried, oil-bearing microbial biomass that has been physically altered in some way after being dried, typically without releasing more than 5% of total oil from the biomass and heated to a temperature in the range of 70°C to 150 °C (160°F to 300°F). As used in this context, the substance to which "releasing" and "released" refer is oil. Examples of conditioning include putting the dried, oil-bearing microbial biomass through a vertical stacked conditioner, an expander, an extruder, or an expeller; and/or subjecting the dried, oil-bearing microbial biomass to flaking, cracking, grinding, crushing, heating, steaming, thermal conditioning, low pressure, high pressure, and other methods for changing the physical nature of the dried, oil-bearing microbial biomass to maximize oil extraction using non-chemical or solventless extraction methods. Changes that occur upon subjecting the dried, oil-bearing microbial biomass to conditioning include changes at the micron scale, such as ruptured cells walls, as well as changes at macro scale, such as conversion of dried flakes into pellets without releasing oil by low pressure pressing.

"Delipidated meal" and "delipidated microbial biomass" refer to microbial biomass after oil (including lipid) has been extracted from it, either through the use of an expeller press or solvent extraction or both.

"Dry back" refers to the process of adding pressed cake (also referred to herein as spent biomass) back into the feed end of the press where it is mixed with unpressed biomass. Essentially, the pressed cake is acting as a bulking agent or press aid for the unpressed material. Residual oil in the pressed cake can be further recovered along with oil from the unpressed biomass using this process.

"Dry cell weight" refers the weight of microbial biomass once all or substantially all of the water (moisture) has been removed therefrom.

"Dry microbial biomass" refers to microbial biomass from which the free moisture or surface moisture has been removed, usually so that the microbial biomass contains less than 10%, and often less than 6%, of moisture by weight. In one embodiment, dried microbial biomass is derived from microalgae. In one embodiment, the dried microbial biomass is derived from microalgae that contains at least 20% lipids by dry cell weight after drying.

"Expander" refers to a low-shear extruder that heats, homogenizes, and/or shapes oilseeds and other oil-bearing material into porous collets or pellets with a high bulk density. In one embodiment of an expander-mediated process, steam is injected into oilseed flakes/cakes or oil-bearing material under pressure, and this mixture is extruded through plates to the atmosphere. The collets expand when released to the atmosphere, hence the name expander. Historically, the expander has been used to prepare plant seed/oil seed derived collets for solvent extraction because of the higher bulk density of the collets after treatment with the expander, which allows for more surface area and increased efficiency in solvent extraction.

"Expeller press" means a screw press or continuous expeller that is used for mechanical extraction of oilseeds, such as but not limited to soybeans and rapeseed/canola. Oil-bearing raw material (such as oilseeds) is fed into the machine at one end and the material is subjected to friction and high pressure from the screw drive that moves the material along a shaft. Oil is released and seeps through small openings along the shaft and the solids (with reduced oil content) are expelled at the end of the shaft as a pressed cake. Examples of expeller/screw presses include those that are marketed by Anderson International Corp. (Cleveland, OH), Alloco (Santa Fe, Argentina), De Smet Rosedowns (Humberside, UK), The Dupps Co. (Germantown, Ohio), Grupo Tecnal (Sao Paulo, Brazil), Insta Pro (Des Moines, Iowa), Harburg Freudenberger (previously Krupp Extraktionstechnik) (Hamburg, Germany), French Oil Mill Machinery Company (Piqua, OH), Maschinenfabrik Reinartz (Neuss, Germany), Shann Consulting (New South Wales, Australia) and SKET (Magdeburg, Germany).

"Fiber" means the complex carbohydrates from plants and other fiber containing sources such as microorganisms that cannot be digested by humans. The complex carbohydrates found in fiber can include cellulose, hemicellulose and lignin, dextrins, pectins, beta-glucans and oligosaccharides.

"Fixed carbon source" refers to molecule(s) containing carbon, typically organic molecules, that are present at ambient temperature and pressure in solid or liquid form during a fermentation.

"Hydrated microbial biomass" means microbial biomass containing at least 10% moisture content that is in a liquid. In some embodiments, hydrated microbial biomass is contained in a fermentation broth that has not been subjected to separation or water removal processes.

"Hydrocarbon" refers to: (a) a molecule containing only hydrogen and carbon atoms, wherein the carbon atoms are covalently linked to form a linear, branched, cyclic or partially cyclic backbone to which the hydrogen atoms are attached; or (b) a molecule that primarily contains hydrogen and carbon atoms that can be converted to contain only hydrogen and carbon atoms by one to four chemical reactions. Non-limiting examples of the latter include hydrocarbons containing an oxygen atom between one carbon and one hydrogen atom to form an alcohol molecule, as well as aldehydes containing an oxygen atom. Methods for the reduction of alcohols to hydrocarbons containing only carbon and hydrogen atoms are well known. Another example of a hydrocarbon is an ester, in which an organic group replaces a hydrogen atom (or more than one) in an oxygen acid. The molecular structure of hydrocarbon compounds varies from the simplest, in the form of methane (CH₄), which is a constituent of natural gas, to the very large and complex, such as as the asphaltenes found in crude oil, petroleum, and bitumens. Hydrocarbons may be in gaseous, liquid, or solid form, or any combination of these forms, and may have one or more double or triple bonds between adjacent carbon atoms in the backbone. Accordingly, the term includes linear, branched, cyclic or partially cyclic alkanes, alkenes, lipids, and paraffin. Examples include propane, butane, pentane, hexane, octane, squalene and carotenoids.

"Hydrogen:carbon ratio" refers to the ratio of hydrogen atoms to carbon atoms in a molecule on an atom-to-atom basis. The ratio may also be used to refer to the number of carbon and hydrogen atoms in a hydrocarbon molecule. For example, the hydrocarbon with the highest ratio is methane, CH₄ (4:1).

"Hydrophobic fraction" refers to a portion, or fraction, of a material that is more soluble in a hydrophobic phase in comparison to an aqueous phase. A hydrophobic fraction is substantially insoluble in water and usually non-polar.

The phrase "increased lipid yield" refers to an increase in the productivity of a microbial culture by, for example, increasing dry weight of cells per liter of culture, increasing the percentage of lipid in cells and/or the percentage of cells that constitute lipid, and/or increasing the overall amount of lipid per culture volume per unit time.

The phrase "limiting concentration of a nutrient" refers to a concentration of nutrient in a culture that limits the propagation of a cultured organism. A "non-limiting concentration of a nutrient" is a concentration that supports maximal propagation during a given culture period. Thus, the number of cells produced during a given culture period is lower in the presence of a limiting concentration of a nutrient than when the nutrient is non-limiting. A nutrient is said to be "in excess" in a culture, when the nutrient is present at a concentration greater than that which supports maximal propagation.

"Lipid" refers to a lipophilic molecule from a biological organism. Biological functions of a lipid include, but are not limited to, storing energy, serving as a structural component of a cell membrane, and acting as a signaling molecule. Lipid molecules are soluble in nonpolar solvents (such as ether and chloroform) and are relatively or completely insoluble in water. Lipid molecules have these properties, because they consist largely of relatively long hydrocarbon chains which are hydrophobic in nature. Examples of lipids include fatty acids (saturated and unsaturated); glycerides or glycerolipids (such as monoglycerides, diglycerides, triglycerides, and neutral fats, and phosphoglycerides or glycerophospholipids); nonglycerides (sphingolipids, sterol lipids, including cholesterol and steroid hormones, prenol lipids, including terpenoids, waxes, and polyketides); and complex lipid derivatives (sugar-linked lipids, or glycolipids, and protein-linked lipids). Other examples of lipid include free fatty acids; esters of fatty acids; sterols; pigments (e.g., carotenoids and oxycarotenoids), phytosterols, ergothionine, lipoic acid, antioxidants including beta-carotene and tocopherol. Also included in the class of lipids are polyunsaturated fatty acids such as arachidonic acid, stearidonic acid, cholesterol, desmesterol, astaxanthin, canthaxanthin, and n-6 and n-3 highly unsaturated fatty acids such as eicosapentaenoic acid (EPA), docosapentaenoic acid, and docosahexaenoic acid (DHA). Microbial oil, as used herein, refers to lipid.

The phrase "lipid:organic solvent composition" refers to a mixture of lipid and organic solvent.

"Lysed" refers to having broken or disrupted the cellular or plasma membrane and optionally the cell wall of a biological organism or cell, and releasing at least some intracellular content into the extracellular environment. "Lysis" refers to the breakage of the cellular or plasma membrane and optionally the cell wall of a biological organism sufficient to release at least some intracellular content into the extracellular environment, often by mechanical, viral, osmotic, or temperature variation mechanisms that compromise its integrity. "Lysing" refers to disrupting the cellular or plasma membrane and optionally the cell wall of a biological organism or cell sufficient to release at least some intracellular content into the extracellular environment.

"Microalgae" refers to a microbial organism that contains a chloroplast, and optionally that is capable of performing photosynthesis. Microalgae include obligate photoautotrophs, which cannot metabolize a fixed carbon source as energy, as well as heterotrophs, which can live solely off of a fixed carbon source. Microalgae can refer to unicellular organisms that separate from sister cells shortly after cell division, such as *Chlamydomonas*, and to microbes such as, for example, *Volvox,* which is a simple multicellular photosynthetic microbe of two distinct cell types. "Microalgae" can also refer to cells such as *Chlorella* and *Dunaliella.* "Microalgae" also includes other microbial photosynthetic organisms that exhibit cell-cell adhesion, such as *Agmenellum, Anabaena,* and *Pyrobotrys.* "Microalgae" also includes obligate heterotrophic microorganisms that have lost the ability to perform photosynthesis, such as certain dinoflagellate species and species of the genus Prototheca.

"Microbial biomass" refers to biomass derived from a microbe.

"Microorganism" and "microbe" are used interchangeably herein and refer to microscopic unicellular organisms.

"Oil" refers to a hydrophobic, lipophilic, nonpolar carbon-containing substance including but not limited to geologically-derived crude oil, distillate fractions of geologically-derived crude oil, hydrocarbons, vegetable oil, algal oil, and microbial lipids.

"Oleaginous yeast" refers to a yeast that can accumulate more than 20% of its dry cell weight as lipid. Oleaginous yeast include organisms such as *Yarrowia lipolytica* and other species of the *Dikarya* subkingdom of fungi such as *Rhodosporidium toruloides* (*Eukaryota; Fungi*/*Metazoa group; Fungi; Dikarya; Basidiomycota; Pucciniomycotina; Microbotryomycetes; Sporidiobolales; Rhodosporidium*); *Rhodotorula glutinis* (*Eukaryota; Fungi*/*Metazoa group; Fungi; Dikarya; Basidiomycota; Pucciniomycotina; Microbotryomycetes; Sporidiobolales; mitosporic Sporidiobolales; Rhodotorula); Lipomyces tetrasporus* (*Eukaryota; Fungi*/*Metazoa group; Fungi; Dikarya; Ascomycota; Saccharomyceta; Saccharomycotina; Saccharomycetes; Saccharomycetales; Lipomycetaceae; Lipomyces); Cryptococcus curvatus* (*Eukaryota; Fungi*/*Metazoa group; Fungi; Dikarya; Basidiomycota; Agaricomycotina; Tremellomycetes; Tremellales; mitosporic Tremellales; Cryptococcus*); *Trichosporon domesticum* (*Eukaryota; Fungi*/*Metazoa group; Fungi; Dikarya; Basidiomycota; Agaricomycotina; Tremellomycetes; Tremellales; mitosporic Tremellales; Trichosporon*); *Yarrowia lipolytica* (*Eukaryota; Fungi*/*Metazoa group; Fungi; Dikarya; Ascomycota; Saccharomyceta; Saccharomycotina; Saccharomycetes; Saccharomycetales; Dipodascaceae; Yarrowia); Sporobolomyces alborubescens* (*Eukaryota; Fungi*/*Metazoa group; Fungi; Dikarya; Basidiomycota; Pucciniomycotina; Microbotryomycetes; Sporidiobolales; mitosporic Sporidiobolales; Sporobolomyces*); *Geotrichum vulgare* (*Eukaryota; Fungi*/*Metazoa group; Fungi; Dikarya; Ascomycota; Saccharomyceta; Saccharomycotina; Saccharomycetes; Saccharomycetales; Dipodascaceae; mitosporic Dipodascaceae; Geotrichum*): and *Torulaspora delbrueckii* (*Eukaryota; Fungi*/*Metazoa group; Fungi; Dikarya; Ascomycota; Saccharomyceta; Saccharomycotina; Saccharomycetes; Saccharomycetales; Saccharomycetaceae; Torulaspora*). Within *Dikarya,* the invention includes use of organisms from all sub-domains of *Dikarya* (*Ascomycota* and *Basidiomycota*) and taxonomic sub-classifications within *Ascomycota* and *Basidiomycota.*

"Organic solvent" refers to a carbon-containing material that dissolves a solid, liquid, or gaseous solute, resulting in a solution.

"Photobioreactor" refers to a container, at least part of which is at least partially transparent or partially open, thereby allowing light to pass through, in which, e.g., one or more microalgae cells are cultured. Photobioreactors may be closed, as in the instance of a polyethylene bag or Erlenmeyer flask, or may be open to the environment, as in the instance of an outdoor pond.

"Polysaccharide" (also called "glycan") refers to carbohydrate made up of monosaccharides joined together by glycosidic linkages. Cellulose is an example of a polysaccharide that makes up certain plant cell walls. Cellulose can be depolymerized by enzymes to yield monosaccharides such as xylose and glucose, as well as larger disaccharides and oligosaccharides. Other examples of polysaccharides include fiber, soluble and insoluble dietary fiber, hemicellulose, and the carbohydrate from microbial cell walls, such as that contained in spent biomass.

"Polysaccharide-degrading enzyme" refers to any enzyme capable of catalyzing the hydrolysis, or depolymerization, of any polysaccharide. For example, cellulose catalyzes the hydrolysis of cellulose.

"Port", in the context of a bioreactor, refers to an opening in the bioreactor that allows influx or efflux of materials such as gases, liquids and cells. Ports are usually connected to tubing leading from the photobioreactor.

"Pressing" refers to the application of sufficient pressure to force intracellular oil from microbial biomass, which may also be referred to herein as a "pressing step." Pressing may be sufficient to lyse all or substantially all of the cells in the microbial biomass.

"Spent biomass", "spent microbial biomass" and "pressed cake" all refer to microbial biomass that has been made into conditioned feedstock and then has been subjected to high pressure so that the resulting material has less lipid content on a w/w basis than the conditioned feedstock from which it is derived. High pressure can be achieved by the use of compression pressure, such as that provided by machines such as an expeller press, a screw oil expeller, and a mechanical press, as well as by direct hydraulic pressure and other processes so that the oil is squeezed out of the conditioned feed stock. In one embodiment, the spent microbial biomass is prepared by passing oil-bearing microbial biomass through an oilseed press. In one embodiment, the spent microbial biomass is microalgae biomass that has less than 30% oil by dry cell weight

"Suitable for animal feed" means a substance or material can be consumed without deleterious effect by an animal, typically a a non-human mammal of agricultural or veterinary interest, including but not limited to horses, cattle, pigs, chickens, dogs and cats; in preferred embodiments, a material suitable for animal feed provides nutrition to the animal.

### II. METHODS FOR EXTRACTING OIL FROM MICROORGANISMS

In one aspect, the present invention provides methods for extracting, recovering, isolating, or otherwise obtaining oil (lipids) from microorganisms. The methods of the present invention are applicable to extracting a variety of lipids from a variety of microorganisms. In the methods of the present invention, the lipid-producing microorganism (*e.g.,* a microalgae) is first cultivated under conditions that allows for lipid production to generate oil-containing microbial biomass. The oil-containing biomass is then, depending on the method employed, optionally admixed with a bulking agent, and dried and conditioned to prepare a dry, conditioned feedstock that is then pressed to extract the oil. For the convenience of the reader, this discussion is divided into subsections.

Subsection A describes the microbial biomass suitable for oil extraction in accordance with the methods of the invention. Subsection B describes methods for removing water from the biomass, including dewatering and drying. Subsection C describes methods for conditioning the biomass. Subsection D describes bulking agents (press aids) and their use with dry microbial biomass, hydrated microbial biomass, and conditioned feedstock. Subsection E describes various methods for subjecting conditioned feedstock to pressure to extract oil (the pressing step). Subsection F describes the oil produced by the pressing step and methods for its use and further purification. Subsection G describes the spent biomass of reduced oil content produced by the pressing step and methods for its use.

### A. Suitable Biomass

While biomass from a wide variety of microbes, including microalgae, oleaginous bacteria, oleaginous yeast and fungi (see Section III, below), can be employed in the methods of the invention, microbial biomass suitable for use in the methods described herein typically comprises at least 20% oil by dry cell weight. In some embodiments, the biomass comprises oil in a range of from at least 25% to at least 60% or more oil by dry cell weight. In some embodiments, the biomass contains from 15-90% oil, from 25-85% oil, from 40-80% oil, or from 50-75% oil by dry cell weight. In various embodiments of the invention, the microbial biomass (dry or hydrated) or conditioned feedstock contains at least 25% oil by weight. In various embodiments, the dry microbial biomass or conditioned feedstock contains at least 25% lipids by weight or by dry cell weight. In various embodiments, the dry microbial biomass or conditioned feedstock contains at least 40%, at least 50%, or at least 75% lipids by weight or by dry cell weight. In various embodiments, the dry microbial biomass or conditioned feedstock contains at least 15% carbohydrate by weight or by dry cell weight.

The oil of the biomass described herein, or extracted from the biomass for use in the methods and compositions of the present invention can comprise glycerolipids with one or more distinct fatty acid ester side chains. Glycerolipids are comprised of a glycerol molecule esterified to one, two, or three fatty acid molecules, which can be of varying lengths and have varying degrees of saturation. The length and saturation characteristics of the fatty acid molecules (and thus the oil) can be manipulated to modify the properties or proportions of the fatty acid molecules in the oil of the present invention via culture conditions or via lipid pathway engineering, as described herein (see also PCT Patent Application Nos. US09/066141 and US09/066142). Thus, specific blends of algal oil can be prepared either within a single species of microalgae (or other microbe), or by mixing together the biomass or algal oil from two or more species of microalgae (or other microbe(s)).

The oil composition, *i.e*., the properties and proportions of the fatty acid constituents of the glycerolipids, can also be manipulated by combining biomass or oil from at least two distinct genera or species of microbes, i.e., microalgae. In some embodiments, at least two of the distinct genera or species of microbes, i.e., microalgae, have different glycerolipid profiles. The distinct species (or genera) of microbes can be cultured together or separately as described herein (for microalgae, typically under heterotrophic conditions), to generate the respective oils. Different species of microbes can contain different percentages of distinct fatty acid constituents in the cell's glycerolipids.

In various embodiments, the microbial oil is primarily comprised of monounsaturated oil. In some cases, the oil is at least 50% monounsaturated oil by weight or volume. In various embodiments, the oil is at least 50%, at least 60%, at least 70%, or at least 80% or more monounsaturated oil by weight or by volume. In some embodiments, the oil comprises at least 10%, at least 20%, at least 30%, at least 40%, or at least 50% or more esterified oleic acid or esterified alpha-linolenic acid by weight or by volume. In various embodiments, the oil comprises less than 10%, less than 5%, or less than 1% by weight or by volume, or is substantially free of, esterified docosahexanoic acid (DHA).

In various embodiments of this and other aspects of the invention, the biomass is prepared by fermentation of a microbe that contains 18:1 fatty acid. In various embodiments, the microbe has a fatty acid profile of less than 1% C14:0; about 10-11% C16:0; about 3-4% C18:0; about 70-71% C18:1; about 14-15% C18:2; about 1-2% C18:3; and less than 1% C20:0. In various embodiments, the microbe has a fatty acid profile of about 1-2% C14:0; about 20% C16:0; about 4% C18:0; about 64% C18:1; and about 7-8% C18:2. In various embodiments, the microbe has at most 0.5% DHA. In these and other embodiments, the microbe is, in some instances, a microalgae.

Thus, a wide variety of microbial biomass is suitable for use in the methods of the invention. In accordance with these methods, the oil-containing biomass is typically dewatered, dried, conditioned, and then pressed to extract the oil.

### B. Dewatering and Drying the Microbial Biomass

The various embodiments of the methods of the invention involve one or more steps of removing water (or other liquids) from the microbial biomass. These steps of removing water can include the distinct steps referred to herein as dewatering and drying.

Dewatering, as used herein, refers to the separation of the oil-containing microbe from the fermentation broth (liquids) in which it was cultured. Dewatering, if performed, should be performed by a method that does not result in, or results only in minimal loss in, oil content of the biomass. Accordingly, care is generally taken to avoid cell lysis during any dewatering step. Dewatering is a solid-liquid separation and involves the removal of liquids from solid material. Common processes for dewatering include centrifugation, filtration, and/or the use of mechanical pressure.

Centrifugation is a process that involves the use of centrifugal force for the separation of mixtures. The more dense components of the mixture migrate away from the axis of the centrifuge, while the less dense components of the mixture migrate towards the axis. By increasing the effective gravitational force (*i.e.,* by increasing the centrifugation speed), more dense material, usually solids, separate from the less dense material, usually liquids, according to density.

Microbial biomass useful in the methods of the present invention can be dewatered from the fermentation broth through the use of centrifugation, to form a concentrated paste. After centrifugation, there is still a substantial amount of surface or free moisture in the microbial biomass (*e.g.,* upwards of 70%) and thus, centrifugation is not considered to be, for purposes of the present invention, a drying step. Optionally, after centrifugation, the biomass can be washed with a washing solution (*e.g.,* deionized water) to remove remaining fermentation broth and debris.

In some embodiments, dewatering involves the use of filtration. One example of filtration that is suitable for the present invention is tangential flow filtration (TFF), also known as cross-flow filtration. Tangential flow filtration is a separation technique that uses membrane systems and flow force to purify solids from liquids. For a preferred filtration method see Geresh, Carb. Polym. 50; 183-189 (2002), which discusses use of a MaxCell A/G technologies 0.45 uM hollow fiber filter. Also see for example Millipore Pellicon® devices, used with 100kD, 300kD, 1000 kD (catalog number P2C01MC01), 0.1uM (catalog number P2VVPPV01), 0.22uM (catalog number P2GVPPV01), and 0.45uM membranes (catalog number P2HVMPV01). The retentate should not pass through the filter at a significant level. The retentate also should not adhere significantly to the filter material. TFF can also be performed using hollow fiber filtration systems.

Non-limiting examples of tangential flow filtration include those involving the use of a filter with a pore size of at least about 0.1 micrometer, at least about 0.12 micrometer, at least about 0.14 micrometer, at least about 0.16 micrometer, at least about 0.18 micrometer, at least about 0.2 micrometer, at least about 0.22 micrometer, at least about 0.45 micrometer, or at least about 0.65 micrometers. Preferred pore sizes of TFF allow solutes and debris in the fermentation broth to flow through, but not microbial cells.

In other embodiments, dewatering involves the use of mechanical pressure directly applied to the biomass to separate the liquid fermentation broth from the microbial biomass. The amount of mechanical pressure applied should not cause a significant percentage of the microbial cells to rupture, if that would result in loss of oil, but should instead simply be enough to dewater the biomass to the level desired for subsequent processing.

One non-limiting example of using mechanical pressure to dewater microbial biomass employs the belt filter press. A belt filter press is a dewatering device that applies mechanical pressure to a slurry (*e.g*., microbial biomass that is directly from the fermentor or bioreactor) that is passed between the two tensioned belts through a serpentine of decreasing diameter rolls. The belt filter press can actually be divided into three zones: gravity zone, where free draining water/liquid is drained by gravity through a porous belt; a wedge zone, where the solids are prepared for pressure application; and a pressure zone, where adjustable pressure is applied to the gravity drained solids.

One or more of the above dewatering techniques can be used alone or in combination to dewater the microbial biomass for use in the present invention. The present invention results in part from the discovery that the moisture content of the microbial biomass (conditioned feedstock) dramatically affects the yield of oil obtained in the pressing step, and that the optimal moisture level, below 6% and preferably below 2%, is quite different from the optimal moisture levels for pressing oil from many oil-bearing seeds. While the optimal moisture level can vary depending on the type of oil-bearing seed, and can also vary depending on the type of microbial biomass, the optimal moisture level for pressing microbial biomass is less than that for oil seeds. For example, the optimal moisture content for pressing sesame and linseed is about 4% (Willems et al., J. Food Engineering 89:1, pp.8-16, 2008). The optimal moisture content for pressing crambe seeds is between 9.2 and 3.6% (Singh et al., JAOCS 79:2, pp.165-170, 2006). The optimal moisture content for pressing canola seeds is about 5% (Vadke et al., JAOCS 65:7, pp.1169-1176, 1988). The optimal moisture content for pressing coconut is about 11% (Mpagalile et al., Int. J. Food Sciences and Nutrition, 56:2, pp.125 - 132, 2005). Other optimal moisture contents are 7% for rapeseed, 6% for camelina, 8.5% for sunflower, 11% for safflower and 12% for soybean (Alam, M.S. November 2007. Basics of Fats and Oils Chemistry: Factors Affecting Crude Oil Quality. Presented to the Vegetable Oils Extraction Short Course, Texas A&M Food Protein R&D Center, College Station, Texas).

In contrast, the optimal moisture content for pressing microbial biomass is less than 6% by weight, and more preferably less than 3%. For example, optimal moisture content can be 0.5-2% by weight. In various embodiments, particularly those relating to the extraction of oil from microalgal biomass, the optimal moisture content is in the range of 0.5% to 2% of the total weight of the microbial biomass. In one embodiment, the moisture content is in the range of 0.7% to 1.2% of the total weight of the microbial biomass. In one embodiment, the moisture content is in the range of 1.0% to 2.0% of the total weight of the microbial biomass. The optimal moisture level can depend on several factors, including but not limited to the percent lipids (oil) as measured by dry cell weight (DCW) or the amount of fiber and hemicellulose in the biomass. In some embodiments of the methods of the invention, such as, for example, those in which a bulking agent is employed (see subsection D), dewatering alone provides a suitable moisture content of the microbial biomass that is then conditioned prior to the pressing step. In other methods and embodiments of the invention, dewatered biomass is subjected to a drying step and then conditioned prior to the pressing step (in which oil is extracted from the biomass).

Drying, as referred to herein, refers to the removal of some or all of the free moisture or surface moisture of the microbial biomass. Like dewatering, the drying process should not result in significant loss of oil from the microbial biomass. Thus, the drying step should typically not cause lysis of a significant number of the microbial cells, because in most cases, the lipids are located in intracellular compartments of the microbial biomass. Several methods of drying microbial biomass known in the art for other purposes are suitable for use in the methods of the present invention. Microbial biomass after the free moisture or surface moisture has been removed is referred to as dried microbial biomass.

In various embodiments, the dry microbial biomass has a moisture content in the range of 0.1% to 5% by weight. In various embodiments, the dry microbial biomass has a moisture content of less than 4% by weight. In various embodiments, the dry microbial biomass has a moisture content in the range of 0.5% to 3.5% by weight. In various embodiments, the dry microbial biomass has a moisture content in the range of 0.1% to 3% by weight. Non-limiting examples of drying methods suitable for use in preparing dry microbial biomass in accordance with the methods of the invention include lyophilization and the use of dryers such as a drum dryer, spray dryer, and a tray dryer, each of which is described below.

Lyophilization, also known as freeze drying or cryodessication, is a dehydration process that is typically used to preserve a perishable material. The lyophilization process involves the freezing of the material and then reducing the surrounding pressure and adding enough heat to allow the frozen water in the material to sublime from the solid phase to gas. In the case of lyophilizing microbial biomass, such as microalgae derived biomass, the cell wall of the microalgae acts as a cryoprotectant that prevents degradation of the intracellular lipids during the freeze dry process.

Drum dryers are one of the most economical methods for drying large amounts of microbial biomass. Drum dryers, or roller dryers, consist of two large steel cylinders that turn toward each other and are heated from the inside by steam. In some embodiments, the microbial biomass is applied to the outside of the large cylinders in thin sheets. Through the heat from the steam, the microbial biomass is then dried, typically in less than one revolution of the large cylinders, and the resulting dry microbial biomass is scraped off of the cylinders by a steel blade. The resulting dry microbial biomass has a flaky consistency. In various embodiments, the microbial biomass is first dewatered and then dried using a drum dryer. More detailed description of a drum dryer can be found in US Patent No. 5,729,910, which discloses a rotary drying drum.

Spray drying is a commonly used method of drying a liquid feed using a hot gas. A spray dryer takes a liquid stream (*e.g*., containing the microbial biomass) and separates the solute as a solid and the liquid into a vapor. The liquid input stream is sprayed through a nozzle into a hot vapor stream and vaporized. Solids form as moisture quickly leaves the droplets. The nozzle of the spray dryer is adjustable, and typically is adjusted to make the droplets as small as possible to maximize heat transfer and the rate of water vaporization. The resulting dry solids may have a fine, powdery consistency, depending on the size of the nozzle used. In other embodiments, spray dryers can use a lyophilization process instead of steam heating to dry the material.

Tray dryers are typically used for laboratory work and small pilot scale drying operations. Tray dryers work on the basis of convection heating and evaporation. Fermentation broth containing the microbial biomass can be dried effectively from a wide range of cell concentrations using heat and an air vent to remove evaporated water.

Flash dryers are typically used for drying solids that have been de-watered or inherently have a low moisture content. Also known as "pneumatic dryers", these dryers typically disperse wet material into a stream of heated air (or gas) which conveys it through a drying duct. The heat from the airstream (or gas stream) dries the material as it is conveyed through the drying duct. The dried product is then separated using cyclones and/or bag filters. Elevated drying temperatures can be used with many products, because the flashing off of surface moisture instantly cools the drying gas/air without appreciably increasing the product temperature. More detailed descriptions of flash dryers and pneumatic dryers can be found in US Patent No. 4,214,375, which describes a flash dryer, and US Patent Nos. 3,789,513 and 4,101,264, which describe pneumatic dryers.

Regardless of the method selected for a drying step, the objective of the drying step is to reduce moisture content in the microbial biomass. The dry microbial biomass (conditioned feedstock) suitable for pressing has a moisture content of 0.5-2.5% by weight. Moisture may be added back to the biomass, if necessary, after drying to adjust moisture content to the optimal level. If the dry microbial biomass will be admixed with a dry bulking agent (see subsection D) or conditioned in a manner that will reduce moisture content further (see subsection C), then higher (above 6% by weight) moisture content may be acceptable, as bulking agents and/or conditioning can, in some embodiments, reduce the moisture content to the desired optimal level.

Dewatered and/or dried microbial biomass is conditioned prior to the pressing step, as described in the following subsection.

### C. Conditioning the Microbial Biomass

Conditioning of the microbial biomass helps to achieve desired levels of oil extraction. Conditioning refers to heating the biomass to a temperature in the range of 70°C to 150 °C (160°F to 300°F) and changing the physical or physiochemical nature of the microbial biomass to improve oil yields in the subsequent oil extraction (pressing) step. Conditioning microbial biomass results in the production of "conditioned feedstock." In addition to heating or "cooking" the biomass, non-limiting examples of conditioning the biomass include adjusting the moisture content within the dry microbial biomass, subjecting the dry microbial biomass to a low pressure "pre-press", subjecting the dry microbial biomass to cycles of heating and cooling, subjecting the dry microbial biomass to an expander, and/or adjusting the particle size of the dry microbial biomass.

The conditioning step can include techniques (e.g., heating or application or pressure) that overlap in part with techniques used in the drying or pressing steps. However, the primary goals of these steps are different: the primary goal of the drying step is the removal of some or all of the free moisture or surface moisture from the microbial biomass. The primary goal of the conditioning step is to heat the biomass, which can optionally result in the removal of intracellular water from, i.e., adjusting the intracellular moisture content of, the microbial biomass and/or altering the physical or physiochemical nature of the microbial biomass without substantial release of lipids to facilitate release of oil during the pressing step. The primary the goal of the pressing step is to release oil from the microbial biomass or conditioned feedstock, i.e., the extraction of the oil.

In various embodiments, conditioning involves altering, or adjusting, the moisture content of the microbial biomass by the application of heat, i.e., heat conditioning. Heat conditioning, as used herein, refers to heat treatment (either direct or indirect) of microbial biomass. The moisture content of the microbial biomass can be adjusted by conditioning using heat (either direct or indirect), which is done after a drying step. Even though the biomass may be dried by any of the above described methods, the moisture content of the microbial biomass after drying can range, for example, from 3% to 15% moisture by weight, or 5-10% moisture by weight. Such a moisture range may not be optimal for maximal oil recovery in the pressing step. Therefore, there may be benefit in heat-conditioning dewatered and/or dry microbial biomass to adjust the moisture level to a level (below 6%) optimal for maximal oil recovery.

Heat conditioners used in oil seed processing are suitable for use in conditioning microbial biomass in accordance with the methods of the present invention, such as vertical stacked conditioners. These consist of a series of three to seven or moreclosed, superimposed cylindrical steel pans. Each pan is independently jacketed for steam heating on both sides and bottom and is equipped with a sweep-type stirrer mounted close to the bottom, and operated by a common shaft extending through the entire series of pans. The temperature of the heat conditioner is also adjustable through regulation of the steam heating. There is an automatically operated gate in the bottom of each pan, except the last, for discharging the contents to the pan below. The top pan is provided with spray jets for the addition of moisture if desired. While moisture is sprayed onto seeds in many agricultural oil extraction processes during conditioning, this common process is not desirable for conditioning microbial biomass. Cookers also typically have an exhaust pipe and fan for removal of moisture. Thus, it is possible to control the moisture of the microbial biomass, not only with respect to final moisture content but also at each stage of the operation. In this respect, a conditioning step of heating microbial biomass for an extended period of time (10-60 minutes for example) provides the effect of not only reducing moisture and increasing the temperature of the biomass, but also altering the biophysical nature of the microbial biomass beyond any heating effects that might occur in a subsequent pressing step, i.e., simply from friction of the material as it is forced through, e.g., a press.

Additionally, a steam jacketed horizontal cooker is another type of heat conditioner that is suitable for use in accordance with the methods of the invention herein. In this design, the biomass is mixed, heated and conveyed in a horizontal plane in deeper beds as compared to conventional vertical stacked cookers. In the horizontal cooker, the action of a specially designed auger mixes conveys the biomass, while the biomass is simultaneously heated with indirect steam from the steam jacket. Water and vapor and air are vented out from the cooker through an upper duct, which may or may not have an exhaust fan depending on the cooker's capacity. For cooking biomass at a high flow rate, several horizontal cookers can be stacked together. In this configuration, the biomass is fed into the top level cooker and heated and conveyed through by the auger and then thrown by gravity into a lower level cooker where the process is repeated. Several levels of horizontal cookers can be stacked together depending on the needed flow rate and the time/temperature of conditioning required. Moisture and temperature can be monitored and adjusted independently for each horizontal cooker level.

For the heat conditioning of microbial biomass, especially microalgal biomass, the optimal time and temperature that the biomass spends in a vertical stacked conditioner can vary depending on the moisture level of the biomass after drying. Heat conditioning (sometimes referred to as "cooking") should not result in burning or scorching significant amounts of the microbial biomass during cooking. Depending on the moisture content of the microbial biomass prior to heat conditioning, i.e., for very low levels of moisture, it may be beneficial or even necessary to moisten the biomass before heat conditioning to avoid burning or scorching. Depending on the type of microbial biomass that is going to be fed through an expeller press, the optimal temperature for heat conditioning will vary. For some types of microalgal, the optimal temperature for heat conditioning is between 93-132 °C (200-270°F). In some embodiments, the microalgal biomass is heat conditioned at 99-110 °C (210-230°F). In other embodiments, the microalgal biomass is heat conditioned at 104-132 °C (220-270°F). In still other embodiments, the microalgal biomass is heat conditioned at 116-127 °C (240-260°F). These temperature ranges are, like moisture content, significantly different from what is typically used in an oilseed conditioning process, as oilseed processes typically use lower conditioning temperatures.

Heating the oil-bearing microbial biomass before pressing can aid in the liberation of oil from and/or accessing the oil-laden compartments of the cells. Oil-bearing microbial biomass contains the oil in compartments made of cellular components such as proteins and phospholipids. Repetitive cycles of heating and cooling can denature the proteins and alter the chemical structure of the cellular components of these oil compartments and thereby provide better access to the oil during the subsequent extraction process. Thus, in various embodiments of the invention, the microbial biomass is conditioned to prepare conditioned feedstock that is used in the pressing step, and the conditioning step involves heating and, optionally, one or more cycles of heating and cooling.

The conditioned feedstock has a moisture content of from 0.5% to 2.5% by weight.

In addition to heating the biomass, conditioning can, in some embodiments, involve the application of pressure to the microbial biomass. To distinguish this type of conditioning from the pressure applied during oil extraction (the pressing step), this type of conditioning is referred to as a "pre-press." The pre-press is conducted at low pressure, a pressure lower than that used for oil extraction in the pressing step. Ordinary high-pressure expeller (screw) presses may be operated at low pressure for this pre-press conditioning step. Pre-pressing the biomass at low pressure may aid in breaking open the cells to allow for better flow of oil during the subsequent high pressure pressing; however, pre-pressing does not cause a significant amount (e.g. more than 5%) of the oil to separate from the microbial biomass. Also, the friction and heat generated during the pre-press may also help break open the oil compartments in the cells. Pre-pressing the biomass at low pressure also changes the texture and particle size of the biomass, because the biomass will extrude out of the press in a pellet-like form. In some embodiments, an extruder (see discussion below) is used to achieve the same or similar results as a low pressure pre-press conditioning step. In some embodiments, the pellets of conditioned biomass are further processed to achieve an optimal particle size for the subsequent full pressure pressing.

Thus, another parameter relevant to optimal extraction of oil from microbial biomass is the particle size. Typically, the optimum particle size for an oil expeller press (screw press) is approximately 0.159 cm (1/16^{th} of an inch) thick. Factors that may affect the range of particle size include, but are not limited to, the method used to dry the microbial biomass and/or the addition of a bulking agent or press aid to the biomass. If the biomass is tray dried, e.g., spread wet onto a tray and then dried in an oven, the resulting dried microbial biomass may need to be broken up into uniform pieces of the optimal particle size to make it optimal for pressing in an expeller press. The same is true if a bulking agent is added to the microbial biomass before the drying process. Thus, conditioning may involve a step that results in altering the particle size or average particle size of the microbial biomass. Machines such as hammer mills or flakers may be employed in accordance with the methods of the invention to adjust the thickness and particle size of the oil-bearing microbial biomass.

In similar fashion, improved oil extraction can result from altering other physical properties of the dried microbial biomass. In particular, the porosity and/or the density of the microbial biomass can affect oil extraction yields. In various embodiments of the methods of the invention, conditioning of the biomass to alter its porosity and/or density is performed. Commonly used prior to hexane or other solvent extraction of oil from oil seeds, expanders and extruders increase the porosity and the bulk density of the feedstock. In accordance with the methods of the present invention, expanders and extruders can be employed to condition the microbial biomass before oil extraction and may or may not cause a significant amount of oil to separate from the microbial biomass. Both expanders and extruders are low-shear machines that heat, homogenize, and shape oil-bearing material into collets or pellets. Expanders and extruders work similarly; both have a worm/collar setup inside a shaft such that, as it moves the material inside the shaft, mechanical pressure and shearing break open the cells. The biggest difference between expanders and extruders is that the expander uses water and/or steam to puff the material at the end of the shaft. The sudden high pressure (and change in pressure) causes the moisture in the material to vaporize, thus "puffing" or expanding the material using the internal moisture. Extruders change the shape of the material, forming collets or pellets. Extruders also lyse the cells and vaporizes water from the biomass (reduction of moisture) while increasing the temperature of the biomass (heating the biomass) through mechanical friction that the extruder exerts on the biomass.. Thus, extruders and expanders can be used in accordance with the methods of the invention to condition the dry microbial biomass. The extruder/expanders can break open the cells, freeing the intracellular lipids, and can also change the porosity and the bulk density of the material. These changes in the physical properties of the feedstock may be advantageous in subsequent oil extraction.

The above-described conditioning methods can be used alone or in combination in accordance with the methods of the invention to achieve the optimal conditioned microbial biomass feedstock for subsequent oil extraction. Thus, the conditioning step involves the application of heat and optionally pressure to the biomass. In various embodiments, the conditioning step comprises heating the biomass at a temperature in the range of 70°C to 150 °C (160°F to 300°F). In various embodiments, the heating is performed using a vertical stacked shaker. In various embodiments, the conditioning step further comprises treating the dry biomass with an expander or extruder to shape and/or homogenize the biomass.

### D. Bulking Agents (Press Aids)

In various embodiments of the invention, a bulking agent or press aid is added to the microbial biomass, which may be either dry or hydrated (i.e., biomass that has not been dried or that contains significant, i.e., more than 6% by weight, moisture, including biomass in fermentation broth that has not been subjected to any process to remove or separate water) microbial biomass or conditioned feedstock, prior to the pressing step. In various embodiments, the bulking agent has an average particle size of less than 1.5 mm. In some embodiments, the bulking agent or press aid has a particle size of between 50 microns and 1.5mm. In other embodiments, the press aid has a particle size of between 150 microns and 350 microns. In some embodiments, the bulking agent is a filter aid. In various embodiments, the bulking agent is selected from the group consisting of cellulose, corn stover, dried rosemary, soybean hulls, spent biomass (biomass of reduced lipid content relative to the biomass from which it was prepared), including spent microbial biomass, sugar cane bagasse, and switchgrass. In various embodiments, the bulking agent is spent microbial biomass (see subsection G below) that contains between 40% and 90% polysaccharide by weight, such as cellulose, hemicellulose, soluble and insoluble fiber, and combinations of these different polysaccharides and/or less than 10% oil by weight. In various embodiments, the polysaccharide in the spent microbial biomass used as a bulking agent contains 20-30 mole percent galactose, 55-65 mole percent glucose, and/or 5-15 mole percent mannose.

Thus, the addition of a press aid or bulking agent may be advantageous in some embodiments of the invention. When there is high oil content and low fiber in the biomass, feeding the biomass through a press can result in an emulsion. This results in low oil yields, because the oil is trapped within the solids. One way in accordance with the methods of the invention to improve the yield in such instances is to add polysaccharide to the biomass in the form of a bulking agent, also known as a "press aid" or "pressing aid". Bulking agents are typically high fiber additives that work by adjusting the total fiber content of the microbial biomass to an optimal range. Microbial biomass such as microalgae and the like typically have very little crude fiber content. Typically, microbial biomass including microalgae biomass have a crude fiber content of less than 2%. The addition of high fiber additives (in the form of a press aid) may help adjust the total fiber content of the microbial biomass to an optimal range for oil extraction using an expeller press. Optimal fiber content for a typical oil seed may range from 10-20%. In accordance with the methods of the present invention, it may be helpful to adjust the fiber content of the microbial biomass for optimal oil extraction. The range for fiber content in the biomass may be the same or a similar range as the optimal fiber content for a typical oil seed, although the optimal fiber content for each microbial biomass may be lower or higher than the optimal fiber content of a typical oil seed. Suitable pressing aids include, but are not limited to, switchgrass, rice straw, sugar beet pulp, sugar cane bagasse, soybean hulls, dry rosemary, cellulose, corn stover, delipidated (either pressed or solvent extracted) cake from soybean, canola, cottonseed, sunflower, jatropha seeds, paper pulp, waste paper and the like. In some embodiments, the spent microbial biomass of reduced lipid content from a previous press is used as a bulking agent. In some applications, especially when the oil is going to be used in a food application or is going to be consumed, the pressing aid used in mixing with the microbial biomass (dry or hydrated) or conditioned feedstock will be selected to meet regulatory requirements (for use as a foodstuff). Thus, bulking agents, when incorporated into a biomass, change the physiochemical properties of the biomass so as to facilitate more uniform application of pressure to cells in the biomass.

In some cases, the bulking agent can be added to the microbial biomass after it has been dried, but not yet conditioned. In such cases, it may advantageous to mix the dry microbial biomass with the desired amount of the press aid and then condition the microbial biomass and the press aid together before feeding to a screw press. In other cases, the press aid can be added to a hydrated microbial biomass before the microbial biomass has been subjected to any separation or dewatering processes, drying, or conditioning. In such cases, the press aid can be added directly to the fermentation broth containing the microbial biomass before any dewatering or other step.

The invention provides various methods relating to the extraction of oil from microbial biomass that employ the bulking agents described above. In one method, hydrated microbial biomass suitable for oil extraction is prepared by adding a bulking agent to the biomass and drying the mixture obtained thereby to a moisture content less than 6% by weight, thereby forming a dried bulking agent/biomass mixture. In another method, oil is extracted from microbial biomass by co-drying hydrated microbial biomass containing at least 20% oil (including at least 40% oil) by weight and a bulking agent to form a dried bulking agent/biomass mixture; reducing the moisture content in the mixture to less than 4% by weight, i.e., by drying and/or conditioning; and pressing the reduced moisture content mixture to extract oil therefrom, thereby forming spent biomass of reduced lipid content. In another method, increased yields of oil are obtained from microbial biomass containing at least 20% lipid by weight by co-drying the microbial biomass with a bulking agent, because the co-dried mixture will, upon pressing, release more oil than can be obtained from the biomass under the same conditions in the absence of a bulking agent. In various embodiments of these and other methods of the invention, the hydrated microbial biomass is contained in fermentation broth that has not been subjected to processes to separate or remove water from the biomass.

In an embodiment, the bulking agent is spent microbial biomass, optionally that has been processed or milled (for homogeneous and ease of blending), that is combined with microbial biomass that has not been extracted. In such cases, the total polysaccharide content of the blended (spent biomass as a press aid and non-extracted microbial biomass) microbial biomass before it is fed into an expeller press contains between 10% and 40% of the total weight of the blended biomass.

### E. Pressing Microbial Biomass

Thus, in accordance with the methods of the invention conditioned feedstock, optionally comprising a bulking agent, is subjected to pressure in a pressing step to extract oil, producing oil separated from the spent biomass. The pressing step involves subjecting pressure sufficient to extract oil from the conditioned feedstock. Thus, in some embodiments, the conditioned feedstock that is pressed in the pressing step comprises oil predominantly or completely encapsulated in cells of the biomass. In other embodiments, the biomass comprises predominantly lysed cells and the oil is thus primarily not encapsulated in cells.

In various embodiments of the different aspects of the invention, the pressing step will involve subjecting the conditioned feedstock to at least 689 bar (10,000 psi) of pressure. In various embodiments, the pressing step involves the application of pressure for a first period of time and then application of a higher pressure for a second period of time. This process may be repeated one or more times ("oscillating pressure"). In various embodiments, more than 5 cycles of oscillating pressure are applied. In various embodiments, one or more of the subsequent cycles may exert an average pressure that is higher than the average pressure exerted in one or more earlier cycles. For example and without limitation, the average pressure in the last cycle can be at least 2-fold higher than the average pressure in the first or any earlier cycle. In various embodiments, moisture content of conditioned feedstock is controlled during the pressing step. In various embodiments, the moisture is controlled in a range of from 0.1% to 3% by weight.

In various embodiments, the pressing step is conducted with an expeller press. In various embodiments, the pressing step is conducted in a continuous flow mode. In various embodiments, the oiling rate is at least 500 g/min. to no more than 1000 g/min. In various continuous flow embodiments, the expeller press is a device comprising a continuously rotating worm shaft within a cage having a feeder at one end and a choke at the opposite end, having openings within the cage is utilized. The conditioned feedstock enters the cage through the feeder, and rotation of the worm shaft advances the feedstock along the cage and applies pressure to the feedstock disposed between the cage and the choke, the pressure releasing oil through the openings of cage and extruding spent biomass from the choke end of the cage. In various embodiments, the cage has an internal length that is between at least ten times to at least 20 times its internal diameter. In various embodiments, the cage comprises a plurality of elongated bars with at least some of the elongated bars separated by one or more spacers, the bars resting on a frame, wherein the one or more spacers between the bars form the openings, and oil is released through the openings to a collecting vessel fluidly coupled with the cage. In various embodiments, the spacers between the elongated bars are of different thicknesses thereby allowing variation of the space between each elongated bar. In various embodiments, either the spacers or the gaps between the bars are from 0.13 to 0.76 mm (0.005 to 0.030 inches) thick.

The cage on some expeller press can be heated using steam or cooled using water depending on the optimal temperature needed for maximum yield. Optimal temperature should be enough heat to aid in pressing, but not too high heat as to burn the biomass while it feeds through the press. The optimal temperature for the cage of the expeller press can vary depending on the microbial biomass that is to be pressed. In some embodiments, for pressing microbial or microalgal biomass, the cage is preheated and held to a temperature of between 93-132 °C (200-270°F). In other embodiments, the optimal cage temperature for microbial or some species of microalgal biomass is between 99-110 °C (210-230°F). In still other embodiments, the optimal cage temperature for microbial or some species of microalgal biomass is between 116-127 °C (240-260°F). These temperature ranges differ significantly from many oilseed pressing processes, and in fact some oilseed pressing processes are referred to as "cold pressing" due to the lack of heating the seeds or the press during the process.

In various embodiments, the pressure increases by a factor of between 10 and 20 from the feeder end to the choke end of the cage. In various embodiments, the pressure along the cage does not increase by more than 100% of the pressure at the feeder end of the cage per linear foot of the cage between the feeder and choke ends of the cage. In various embodiments, the power consumed by the device does not increase by more than 10% when fully loaded with biomass or conditioned feedstock relative to running empty. In various embodiments, the residence time of feedstock in the barrel of the device is no longer than 5-10 min. In various embodiments, either the temperature of the device or the pressure exerted by the device or both are monitored and/or controlled.

In various embodiments, pressure is controlled by adjusting rotational velocity of a worm shaft. In various embodiments, including those in which pressure is not controlled, an expeller (screw) press comprising a worm shaft and a barrel can be used. In various embodiments, the barrel has a length and a channel having a diameter sized to receive the worm shaft, and wherein the barrel length is at least 10 to 15 times greater than the channel diameter. In various embodiments, the barrel of the press has an entrance and an exit and the diameter of the worm shaft increases from the entrance to the exit, and the pressing comprises increasing the pressure from the entrance to the exit of the barrel; in various embodiments, the pressure at the exit is 12 to 16 or even up to 20 times higher than the pressure at the entrance. In various embodiments, the expeller (screw) press comprises a worm shaft and a barrel having a first channel and a second channel, both channels concentric and sized to receive the worm shaft, wherein the first channel has a first diameter and the second channel has a second diameter different than the first diameter. In various embodiments, the conditioned feedstock remains resident in the barrel of the screw press for 5 to 10 minutes.

In various embodiments, the expeller (screw) press comprises a worm shaft disposed in a barrel lined with a plurality of elongate bars separated by one or more spacers therebetween, the spacers creating a gap between the elongate bars. In such a press, pressure can be controlled by adjusting the gap by changing the size or number of spacers between the elongate bars, and/or if the press has a space between an outer surface of the worm shaft and an inner surface of the elongate bars, pressure can be controlled by replacing at least some of the elongate bars with different sized bars so as to change the space. In various embodiments, the press comprises an output aperture and an adjustable choke coupled therewith, and pressure is controlled by adjusting the choke to increase or decrease the pressure. In various embodiments, the expeller (screw) press comprises a worm shaft disposed in a barrel, and pressure is controlled by adjusting a gap between an outer surface of the worm shaft and an inside surface of the barrel.

Expeller presses (screw presses) are routinely used for mechanical extraction of oil from soybeans and oil seeds. Generally, the main sections of an expeller press include an intake, a rotating feeder screw, a cage or barrel, a worm shaft and an oil pan. The expeller press is a continuous cage press, in which pressure is developed by a continuously rotating worm shaft. An extremely high pressure, approximately 689-1379 bar (10,000-20,000 pounds per square inch), is built up in the cage or barrel through the action of the worm working against an adjustable choke, which constricts the discharge of the pressed cake (spent biomass) from the end of the barrel. In various embodiments, screw presses from the following manufacturers are suitable for use : Anderson International Corp. (Cleveland, OH), Alloco (Santa Fe, Argentina), De Smet Rosedowns (Humberside, UK), The Dupps Co. (Germantown, Ohio), Grupo Tecnal (Sao Paulo, Brazil), Insta Pro (Des Moines, Iowa), French Oil Mill (Piqua, OH), Harburg Freudenberger (previously Krupp Extraktionstechnik) (Hamburg, Germany), Maschinenfabrik Reinartz (Neuss, Germany), Shann Consulting (New South Wales, Australia) and SKET (Magdeburg, Germany).

Microbial biomass or conditioned feedstock is supplied to the expeller press via an intake. A rotating feeder screw advances the material supplied from the intake into the barrel where it is then compressed by rotation of the worm shaft. Oil extracted from the material is then collected in an oil pan and then pumped to a storage tank. The remaining spent biomass is then extruded out of the press as a cake and can be collected for additional processing (see subsection G below). The cake may be pelletized.

The worm shaft is associated with a collar setup and is divided into sections. The worm and collar setup within each section is customizable. The worm shaft is responsible for conveying biomass (feedstock) through the press. It may be characterized as having a certain diameter and a thread pitch. Changing shaft diameter and pitch can increase or decrease the pressure and shear stress applied to feedstock as it passes through the press. The collar's purpose is to increase the pressure on the feedstock within the press and also apply a shear stress to the biomass.

The press load in terms of electrical current required to run the press loaded with microbial biomass (conditioned feedstock) is usually not more than about 10% of the electrical current required to run the press empty, and this suggests that the power required to press microbial biomass (conditioned feedstock disclosed herein is lower than other typical power requirements from the oil seed industry where the full press load is greater than 10% of the electrical current required to run the press empty of an oil seed feedstock.

The worm shaft preferably is tapered so that its outer diameter increases along the longitudinal length away from the barrel entrance. This decreases the gap between the worm shaft and the inside of the barrel thus creating greater pressure and shear stress as the biomass travels through the barrel. Additionally, the interior of the barrel is made up of flat steel bars separated by spacers (also referred to as shims), which are set edgewise around the periphery of the barrel, and are held in place by a heavy cradle-type cage. Adjusting the shim between the bars controls the gap between the bars which helps the extracted oil to drain as well as also helping to regulate barrel pressure. The shims are often from 0.00762 cm (0.003") thick to 0.0762 cm (0.030") thick and preferably from 0.0127 cm (0.005") to 0.0508 cm (0.020") thick, although other thicknesses may also be employed. Additionally, the bars may be adjusted, thereby creating sections within the barrel.

As the feed material is pressed or moved down the barrel, significant heat is generated by friction. In some cases, the amount of heat is controlled using a water-jacketed cooling system that surrounds the barrel. Because of the extreme pressure, oil that is pressed from a screw press or expeller press contains a proportion of "foots" or solid material from the biomass that flows out with the oil between the bars. The foots can be screened, drained and fed back into the press along with unpressed feedstock. Temperature sensors may be disposed at various locations around the barrel to monitor and aid in temperature control. Additionally, pressure sensors may also be attached to the barrel at various locations to help monitor and control the pressure.

Various operating characteristics of the expeller (screw) press can be expressed or analyzed as a compression ratio. Compression ratio is the ratio of the volume of material displaced per revolution of the worm shaft at the beginning of the barrel divided by the volume of material displaced per revolution of the worm shaft at the end of the barrel. For example, due to increasing compression ratios the pressure may be 10 to 18 times higher at the end of the barrel as compared with the beginning of the barrel. Internal barrel length may be at least ten times or even thirteen times the internal barrel diameter. Typical compression ratio for a screw or expeller press ranges from 1 to 18, depending on the feed material.

Residence time of the feed material in an expeller (screw) press may affect the amount of oil recovery. Increased residence time in the press gives the feedstock more exposure to the shear stress and pressure generated by the press, which may yield higher oil recovery. Residence time of the feedstock depends on the speed at which the press is run and the length vs. diameter of the screw press (or L/D). The greater the ratio of the length of the shaft to the diameter of the shaft, the longer the residence time of the feedstock (when rotational speed is held at a constant). In some embodiments, the residence time of the algal biomass that is being pressed with an expeller press is no more than 5 to 10 minutes. This residence time for algal biomass is about double the average residence time for other oil seeds such as soybean, canola or cottonseed.

The resulting pressed solids or cake (spent biomass of reduced oil content relative to the feedstock supplied to the screw press) is expelled from the expeller press through the discharge cone at the end of the barrel/shaft. The choke utilizes a hydraulic system to control the exit aperture on the expeller press. A fully optimized oil press operation can extract most of the available oil in the oil-bearing material. For example, optimized conditions for oil extraction from soybeans using an expeller press leaves about 4-6% residual oil; similar yields can be obtained from microbial biomass (conditioned feedstock) in accordance with the methods of the invention. A variety of factors can affect the residual oil content in the pressed cake. These factors include, but are not limited to, the ability of the press to rupture oil-containing cells and cellular compartments and the composition of the oil-bearing material itself, which can have an affinity for the expelled oil. In some cases, the oil-bearing material may have a high affinity for the expelled oil and can absorb the expelled oil back into the material, thereby trapping it. In that event, the oil remaining in the spent biomass can be re-pressed or subjected to solvent extraction, as described herein, to recover the oil.

It is not necessary to use biological agents to extract oil using an expeller press, ie: agents such as enzymes that are produced independently of the microbial biomass. The pressure exerted on the conditioned biomass is the primary mechanism by which oil is released from oil vesicles in the microbial biomass.

### F. Microbial Oil Produced

After the pressing step, the method of the invention results in the extraction of oil and, consequently, the production of extracted oil and spent biomass of reduced oil content relative to the conditioned feedstock supplied to the pressing step. In various embodiments, the released oil contains solid particles of biomass (conditioned feedstock), and the method further comprises separating the released oil from the solid particles.

Contaminants may be present in the oil after pressing (or solvent extraction, see subsection G below, or both). In some embodiments, it may be advantageous to remove these contaminants before subsequent use of the oil (either for food applications or in subsequent chemical reactions, as in the production of fuels). Fines, or small particulates from the biomass, may be present in the extracted oil. Usually, fines are removed through passing the oil through a filter or some other process that physically separates the particulates from the oil. Optionally, the separated solid particles can be subjected to pressure or solvent extraction to extract any remaining oil therefrom.

Degumming is another process suitable for use in the methods of the invention that removes contaminants such as phospholipids from the oil. In some embodiments of the invention, degumming of the extracted oil is combined with refining, bleaching and deodorizing (or RBD). The RBD process eliminates or reduces the odor, color and/or taste of the extracted oil. The refining process usually consists of two steps, degumming and a neutralization step that removes the free fatty acids (FFA) in the oil through caustic stripping with sodium hydroxide. The bleaching step involves mixing the oil with various bleaching clays to absorb color, trace metals and sulfur compounds. The deodorizing step is a distillation process that occurs at low pressure and high temperature. The oil is put under a vaccum and heated with steam to remove any leftover taste or odors and FFAs. Deodorizing can also be achieved by treatment with activated charcoal.

Other methods of removal of contaminants such as heavy metals involve alkai-refining, acid pretreatment and the use of activated clays or zeolites may also be employed in various embodiments of the invention. The oil is mixed at moderate temperatures with small amounts of certain alkaline or ammonia hydroxides and alkaline or ammonia salts in the presence of a phase transfer catalyst. The PROP technology, developed by the Phillips Petroleum Company, combines chemical demetallisation and hydrogenation to remove contaminants from oil. The process involves mixing the oil with an aqueous solution of diammonium phosphate at an elevated temperature in order to reduce the metal content of the oil. This process leads to chemical reactions that form metallic phosphates, which can then be removed from the oil by filtration. Next, the oil is mixed with hydrogen and percolated from a bed of clay and passed over an Ni/Mo catalyst in a hydrogenation reactor. This adsorption step removes the remaining traces of contaminating compounds, such as sulfur, oxygen, chlorine and nitrogen.

In various embodiments, the extracted oil produced by the methods of the invention contains no more than 8 ppm chloride, no more than 2 ppm phosphorus, no more than 26 ppm potassium, no more than 12 ppm sodium, and/or no more than 5 ppm sulfur. The oil produced by the process is useful in a variety of applications, including but not limited to the production of fuels such as biodiesel and renewable diesel (see, e.g., PCT Publication No. 2008/151149 and PCT Application Nos. US09/066141 and US09/066142) and the production of food (see, e.g., PCT Application No. US09/060692).

### G. Spent Biomass Produced

The oil extraction methods of the present invention result in the production of microbial biomass of reduced oil content (spent biomass also referred to as pressed cake or pressed biomass) relative to the conditioned feedstock subjected to pressure in the pressing step. In various embodiments of the present invention, the oil content in the spent biomass of reduced oil content is at least 45 percent less than the oil content of the microbial biomass before the pressing step. In various embodiments, the spent biomass of reduced oil content remaining after the pressing step is pelletized or extruded as a cake. The spent cake, which may be subjected to additional processes, including additional conditioning and pressing or solvent-based extraction methods to extract residual oil in accordance with the invention, is similarly useful in a variety of applications, including but not limited to use as food, particularly for animals, and as a press aid. In various embodiments of the invention, remaining oil is extracted from the spent biomass of reduced oil content; in various embodiments, the extracting is performed by subjecting the spent biomass to pressure or by extracting the oil with an organic solvent.

In some instances, the pressed cake contains a range of from less than 50% oil to less than 1% oil by weight, including, for example, less than 40% oil by weight, less than 20% oil by weight, less than 10%, less than 5% oil by weight, and less than 2% oil by weight. In all cases, the oil content in the pressed cake is less than the oil content in the unpressed material.

In some embodiments, the spent biomass or pressed cake is collected and recycled back into the press with fresh conditioned feedstock or dry biomass as a bulking agent pressing aid. In this case, it may be necessary to condition the spent biomass before or after it is admixed with unpressed feedstock or biomass to make it suitable as a pressing aid. In other embodiments, the spent biomass or pressed cake, which can contain residual oil and other components, i.e., dietary fiber, is suitable for use as human or animal feed or feed additive. In such applications, the spent biomass produced by the methods of the invention may be referred to as "meal" or "delipidated meal."

Thus, spent biomass produced by the methods of the invention is useful as animal feed for farm animals, e.g., ruminants, poultry, swine, and aquaculture. This delipidated meal, as described above, is microbial biomass of reduced lipid/oil content, and can be produced through a mechanical process (e.g., pressing) or through a solvent extraction process (see below), or both. Typically, delipidated meal has less than 15% oil by weight. In a preferred embodiment, the delipidated meal generated from expeller (screw) pressing of microbial biomass followed by solvent extraction, has an oil content of less than 10% by weight. As described above, delipidated meal is suitable for use as a bulking agent (press aid). Addtionally, delipidated meal, although of reduced oil content, still contains high quality proteins, carbohydrates, fiber, minerals, and other nutrients appropriate for an animal feed. Because the cells are predominantly lysed, delipidated meal is easily digested. Delipidated meal can optionally be combined with other ingredients, such as grain, in an animal feed. Because delipidated meal has a powdery consistency, it can be pressed into pellets using an extruder or expanders, which are commercially available.

As noted above, spent biomass, depending on the efficiency of the pressing step, can contain significant amounts of oil. While, in various embodiments, this oil can be extracted by pressing in accordance with the methods of the invention (for example, as when the spent biomass is used as a bulking agent), the spent biomass can also be subjected to solvent extraction to recover more oil from the microbial biomass.

One example of solvent extraction suitable for use in such embodiments of the invention is hexane solvent extraction. In this embodiment, after the oil has been extracted using pressing, the remaining spent biomass is mixed with hexane to extract the remaining oil content. The free oil in the spent microbial biomass forms miscella with the solvent (e.g., hexane) and is separated from the solids (delipidated biomass meal). The oil-solvent miscella is filtered and the solvent is evaporated and recycled for use in future solvent extractions. The delipidated biomass meal can be desolventized and so rendered suitable for use in animal feed or feed additive in accordance with the methods of the invention.

Solvent extraction can recover the free oil that is trapped or reabsorbed in the spent microbial biomass; however, solvent extraction cannot recover oil that is still trapped in unbroken/unlysed microbial cells. Microbial biomass that has been conditioned (and lysed) in an extruder or expander but not subjected to the high pressure of a screw press may also be solvent extracted in order to recover the oil freed from the biomass during the conditioning step. Because the efficiency of solvent extraction depends on the accessibility of the solvent to the free oil, increasing the porosity and/or the surface area of the material for solvent extraction is important. Ideally, for solvent extraction, the spent microbial biomass or pressed cake should contain a high percentage of lysed or broken microbial cells, be of porous texture and increased surface area for solvent extraction, should not be highly compressed or burned, and should not be powdery and dry. In a preferred embodiment, the spent microbial biomass contains at least 85% lysed or broken microbial cells.

Several types of solvent extractors are used in the art and are suitable for use with the spent biomass as described above. In one embodiment, a continuous, percolation solvent extractor is used to extract residual free oil from the spent microbial biomass. Another method of oil extraction suitable for use in accordance with the methods of the invention is the supercritical fluid/carbon dioxide extraction method in which carbon dioxide is liquefied under pressure and heated to the point that it has the properties of both a liquid and a gas. This liquefied fluid then acts as the solvent in extracting the oil from the spent microbial biomass.

Solventless extraction methods known in the art for lipids can also be used for recovering oil from spent biomass in accordance with the methods of the invention. For example, the methods described in US Patent No. 6,750,048 can be used to recover oil from spent biomass produced by the methods of the invention. Another suitable solventless extraction method involves treating the spent biomass with an acid to create a liquid slurry. Optionally, the slurry can be sonicated to ensure the complete lysis of the microalgae cells in the spent biomass. The lysate produced by acid treatment is preferably created at temperatures above room temperature. Such a lysate, upon centrifugation or settling by gravity, can be separated into layers, one of which is an aqueous:lipid layer. Other layers can include a solid pellet, an aqueous layer, and a lipid layer. Lipid can be extracted from the emulsion layer by freeze thawing or otherwise cooling the emulsion. In such methods, it is not necessary to add any organic solvent, although in some embodiments it may be advantageous to do so.

The following section describes microorganisms useful for producing oil-containing microbial biomass suitable for use in the methods of the invention.

### III. MICROORGANISMS USEFUL FOR PRODUCING OIL AND METHODS FOR CULTURING THEM

The present invention arose in part from the discovery that certain microorganisms can be used to produce oil, and hydrocarbon compositions derived therefrom, economically and in large quantities for use in the transportation fuel and petrochemical industry, as well as many other applications. Suitable microorganisms include microalgae, oleaginous bacteria, oleaginous yeast, and fungi. Acidic transesterfication of lipids yields long-chain fatty acid esters useful as a biodiesel. Other enzymatic processes can be applied to lipids derived from these organisms as described herein to yield fatty acids, aldehydes, alcohols, and alkanes. The present invention also provides methods for cultivating microorganisms such as microalgae to achieve increased productivity of lipids and increased lipid yield.

Microorganisms useful in the invention produce oil (lipids or hydrocarbons) suitable for biodiesel production or as feedstock for industrial applications. Suitable hydrocarbons for biodiesel production include triacylglycerides (TAGs) containing long-chain fatty acid molecules. Suitable hydrocarbons for industrial applications, such as manufacturing, include fatty acids, aldehydes, alcohols, and alkanes. In some embodiments, suitable fatty acids, or the corresponding primary alcohols, aldehydes or alkanes, generated by the methods described herein, contain from at least 8 to at least 35 carbon atoms. Long-chain fatty acids for biodiesel generally contain at least 14 carbon or more atoms.

Preferred fatty acids, or the corresponding primary alcohols, aldehydes, and alkanes, for industrial applications contain at least 8 or more carbon atoms. In certain embodiments of the invention, the above fatty acids, as well as the other corresponding hydrocarbon molecules, are saturated (with no carbon-carbon double or triple bonds); mono-unsaturated (single carbon-carbon double bond); or poly-unsaturated (two or more carbon-carbon double bonds); and are linear (not cyclic); and/or have little or no branching in their structures.

Triacylglycerols containing carbon chain lengths in the C8 to C22 range can be produced using the methods of the invention and are preferred for a variety of applications. For surfactants, the preferred TAGs are typically C10-C14. For biodiesel or renewable diesel, the preferred TAGS are typically C16-C18. For jet fuel, the preferred TAGS are typically are C8-C10. For nutrition, the preferred TAGs are C22 polyunsaturated fatty acids (such as, DHA) and carotenoids (such as astaxanthin).

Any species of organism that produces suitable lipid or hydrocarbon can be used in the methods of the invention, although microorganisms that naturally produce high levels of suitable lipid or hydrocarbon are preferred. Production of hydrocarbons by microorganisms is reviewed by Metzger et al., Appl Microbiol Biotechnol (2005) 66: 486-496 and A Look Back at the U.S. Department of Energy's Aquatic Species Program: Biodiesel from Algae, NREL/TP-580-24190, John Sheehan, Terri Dunahay, John Benemann and Paul Roessler (1998).

Considerations affecting the selection of a microorganism for use in the invention include, in addition to production of suitable hydrocarbon for biodiesel or for industrial applications: (1) high lipid content as a percentage of cell weight; (2) ease of growth; and (3) ease of processing. In particular embodiments, the microorganism yields cells that are at least: about 40%, to 60% or more (including more than 70%) lipid when harvested for oil extraction. For certain applications, organisms that grow heterotrophically (on sugar in the absence of light) or can be engineered to do so, are useful in the methods of the invention. See U.S. Patent Application Nos. 60/837,839, 61/118,994, 11/893,364, and 12/194,389, as well as US Patent Application Publication Nos. 20090004715, 20090047721, 20090011480, 20090035842, 20090061493, and 20090148918; PCT Application Nos. 2009/066141 and 2009/066142; and PCT Publication No.2008/151149. For applications in which an organism will be genetically modified, the ease of transformation and availability of selectable markers and promoters, constitutive and/or inducible, that are functional in the microorganism will affect the selection of the organism to be modified.

Naturally occurring microalgae are preferred microorganisms for use in the methods of the invention. Thus, in various preferred embodiments of the present invention, the microorganism producing a lipid - the microorganism from which oil is extracted, recovered, or obtained - is a microalgae. Examples of genera and species of microalgae that can be used in the methods of the present invention include, but are not limited to, the following genera and species microalgae.

**Table 1. Microalgae.**

| |
|---|
| *Achnanthes orientalis, Agmenellum, Amphiprora hyaline*, *Amphora coffeiformis*, *Amphora coffeiformis linea, Amphora coffeiformis punctata, Amphora coffeiformis taylori*, *Amphora coffeiformis tenuis, Amphora delicatissima, Amphora delicatissima capitata*, *Amphora sp., Anabaena, Ankistrodesmus, Ankistrodesmus falcatus, Boekelovia hooglandii, Borodinella sp., Botryococcus braunii*, *Botryococcus sudeticus*, *Bracteoccocus aerius*, *Bracteococcus sp., Bracteacoccus grandis*, *Bracteacoccus cinnabarinas*, *Bracteococcus minor, Bracteococcus edionucleatus*, *Carteria*, *Chaetoceros gracilis*, *Chaetoceros muelleri, Chaetoceros muelleri subsalsum, Chaetoceros sp., Chlorella anitrata, Chlorella Antarctica, Chlorella aureoviridis, Chlorella candida*, *Chlorella capsulate*, *Chlorella esiccate, Chlorella ellipsoidea, Chlorella emersonii, Chlorella fusca, Chlorella fusca var. vacuolata, Chlorella glucotropha, Chlorella infusionum, Chlorella infusionum var. actophila, Chlorella infusionum var. auxenophila, Chlorella kessleri*, *Chlorella lobophora (strain SAG 37.88), Chlorella luteoviridis, Chlorella luteoviridis var. aureoviridis, Chlorella luteoviridis var. lutescens*, *Chlorella miniata, Chlorella cf. minutissima, Chlorella minutissima, Chlorella mutabilis*, *Chlorella nocturna, Chlorella ovalis*, *Chlorella parva*, *Chlorella photophila*, *Chlorella pringsheimii, Chlorella protothecoides (including any of UTEX strains 1806, 411, 264, 256, 255, 250, 249, 31, 29, 25), Chlorella protothecoides var. acidicola, Chlorella regularis, Chlorella regularis var. minima*, *Chlorella regularis var. umbricata, Chlorella reisiglii*, *Chlorella saccharophila*, *Chlorella saccharophila var. ellipsoidea, Chlorella salina*, *Chlorella simplex*, *Chlorella sorokiniana, Chlorella sp., Chlorella sphaerica, Chlorella stigmatophora, Chlorella vanniellii*, *Chlorella vulgaris, Chlorella vulgaris f. tertia, Chlorella vulgaris var. autotrophica, Chlorella vulgaris var. viridis*, *Chlorella vulgaris var. vulgaris, Chlorella vulgaris var. vulgaris f. tertia, Chlorella vulgaris var. vulgaris f. viridis, Chlorella xanthella, Chlorella zofingiensis, Chlorella trebouxioides*, *Chlorella vulgaris, Chlorococcum infusionum, Chlorococcum sp., Chlorogonium*, *Chroomonas sp*., *Chrysosphaera sp., Cricosphaera sp., Crypthecodinium cohnii, Cryptomonas sp., Cyclotella cryptica, Cyclotella meneghiniana, Cyclotella sp., Dunaliella sp., Dunaliella bardawil, Dunaliella bioculata, Dunaliella granulate, Dunaliella maritime, Dunaliella minuta, Dunaliella parva, Dunaliella peircei, Dunaliella primolecta, Dunaliella salina, Dunaliella terricola, Dunaliella tertiolecta, Dunaliella viridis, Dunaliella tertiolecta, Eremosphaera viridis, Eremosphaera sp., Ellipsoidon sp., Euglena, Franceia sp., Fragilaria crotonensis, Fragilaria sp., Gleocapsa sp., Gloeothamnion sp., Hymenomonas sp., Isochrysis aff. galbana, Isochrysis galbana, Lepocinclis, Micractinium, Micractinium (UTEX LB 2614), Monoraphidium minutum*, *Monoraphidium sp., Nannochloris sp., Nannochloropsis salina*, *Nannochloropsis sp., Navicula acceptata, Navicula biskanterae, Navicula pseudotenelloides, Navicula pelliculosa, Navicula saprophila, Navicula sp., Neochloris oleabundans, Nephrochloris sp., Nephroselmis sp., Nitschia communis*, *Nitzschia alexandrina, Nitzschia communis*, *Nitzschia dissipata, Nitzschia frustulum, Nitzschia hantzschiana, Nitzschia inconspicua, Nitzschia intermedia, Nitzschia microcephala, Nitzschia pusilla, Nitzschia pusilla elliptica, Nitzschia pusilla monoensis, Nitzschia quadrangular*, *Nitzschia sp., Ochromonas sp., Oocystis parva, Oocystis pusilla, Oocystis sp., Oscillatoria limnetica, Oscillatoria sp., Oscillatoria subbrevis*, *Parachlorella beijerinckii, Parachlorella kessleri, Pascheria acidophila, Pavlova sp., Phagus, Phormidium*, *Platymonas sp*., *Pleurochrysis carterae*, *Pleurochrysis dentate, Pleurochrysis sp., Prototheca stagnora, Prototheca portoricensis*, *Prototheca moriformis, Prototheca wickerhamii, Prototheca zopfii, Pseudochlorella aquatica, Pyramimonas sp., Pyrobotrys, Sarcinoid chrysophyte, Scenedesmus armatus, Scenedesmus rubescens, Schizochytrium, Spirogyra, Spirulina platensis, Stichococcus sp., Synechococcus sp., Tetraedron*, *Tetraselmis sp., Tetraselmis suecica, Thalassiosira weissflogii*, and *Viridiella fridericiana.* |

In various preferred embodiments of the present invention, the microorganism producing a lipid or a microorganism from which oil can be extracted, recovered, or obtained is an organism of a species of the genus Chlorella. In various preferred embodiments, the microalgae is *Chlorella protothecoides, Chlorella ellipsoidea, Chlorella minutissima, Chlorella zofinienesi, Chlorella luteoviridis, Chlorella kessleri, Chlorella sorokiniana, Chlorella fusca var. vacuolata Chlorella sp., Chlorella cf. minutissima* or *Chlorella emersonii. Chlorella* is a genus of single-celled green algae, belonging to the phylum Chlorophyta. It is spherical in shape, about 2 to 10 µm in diameter, and is without flagella. Some species of *Chlorella* are naturally heterotrophic. *Chlorella,* particularly *Chlorella protothecoides*, is a preferred microorganism for use in the invention because of its high composition of lipid and its ability to grow heterotrophically.

*Chlorella,* preferably, *Chlorella protothecoides, Chlorella minutissima,* or *Chlorella emersonii,* can be genetically engineered to express one or more heterologous genes ("transgenes"). Examples of expression of transgenes in, *e.g., Chlorella,* can be found in the literature (see for example Current Microbiology Vol. 35 (1997), pp. 356-362; Sheng Wu Gong Cheng Xue Bao. 2000 Jul;16(4):443-6; Current Microbiology Vol. 38 (1999), pp. 335-341; Appl Microbiol Biotechnol (2006) 72: 197-205; Marine Biotechnology 4, 63-73, 2002; Current Genetics 39:5, 365-370 (2001); Plant Cell Reports 18:9, 778-780, (1999); Biologia Plantarium 42(2): 209-216, (1999); Plant Pathol. J 21(1): 13-20, (2005)), and such references teach various methods and materials for introducing and expressing genes of interest in such organisms, as the patent applications referenced above. Other lipid-producing microalgae can be engineered as well, including prokaryotic Microalgae (see Kalscheuer et al., Applied Microbiology and Biotechnology, Volume 52, Number 4 / October, 1999), which are suitable for use in the methods of the invention.

Species of *Chlorella* suitable for use in the invention can also be identified by a method that involves amplification of certain target regions of the genome. For example, identification of a specific *Chlorella* species or strain can be achieved through amplification and sequencing of nuclear and/or chloroplast DNA using primers and methodology using any region of the genome, such as, for example, the methods described in Wu et al., Bot. Bull. Acad. Sin. 42:115-121 (2001). Identification of *Chlorella spp.* isolates using ribosomal DNA sequences. Well established methods of phylogenetic analysis, such as amplification and sequencing of ribosomal internal transcribed spacer (ITS1 and ITS2 rDNA), 18S rRNA, and other conserved genomic regions can be used by those skilled in the art to identify species of not only *Chlorella,* but other oil and lipid producing organisms capable of using the methods disclosed herein. For examples of methods of identification and classification of algae see Genetics, 170(4):1601-10 (2005) and RNA, 11(4):361-4 (2005).

Genomic DNA comparison can also be used to identify suitable species of microalgae for use in the methods of the present invention. Regions of conserved DNA, including but not limited to DNA encoding 23S rRNA, can be amplified from microalgal species and compared to consensus sequences to screen for microalgal species that are taxonomically related to a preferred microalgae for use in the methods of the present invention. Similar genomic DNA comparisons can also be used to identify suitable species of oleaginous yeast for use in the methods of the present invention. Regions of conserved genomic DNA, such as, but not limited to conserved genomic sequences between three prime regions of fungal 18S and five prime regions of fungal 26S rRNA genes can be amplified from oleaginous yeast species that may be, for example, taxonomically related to the preferred oleaginous yeast species used in the present invention and compared to the corresponding regions of those preferred species. Example 13 describes genomic sequencing of conserved 3' regions of fungal 18S and 5'regions of fungal 26S rRNA for 48 strains of oleaginous yeasts and the genomic sequences are listed as SEQ ID NOs: 37-76.

In some embodiments, oleaginous yeast preferred for use in the methods of the present invention have genomic DNA sequences encoding for fungal 18S and 26S rRNA genomic sequence with at least 75%, 85% or 95% nucleotide identity to one or more of SEQ ID NOs: 37-76.

In some embodiments, microalgae preferred for use in the methods of the present invention have genomic DNA sequences encoding 23S rRNA that are at least 99%, or at least 95%, or at least 90%, or at least 85% identical to a 23S rRNA sequences of a *Chlorella* species.

*Prototheca* is a genus of single-cell microalgae believed to be a non-photosynthetic mutant of *Chlorella.* While *Chlorella* can obtain its energy through photosynthesis, species of the genus *Prototheca* are obligate heterotrophs. *Prototheca* are spherical in shape, about 2 to 15 micrometers in diameter, and lack flagella. In various preferred embodiments, the microalgae used in the methods of the invention is selected from the following species of *Prototheca: Prototheca stagnora, Prototheca portoricensis, Prototheca moriformis, Prototheca wickerhamii* and *Prototheca zopfii.*

In some embodiments, microalgae preferred for use in the methods of the present invention have genomic DNA sequences encoding 23S rRNA that have at least 99%, or at least 95%, or at least 90%, or at least 85% identical to a 23S rRNA sequence of a *Prototheca* species.

In addition to *Prototheca* and *Chlorella,* other microalgae can be used in accordance with the methods of the present invention. In various preferred embodiments, the microalgae is selected from a genus or species from any of the following genera and species: *Parachlorella kessleri, Parachlorella beijerinckii, Neochloris oleabundans, Bracteacoccus grandis, Bracteacoccus cinnabarinas, Bracteococcus aerius, Bracteococcus sp.* or *Scenedesmus rebescens.* Other non-limiting examples of microalgae (including *Chlorella*) are listed in Table 1, above.

In addition to microalgae, oleaginous yeast can accumulate more than 20% of their dry cell weight as lipid and so are useful in the methods of the invention. In one preferred embodiment of the present invention, a microorganism producing a lipid or a microorganism from which oil can be extracted, recovered, or obtained, is an oleaginous yeast. Examples of oleaginous yeast that can be used in the methods of the present invention include, but are not limited to, the oleaginous yeast listed in Table 2. Illustrative methods for the cultivation of oleaginous yeast (*Yarrowia lipolytica* and *Rhodotorula graminis*) in order to achieve high oil content are provided in the examples below.

**Table 2. Oleaginous Yeast.**

| |
|---|
| *Candida apicola, Candida sp., Cryptococcus curvatus, Cryptococcus terricolus, Debaromyces hansenii, Endomycopsis vernalis*, *Geotrichum carabidarum*, *Geotrichum cucujoidarum*, *Geotrichum histeridarum, Geotrichum silvicola, Geotrichum vulgare, Hyphopichia burtonii, Lipomyces lipofer*, *Lypomyces orentalis*, *Lipomyces starkeyi, Lipomyces tetrasporous*, *Pichia mexicana, Rodosporidium sphaerocarpum, Rhodosporidium toruloides Rhodotorula aurantiaca, Rhodotorula dairenensis, Rhodotorula diffluens*, *Rhodotorula glutinus, Rhodotorula glutinis var. glutinis, Rhodotorula gracilis*, *Rhodotorula graminis Rhodotorula minuta*, *Rhodotorula mucilaginosa*, *Rhodotorula mucilaginosa var. mucilaginosa, Rhodotorula terpenoidalis, Rhodotorula toruloides, Sporobolomyces alborubescens, Starmerella bombicola, Torulaspora delbruekii, Torulaspora pretoriensis*, *Trichosporon behrend*, *Trichosporon brassicae*, *Trichosporon domesticum, Trichosporon laibachii, Trichosporon loubieri, Trichosporon loubieri var. loubieri*, *Trichosporon montevideense*, *Trichosporon pullulans*, *Trichosporon sp.*, *Wickerhamomyces Canadensis*, *Yarrowia lipolytica*, *and Zygoascus meyerae.* |

In one preferred embodiment of the present invention, a microorganism producing a lipid or a microorganism from which a lipid can be extracted, recovered or obtained, is a fungus. Examples of fungi that can be used in the methods of the present invention include, but are not limited to, the fungi listed in Table 3.

**Table 3. Oleaginous Fungi.**

| |
|---|
| *Mortierella*, *Mortierrla vinacea*, *Mortierella alpine*, *Pythium debaryanum*, *Mucor circinelloides*, *Aspergillus ochraceus*, *Aspergillus terreus*, *Pennicillium iilacinum*, *Hensenulo, Chaetomium*, *Cladosporium*, *Malbranchea*, *Rhizopus*, and *Pythium* |

Thus, in one embodiment of the present invention, the microorganism used for the production of microbial biomass for use in the methods of the invention is a fungus. Examples of suitable fungi (*e.g., Mortierella alpine, Mucor circinelloides,* and *Aspergillus ochraceus*) include those that have been shown to be amenable to genetic manipulation, as described in the literature (see, for example, Microbiology, Jul; 153(Pt.7): 2013-25 (2007); Mol Genet Genomics, Jun; 271(5): 595-602 (2004); Curr Genet, Mar;21(3):215-23 (1992); Current Microbiology, 30(2):83-86 (1995); Sakuradani, NISR Research Grant, "Studies of Metabolic Engineering of Useful Lipid-producing Microorganisms" (2004); and PCT/JP2004/012021).

In other embodiments of the present invention, a microorganism producing a lipid or a microorganism from which oil can be extracted, recovered, or obtained is an oleaginous bacterium. Oleaginous bacteria are bacteria that can accumulate more than 20% of their dry cell weight as lipid. Species of oleaginous bacteria for use in the methods of the present invention, include species of the genus *Rhodococcus,* such as *Rhodococcus opacus* and *Rhodococcus sp.* Methods of cultivating oleaginous bacteria, such as *Rhodococcus opacus,* are known in the art (see Waltermann, et al., (2000) Microbiology, 146: 1143-1149). Illustrative methods for cultivating *Rhodococcus opacus* to achieve high oil content are provided in the examples below.

To produce oil-containing microbial biomass suitable for use in the methods of the invention, microorganisms are cultured for production of oil (e.g., hydrocarbons, lipids, fatty acids, aldehydes, alcohols and alkanes). This type of culture is typically first conducted on a small scale and, initially, at least, under conditions in which the starting microorganism can grow. For example, if the starting microorganism is a photoautotroph, the initial culture is conducted in the presence of light. The culture conditions can be changed if the microorganism is evolved or engineered to grow independently of light. Culture for purposes of hydrocarbon production is preferentially conducted on a large scale. Preferably, a fixed carbon source is present in excess. The culture can also be exposed to light some or all of the time, if desired or beneficial.

Microalgae can be cultured in liquid media. The culture can be contained within a bioreactor. Optionally, the bioreactor does not allow light to enter. Alternatively, microalgae can be cultured in photobioreactors that contain a fixed carbon source and allow light to strike the cells. For microalgae cells that can utilize light as an energy source, exposure of those cells to light, even in the presence of a fixed carbon source that the cells transport and utilize (*i*.*e*., mixotrophic growth), nonetheless accelerates growth compared to culturing those cells in the dark. Culture condition parameters can be manipulated to optimize total oil production, the combination of hydrocarbon species produced, and/or production of a particular hydrocarbon species. In some instances, it is preferable to culture cells in the dark, such as, for example, when using extremely large (40,000 liter and higher) fermentors that do not allow light to strike a significant proportion (or any) of the culture.

Microalgal culture medium typically contains components such as a fixed nitrogen source, trace elements, optionally a buffer for pH maintenance, and phosphate. Components in addition to a fixed carbon source, such as acetate or glucose, may include salts such as sodium chloride, particularly for seawater microalgae. Examples of trace elements include zinc, boron, cobalt, copper, manganese, and molybdenum, in, for example, the respective forms of ZnCl₂, H₃BO₃, CoCL₂·6H₂O, CᵤCl₂·2H₂O, MnCl₂·4H₂O and (NH₄)₆Mo₇O₂₄·4H₂O. Other culture parameters can also be manipulated, such as the pH of the culture media, the identity and concentration of trace elements and other media constituents.

For organisms able to grow on a fixed carbon source, the fixed carbon source can be, for example, glucose, fructose, sucrose, galactose, xylose, mannose, rhamnose, N-acetylglucosamine, glycerol, floridoside, glucuronic acid, and/or acetate. The one or more exogenously provided fixed carbon source(s) can be supplied to the culture medium at a concentration of from at least about 50 µM to at least 500 mM, and at various amounts in that range (i.e., 100 µM, 500 µM, 5 mM, 50 mM).

Certain microalgae can be grown in the presence of light. The number of photons striking a culture of such microalgae cells can be manipulated, as well as other parameters such as the wavelength spectrum and ratio of dark:light hours per day. Microalgae can also be cultured in natural light, as well as simultaneous and/or alternating combinations of natural light and artificial light. For example, microalgae of the genus *Chlorella* can be cultured under natural light during daylight hours and under artificial light during night hours.

The gas content of a photobioreactor to grow microorganisms like microalgae can be manipulated. Part of the volume of a photobioreactor can contain gas rather than liquid. Gas inlets can be used to pump gases into the photobioreactor. Any gas can be pumped into a photobioreactor, including air, air/CO₂ mixtures, noble gases such as argon and others. The rate of entry of gas into a photobioreactor can also be manipulated. Increasing gas flow into a photobioreactor increases the turbidity of a culture of microalgae. Placement of ports conveying gases into a photobioreactor can also affect the turbidity of a culture at a given gas flow rate. Air/CO₂ mixtures can be modulated to generate optimal amounts of CO₂ for maximal growth by a particular organism. Microalgae grow significantly faster in the light under, for example, 3% CO₂/97% air than in 100% air. 3% CO₂/97% air has approximately 100-fold more CO₂ than found in air. For example, air:CO₂ mixtures in a range of from about 99.75% air:0.25% CO₂ to 95.00% air:5.0% CO₂ can be infused into a bioreactor or photobioreactor.

Microalgae cultures can also be subjected to mixing using devices such as spinning blades and impellers, rocking of a culture, stir bars, infusion of pressurized gas, and other instruments; such methods can be used to ensure that all cells in a photobioreactor are exposed to light but of course find application with cultures of cells that are not using light as an energy source.

Some microalgae species can grow by utilizing a fixed carbon source, such as glucose or acetate, in the absence of light. Such growth is known as heterotrophic growth. For *Chlorella protothecoides*, for example, heterotrophic growth results in high production of biomass and accumulation of high lipid content. Thus, an alternative to photosynthetic growth and propagation of microorganisms, as described above, is the use of heterotrophic growth and propagation of microorganisms, under conditions in which a fixed carbon source provides energy for growth and lipid accumulation. In some embodiments, the fixed carbon energy source comprises cellulosic material, including depolymerized cellulosic material, a 5-carbon sugar, or a 6-carbon sugar.

Methods for the growth and propagation of *Chlorella protothecoides* to high oil levels as a percentage of dry weight have been reported (see for example Miao and Wu, J. Biotechnology, 2004, 11:85-93 and Miao and Wu, Biosource Technology (2006) 97:841-846, reporting methods for obtaining 55% oil dry cell weight).

PCT Publication WO2008/151149 describes preferred growth conditions for *Chlorella.* Multiple species of *Chlorella* and multiple strains within a species can be grown in the presence of glycerol. The aforementioned patent application describes culture parameters incorporating the use of glycerol for fermentation of multiple genera of microalgae. Multiple *Chlorella* species and strains proliferate very well on not only purified reagent-grade glycerol, but also on acidulated and non-acidulated glycerol byproduct from biodiesel transesterification. In some instances, microalgae, such as *Chlorella* strains, undergo cell division faster in the presence of glycerol than in the presence of glucose. In these instances, two-stage growth processes in which cells are first fed glycerol to increase cell density, and are then fed glucose to accumulate lipids can improve the efficiency with which lipids are produced.

Other feedstocks for culturing microalgae under heterotrophic growth conditions for purposes of the present invention include mixtures of glycerol and glucose, mixtures of glucose and xylose, mixtures of fructose and glucose, sucrose, glucose, fructose, xylose, arabinose, mannose, galactose, acetate, and molasses. Other suitable feedstocks include corn stover, sugar beet pulp, and switchgrass in combination with depolymerization enzymes.

For lipid and oil production, cells, including recombinant cells, are typically fermented in large quantities. The culturing may be in large liquid volumes, such as in suspension cultures as an example. Other examples include starting with a small culture of cells which expand into a large biomass in combination with cell growth and propagation as well as lipid (oil) production. Bioreactors or steel fermentors can be used to accommodate large culture volumes. For these fermentations, use of photosynthetic growth conditions may be impossible or at least impractical and inefficient, so heterotrophic growth conditions may be preferred.

Appropriate nutrient sources for culture in a fermentor for heterotrophic growth conditions include raw materials such as one or more of the following: a fixed carbon source such as glucose, corn starch, depolymerized cellulosic material, sucrose, sugar cane, sugar beet, lactose, milk whey, molasses, or the like; a nitrogen source, such as protein, soybean meal, cornsteep liquor, ammonia (pure or in salt form), nitrate or nitrate salt; and a phosphorus source, such as phosphate salts. Additionally, a fermentor for heterotrophic growth conditions allows for the control of culture conditions such as temperature, pH, oxygen tension, and carbon dioxide levels. Optionally, gaseous components, like oxygen or nitrogen, can be bubbled through a liquid culture. Other starch (glucose) sources include wheat, potato, rice, and sorghum. Other carbon sources include process streams such as technical grade glycerol, black liquor, and organic acids such as acetate, and molasses. Carbon sources can also be provided as a mixture, such as a mixture of sucrose and depolymerized sugar beet pulp.

A fermentor for heterotrophic growth conditions can be used to allow cells to undergo the various phases of their physiological cycle. As an example, an inoculum of lipid-producing cells can be introduced into a medium followed by a lag period (lag phase) before the cells begin to propagate. Following the lag period, the propagation rate increases steadily and enters the log, or exponential, phase. The exponential phase is in turn followed by a slowing of propagation due to decreases in nutrients such as nitrogen, increases in toxic substances, and quorum sensing mechanisms. After this slowing, propagation stops, and the cells enter a stationary phase or steady growth state, depending on the particular environment provided to the cells.

In one heterotrophic culture method useful for purposes of the present invention, microorganisms are cultured using depolymerized cellulosic biomass as a feedstock. As opposed to other feedstocks that can be used to culture microorganisms, such as corn starch or sucrose from sugar cane or sugar beets, cellulosic biomass (depolymerized or otherwise) is not suitable for human consumption. Cellulosic biomass (e.g., stover, such as corn stover) is inexpensive and readily available; however, attempts to use this material as a feedstock for yeast have failed. In particular, such feedstocks have been found to be inhibitory to yeast growth, and yeast cannot use the 5-carbon sugars produced from cellulosic materials (e.g., xylose from hemi-cellulose). By contrast, microalgae can proliferate on depolymerized cellulosic material. Accordingly, the invention contemplates methods of culturing a microalgae under heterotrophic growth conditions in the presence of a cellulosic material and/or a 5-carbon sugar. Cellulosic materials generally include: 40-60% cellulose; 20-40% hemicellulose; and 10-30% lignin.

Suitable cellulosic materials include residues from herbaceous and woody energy crops, as well as agricultural crops, *i.e.,* the plant parts, primarily stalks and leaves typically not removed from the fields with the primary food or fiber product. Examples include agricultural wastes such as sugarcane bagasse, rice hulls, corn fiber (including stalks, leaves, husks, and cobs), wheat straw, rice straw, sugar beet pulp, citrus pulp, citrus peels; forestry wastes such as hardwood and softwood thinnings, and hardwood and softwood residues from timber operations; wood wastes such as saw mill wastes (wood chips, sawdust) and pulp mill waste; urban wastes such as paper fractions of municipal solid waste, urban wood waste and urban green waste such as municipal grass clippings; and wood construction waste. Additional cellulosics include dedicated cellulosic crops such as switchgrass, hybrid poplar wood, and miscanthus, fiber cane, and fiber sorghum. Five-carbon sugars that are produced from such materials include xylose.

Some microbes are able to process cellulosic material and directly utilize cellulosic materials as a carbon source. However, cellulosic material may need to be treated to increase the accessible surface area or for the cellulose to be first broken down as a preparation for microbial utilization as a carbon source. Ways of preparing or pretreating cellulosic material for enzyme digestion are well known in the art. The methods are divided into two main categories: (1) breaking apart the cellulosic material into smaller particles to increase the accessible surface area; and (2) chemically treating the cellulosic material to create a useable substrate for enzyme digestion.

Methods for increasing the accessible surface area include steam explosion, which involves the use of steam at high temperatures to break apart cellulosic materials. Because of the high temperature requirement of this process, some of the sugars in the cellulosic material may be lost, thus reducing the available carbon source for enzyme digestion (see for example, Chahal, D.S. et al., Proceedings of the 2nd World Congress of Chemical Engineering; (1981) and Kaar et al., Biomass and Bioenergy (1998) 14(3): 277-87). Ammonia explosion allows for explosion of cellulosic material at a lower temperature, but is more costly to perform and the ammonia might interfere with subsequent enzyme digestion processes (see for example, Dale, B.E. et al., Biotechnology and Bioengineering (1982); 12: 31-43). Another explosion technique involves the use of supercritical carbon dioxide explosion to break the cellulosic material into smaller fragments (see for example, Zheng et al., Biotechnology Letters (1995); 17(8): 845-850).

Methods for chemically treating the cellulosic material to create useable substrates for enzyme digestion are also known in the art. U.S. Patent No. 7,413,882 describes the use of genetically engineered microbes that secrete beta-glucosidase into the fermentation broth and treating cellulosic material with the fermentation broth to enhance the hydrolysis of cellulosic material into glucose. Cellulosic material can also be treated with strong acids and bases to aid subsequent enzyme digestion. U.S. Patent No. 3,617,431 describes the use of alkaline digestion to breakdown cellulosic materials.

Microorganisms can possess both the ability to utilize an otherwise inedible feedstock, such as cellulosic material or glycerol, as a carbon source (or a pre-treated cellulosic material as a carbon source) and the natural ability to produce edible oils. By utilizing both of these properties, cellulosic material or glycerol, which is normally not part of the human food chain (as opposed to corn glucose and sucrose from sugar cane and sugar beet, which are food compositions suitable for human consumption) can be converted into high nutrition, edible oils, which can provide nutrients and calories as part of the daily human (or animal) diet. In this manner, previously inedible feedstock can be turned into high nutrition edible oils and other food products and food compositions that contain these high nutrition edible oils, as well as oils useful for other purposes.

Bioreactors can be employed for heterotrophic growth and propagation methods. As will be appreciated, provisions made to make light available to the cells in photosynthetic growth methods are unnecessary when using a fixed-carbon source in the heterotrophic growth and propagation methods described herein.

The specific examples of process conditions and heterotrophic growth and propagation methods described herein can be combined in any suitable manner to improve efficiencies of microbial growth and lipid production. For example, microbes having a greater ability to utilize any of the above-described feedstocks for increased proliferation and/or lipid production may be used in the methods of the invention.

Mixotrophic growth involves the use of both light and fixed carbon source(s) as energy sources for cultivating cells. Mixotrophic growth can be conducted in a photobioreactor. Microalgae can be grown and maintained in closed photobioreactors made of different types of transparent or semitransparent material. Such material can include Plexiglass® enclosures, glass enclosures, bags made from substances such as polyethylene, transparent or semi-transparent pipes and other material. Microalgae can be grown and maintained in open photobioreactors such as raceway ponds, settling ponds and other non-enclosed containers. The following discussion of photobioreactors useful for mixotrophic growth conditions is applicable to photosynthetic growth conditions as well.

Photobioreactors can have ports allowing entry of gases, solids, semisolids, and liquids into the chamber containing the microalgae. Ports are usually attached to tubing or other means of conveying substances. Gas ports, for example, convey gases into the culture. Pumping gases into a photobioreactor can serve both to feed cells CO₂ and other gases and to aerate the culture and therefore generate turbidity. The amount of turbidity of a culture varies as the number and position of gas ports is altered. For example, gas ports can be placed along the bottom of a cylindrical polyethylene bag. Microalgae grow faster when CO₂ is added to air and bubbled into a photobioreactor. For example, a 5% CO₂:95% air mixture can be infused into a photobioreactor containing *Botryococcus* cells for such purposes (see for example J Agric Food Chem. 54(13):4593-9 (2006); J Biosci Bioeng. 87(6):811-5 (1999); and J Nat Prod. 66(6):772-8 (2003)).

Photobioreactors can be exposed to one or more light sources to provide microalgae with light as an energy source via light directed to a surface of the photobioreactor. Preferably the light source provides an intensity that is sufficient for the cells to grow, but not so intense as to cause oxidative damage or cause a photoinhibitive response. In some instances a light source has a wavelength range that mimics or approximately mimics the range of the sun. In other instances a different wavelength range is used. Photobioreactors can be placed outdoors or in a greenhouse or other facility that allows sunlight to strike the surface. Preferred photon intensities for species of the genus *Botryococcus* are between 25 and 500 µE m⁻² s⁻¹ (see for example Photosynth Res. 84(1-3):21-7 (2005)).

As noted above, photobioreactors preferably have one or more ports that allow media entry. It is not necessary that only one substance enter or leave a port. For example, a port can be used to flow culture media into the photobioreactor and then later can be used for sampling, gas entry, gas exit, or other purposes. In some instances, a photobioreactor is filled with culture media at the beginning of a culture, and no more growth media is infused after the culture is inoculated. In other words, the microalgal biomass is cultured in an aqueous medium for a period of time during which the microalgae reproduce and increase in number; however, quantities of aqueous culture medium are not flowed through the photobioreactor throughout the time period. Thus in some embodiments, aqueous culture medium is not flowed through the photobioreactor after inoculation.

In other instances, culture media can be flowed though the photobioreactor throughout the time period during which the microalgae reproduce and increase in number. In some embodiments media is infused into the photobioreactor after inoculation but before the cells reach a desired density. In other words, a turbulent flow regime of gas entry and media entry is not maintained for reproduction of microalgae until a desired increase in number of said microalgae has been achieved.

Photobioreactors typically have one or more ports that allow gas entry. Gas can serve to both provide nutrients such as CO₂ as well as to provide turbulence in the culture media. Turbulence can be achieved by placing a gas entry port below the level of the aqueous culture media so that gas entering the photobioreactor bubbles to the surface of the culture. One or more gas exit ports allow gas to escape, thereby preventing pressure buildup in the photobioreactor. Preferably a gas exit port leads to a "one-way" valve that prevents contaminating microorganisms from entering the photobioreactor. In some instances, cells are cultured in a photobioreactor for a period of time during which the microalgae reproduce and increase in number, however a turbulent flow regime with turbulent eddies predominantly throughout the culture media caused by gas entry is not maintained for all of the period of time. In other instances a turbulent flow regime with turbulent eddies predominantly throughout the culture media caused by gas entry can be maintained for all of the period of time during which the microalgae reproduce and increase in number. In some instances a predetermined range of ratios between the scale of the photobioreactor and the scale of eddies is not maintained for the period of time during which the microalgae reproduce and increase in number. In other instances such a range can be maintained.

Photobioreactors typically have at least one port that can be used for sampling the culture. Preferably, a sampling port can be used repeatedly without altering compromising the axenic nature of the culture. A sampling port can be configured with a valve or other device that allows the flow of sample to be stopped and started. Alternatively a sampling port can allow continuous sampling. Photobioreactors also typically have at least one port that allows inoculation of a culture. Such a port can also be used for other purposes such as media or gas entry.

Microorganisms useful in accordance with the methods of the present invention are found in various locations and environments throughout the world. As a consequence of their isolation from other species and their resulting evolutionary divergence, the particular growth medium for optimal growth and generation of oil and/or lipid from any particular species of microbe may need to be experimentally determined. In some cases, certain strains of microorganisms may be unable to grow on a particular growth medium because of the presence of some inhibitory component or the absence of some essential nutritional requirement required by the particular strain of microorganism. There are a variety of methods known in the art for culturing a wide variety of species of microalgae to accumulate high levels of lipid as a percentage of dry cell weight, and methods for determining optimal growth conditions for any species of interest are also known in the art.

Solid and liquid growth media are generally available from a wide variety of sources, and instructions for the preparation of particular media that is suitable for a wide variety of strains of microorganisms can be found, for example, online at http://www.utex.org/, a site maintained by the University of Texas at Austin for its culture collection of algae (UTEX). For example, various fresh water and salt water media include those shown in Table 4.

**Table 4. Algal Media.**

| **Fresh Water Media** | **Salt Water Media** |
|---|---|
| 1/2 CHEV Diatom Medium | 1% F/2 |
| 1/3 CHEV Diatom Medium | 1/2 Enriched Seawater Medium |
| 1/5 CHEV Diatom Medium | 1/2 Erdschreiber Medium |
| 1:1 DYIII/PEA + Gr+ | 1/2 Soil+Seawater Medium |
| 2/3 CHEV Diatom Medium | 1/3 Soil+Seawater Medium |
| 2X CHEV Diatom Medium | 1/4 ERD |
| Ag Diatom Medium | 1/4 Soil+Seawater Medium |
| Allen Medium | 1/5 Soil+Seawater Medium |
| BG11-1 Medium | 2/3 Enriched Seawater Medium |
| Bold 1NV Medium | 20% Allen + 80 % ERD |
| Bold 3N Medium | 2X Erdschreiber's Medium |
| Botryococcus Medium | 2X Soil+Seawater Medium |
| Bristol Medium | 5% F/2 Medium |
| CHEV Diatom Medium | 5/3 Soil+Seawater Agar Medium |
| Chu's Medium | Artificial Seawater Medium |
| CR1 Diatom Medium | BG11-1 + .36% NaCl Medium |
| CR1+ Diatom Medium | BG11-1 + 1% NaCl Medium |
| CR1-S Diatom Medium | Bold 1NV:Erdshreiber (1:1) |
| Cyanidium Medium | Bold INV:Erdshreiber (4:1) |
| Cyanophycean Medium | Bristol-NaCl Medium |
| Desmid Medium | Dasycladales Seawater Medium |
| DYIII Medium | Enriched Seawater Medium |
| Euglena Medium | Erdschreiber's Medium |
| HEPES Medium | ES/10 Enriched Seawater Medium |
| J Medium | ES/2 Enriched Seawater Medium |
| Malt Medium | ES/4 Enriched Seawater Medium |
| MES Medium | F/2 Medium |
| Modified Bold 3N Medium | F/2+NH4 |
| Modified COMBO Medium | LDM Medium |
| N/20 Medium | Modified 2 X CHEV |
| Ochromonas Medium | Modified 2 X CHEV + Soil |
| P49 Medium | Modified Artificial Seawater Medium |
| Polytomella Medium | Modified CHEV |
| Proteose Medium | Porphridium Medium |
| Snow Algae Media | Soil+Seawater Medium |
| Soil Extract Medium | SS Diatom Medium |
| Soilwater: BAR Medium | |
| Soilwater: GR-Medium | |
| Soilwater: GR-/NH4 Medium | |
| Soilwater: GR+ Medium | |
| Soilwater: GR+/NH4 Medium | |
| Soilwater: PEA Medium | |
| Soilwater: Peat Medium | |
| Soilwater: VT Medium | |
| Spirulina Medium | |
| Tap Medium | |
| Trebouxia Medium | |
| Volvocacean Medium | |
| Volvocacean-3N Medium | |
| Volvox Medium | |
| Volvox-Dextrose Medium | |
| Waris Medium | |
| Waris+Soil Extract Medium | |

A medium suitable for culturing *Chlorella protothecoides* comprises Proteose Medium. This medium is suitable for axenic cultures, and a 1L volume of the medium (pH ∼6.8) can be prepared by addition of 1g of proteose peptone to 1 liter of Bristol Medium. Bristol medium comprises 2.94 mM NaNO₃, 0.17 mM CaCl₂·2H₂O, 0.3 mM MgSO₄·7H₂O, 0.43 mM, 1.29 mM KH₂PO₄, and 1.43 mM NaCl in an aqueous solution. For 1.5% agar medium, 15 g of agar can be added to 1 L of the solution. The solution is covered and autoclaved, and then stored at a refrigerated temperature prior to use.

Other suitable media for use with the methods of the invention can be readily identified by consulting the URL identified above, or by consulting other organizations that maintain cultures of microorganisms, SAG the Culture Collection of Algae at the University of Göttingen (Göttingen, Germany), CCAP the culture collection of algae and protozoa managed by the Scottish Association for Marine Science (Scotland, United Kingdom), and CCALA the culture collection of algal laboratory at the Institute of Botany (Třeboň, Czech Republic).

The present methods are particularly suitable for microalgae having a high lipid content (e.g., at least 20% lipids by dry weight). Process conditions can be adjusted to increase the percentage weight of cells that is lipid. For example, in certain embodiments, a microbe (*e.g.*, a microalgae) is cultured in the presence of a limiting concentration of one or more nutrients, such as, for example, nitrogen and/or phosphorous and/or sulfur, while providing an excess of fixed carbon energy such as glucose. Nitrogen limitation tends to increase microbial lipid yield over microbial lipid yield in a culture in which nitrogen is provided in excess. In particular embodiments, the increase in lipid yield is from at least about 10% to 100% to as much as 500% or more. The microbe can be cultured in the presence of a limiting amount of a nutrient for a portion of the total culture period or for the entire period. In particular embodiments, the nutrient concentration is cycled between a limiting concentration and a non-limiting concentration at least twice during the total culture period.

To increase lipid as a percentage of dry cell weight, acetate can be employed in the feedstock for a lipid-producing microbe (*e.g*., a microalgae). Acetate feeds directly into the point of metabolism that initiates fatty acid synthesis (*i*.*e*., acetyl-CoA); thus providing acetate in the culture can increase fatty acid production. Generally, the microbe is cultured in the presence of a sufficient amount of acetate to increase microbial lipid yield, and/or microbial fatty acid yield, specifically, over microbial lipid (*e.g*., fatty acid) yield in the absence of acetate. Acetate feeding is a useful component of the methods provided herein for generating microalgal biomass that has a high percentage of dry cell weight as lipid.

In a steady growth state, the cells accumulate oil (lipid) but do not undergo cell division. In one embodiment of the invention, the growth state is maintained by continuing to provide all components of the original growth media to the cells with the exception of a fixed nitrogen source. Cultivating microalgae cells by feeding all nutrients originally provided to the cells except a fixed nitrogen source, such as through feeding the cells for an extended period of time, can result in a high percentage of dry cell weight being lipid. In some embodiments, the nutrients, such as trace metals, phosphates, and other components, other than a fixed carbon source, can be provided at a much lower concentration than originally provided in the starting fermentation to avoid "overfeeding" the cells with nutrients that will not be used by the cells, thus reducing costs.

In other embodiments, high lipid (oil) biomass can be generated by feeding a fixed carbon source to the cells after all fixed nitrogen has been consumed for extended periods of time, such as from at least 8 to 16 or more days. In some embodiments, cells are allowed to accumulate oil in the presence of a fixed carbon source and in the absence of a fixed nitrogen source for over 30 days. Preferably, microorganisms grown using conditions described herein and known in the art comprise lipid in a range of from at least about 20% lipid by dry cell weight to about 75% lipid by dry cell weight.

Another tool for allowing cells to accumulate a high percentage of dry cell weight as lipid involves feedstock selection. Multiple species of *Chlorella* and multiple strains within a species of *Chlorella* accumulate a higher percentage of dry cell weight as lipid when cultured in the presence of biodiesel glycerol byproduct than when cultured in the presence of equivalent concentrations of pure reagent grade glycerol. Similarly, *Chlorella* can accumulate a higher percentage of dry cell weight as lipid when cultured in the presence of an equal concentration (weight percent) mixture of glycerol and glucose than when cultured in the presence of only glucose.

Another tool for allowing cells to accumulate a high percentage of dry cell weight as lipid involves feedstock selection as well as the timing of addition of certain feedstocks. For example, *Chlorella* can accumulate a higher percentage of dry cell weight as lipid when glycerol is added to a culture for a first period of time, followed by addition of glucose and continued culturing for a second period of time, than when the same quantities of glycerol and glucose are added together at the beginning of the fermentation. See PCT Publication No. 2008/151149.

The lipid (oil) percentage of dry cell weight in microbial lipid production can therefore be improved, at least with respect to certain cells, by the use of certain feedstocks and temporal separation of carbon sources, as well as by holding cells in a heterotrophic growth state in which they accumulate oil but do not undergo cell division. The examples below show growing various microbes, including several strains of microalgae, to accumulate higher levels of lipids as DCW.

In another embodiment, lipid yield is increased by culturing a lipid-producing microbe (*e.g*., microalgae) in the presence of one or more cofactor(s) for a lipid pathway enzyme (*e*.*g*., a fatty acid synthetic enzyme). Generally, the concentration of the cofactor(s) is sufficient to increase microbial lipid (*e*.*g*., fatty acid) yield over microbial lipid yield in the absence of the cofactor(s). In a particular embodiment, the cofactor(s) are provided to the culture by including in the culture a microbe (*e.g*., microalgae) containing an exogenous gene encoding the cofactor(s). Alternatively, cofactor(s) may be provided to a culture by including a microbe (*e.g*., microalgae) containing an exogenous gene that encodes a protein that participates in the synthesis of the cofactor. In certain embodiments, suitable cofactors include any vitamin required by a lipid pathway enzyme, such as, for example: biotin or pantothenate. Genes encoding cofactors suitable for use in the invention or that participate in the synthesis of such cofactors are well known and can be introduced into microbes (*e.g.,* microalgae), using constructs and techniques such as those described herein.

Process conditions can be adjusted to increase the yields of lipids suitable for multiple uses including, but not limited to, biodiesel. Process conditions can also be adjusted to reduce production cost. For example, in certain embodiments, a microbe (*e.g.,* a microalgae) is cultured in the presence of a limiting concentration of one or more nutrients, such as, for example, nitrogen, phosphorus, and/or sulfur. This condition tends to increase microbial lipid yield over microbial lipid yield in a culture in which the nutrient is provided in excess. In particular embodiments, the increase in lipid yield is at least about: 10% 20 to 500%.

Limiting a nutrient may also tend to reduce the amount of biomass produced. Therefore, the limiting concentration is typically one that increases the percentage yield of lipid for a given biomass but does not unduly reduce total biomass. In exemplary embodiments, biomass is reduced by no more than about 5% to 25%. The microbe can be cultured in the presence of a limiting amount of nutrient for a portion of the total culture period or for the entire period. In particular embodiments, the nutrient concentration is cycled between a limiting concentration and a non-limiting concentration at least twice during the total culture period.

The microalgal biomass generated by the culture methods described herein comprises microalgal oil (lipid) as well as other constituents generated by the microorganisms or incorporated by the microorganisms from the culture medium during fermentation.

Microalgal biomass with a high percentage of oil/lipid accumulation by dry weight has been generated using different methods of culture known in the art. Microalgal biomass with a higher percentage of oil/lipid accumulation is useful in with the methods of the present invention. Li et al. describe *Chlorella vulgaris* cultures with up to 56.6% lipid by dry cell weight (DCW) in stationary cultures grown under autotrophic conditions using high iron (Fe) concentrations (Li et al., Bioresource Technology 99(11):4717-22 (2008). Rodolfi et al. describe *Nanochloropsis sp.* and *Chaetoceros calcitrans* cultures with 60% lipid DCW and 39.8% lipid DCW, respectively, grown in a photobioreactor under nitrogen starvation conditions (Rodolfi et al., Biotechnology & Bioengineering (2008) [Jun 18 Epub ahead of print]). Solovchenko et al. describe *Parietochloris incise* cultures with approximately 30% lipid accumulation (DCW) when grown phototropically and under low nitrogen condtions (Solovchenko et al., Journal of Applied Phycology 20:245-251 (2008). *Chlorella protothecoides* can produce up to 55% lipid (DCW) grown under certain heterotrophic conditions with nitrogen starvation (Miao and Wu, Bioresource Technology 97:841-846 (2006). Other *Chlorella* species including *Chlorella emersonii, Chlorella sorokiniana* and *Chlorella minutissima* have been described to have accumulated up to 63% oil (DCW) when grown in stirred tank bioreactors under low-nitrogen media conditions (Illman et al., Enzyme and Microbial Technology 27:631-635 (2000). Still higher percent lipid accumulation by dry cell weight have been reported, including 70% lipid (DCW) accumulation in *Dumaliella tertiolecta* cultures grown in increased NaCl conditions (Takagi et al., Journal of Bioscience and Bioengineering 101(3): 223-226 (2006)) and 75% lipid accumulation in *Botryococcus braunii* cultures (Banerjee et al., Critical Reviews in Biotechnology 22(3): 245-279 (2002)).

These and other aspects and embodiments of the invention are illustrated, but not limited, by the examples below; the examples also highlight the advantages of the methods of the invention. Any examples falling outside the scope of the claimed invention are provided as comparative examples.

### IV. EXAMPLES

### EXAMPLE 1

### Cultivation of Microalgae to Achieve High Oil Content

Microalgae strains were cultivated to achieve a high percentage of oil by dry cell weight. Cryopreserved cells were thawed at room temperature, and 500 µl of cells were added to 4.5 ml of medium (4.2 g/L K₂HPO₄, 3.1 g/L NaH₂PO₄, 0.24 g/L MgSO₄·7H₂O, 0.25 g/L citric acid monohydrate, 0.025 g/L CaCl₂ 2H₂O, 2 g/L yeast extract) plus 2% glucose and grown for 7 days at 28°C with agitation (200 rpm) in a 6-well plate. Dry cell weights were determined by centrifuging 1 ml of culture at 14,000 rpm for 5 minutes in a pre-weighed Eppendorf tube. The culture supernatant was discarded and the resulting cell pellet washed with 1 ml of deionized water. The culture was again centrifuged, the supernatant discarded, and the cell pellets placed at -80°C until frozen. Samples were then lyophilized for 24 hours and dry cell weights were calculated. For determination of total lipid in cultures, 3 ml of culture was removed and subjected to analysis using an Ankom system (Ankom Inc., Macedon, NY) according to the manufacturer's protocol. Samples were subjected to solvent extraction with an Ankom XT10 extractor according to the manufacturer's protocol. Total lipid was determined as the difference in mass between acid hydrolyzed dried samples and solvent extracted, dried samples. Percent oil dry cell weight measurements are shown below in Table 5.

**Table 5. Cultivation of microalgae to achieve high oil content.**

| **Species** | **Strain** | **% Oil** | **SEQ ID NO:** |
|---|---|---|---|
| Chlorella kessleri | UTEX 397 | 39.42 | 4 |
| Chlorella kessleri | UTEX 2229 | 54.07 | 5 |
| Chlorella kessleri | UTEX 398 | 41.67 | 6 |
| Parachlorella kessleri | SAG 11.80 | 37.78 | 7 |
| Parachlorella kessleri | SAG 14.82 | 50.70 | 8 |
| Parachlorella kessleri | SAG 21.11 H9 | 37.92 | 9 |
| Prototheca stagnora | UTEX 327 | 13.14 | 10 |
| Prototheca moriformis | UTEX 1441 | 18.02 | 11 |
| Prototheca moriformis | UTEX 1435 | 27.17 | 12 |
| Chlorella minutissima | UTEX 2341 | 31.39 | 13 |
| Chlorella protothecoides | UTEX 250 | 34.24 | 1 |
| Chlorella protothecoides | UTEX 25 | 40.00 | 2 |
| Chlorella protothecoides | CCAP 211/8D | 47.56 | 3 |
| Chlorella sp. | UTEX 2068 | 45.32 | 14 |
| Chlorella sp. | CCAP 211/92 | 46.51 | 15 |
| Chlorella sorokiniana | SAG 211.40B | 46.67 | 16 |
| Parachlorella beijerinkii | SAG 2046 | 30.98 | 17 |
| Chlorella luteoviridis | SAG 2203 | 37.88 | 18 |
| Chlorella vulgaris | CCAP 211/11K | 35.85 | 19 |
| Chlorella reisiglii | CCAP 11/8 | 31.17 | 20 |
| Chlorella ellipsoidea | CCAP 211/42 | 32.93 | 21 |
| Chlorella saccharophila | CCAP 211/31 | 34.84 | 22 |
| Chlorella saccharophila | CCAP 211/32 | 30.51 | 23 |

### Culturing Chlorella protothecoides to achieve high oil content

Three fermentation processes were performed with three different media formulations with the goal of generating algal biomass with high oil content. The first formulation (Media 1) was based on medium described in Wu et al. (1994 Science in China, vol. 37, No. 3, pp. 326-335) and consisted of per liter: KH₂PO₄, 0.7g; K₂HPO₄, 0.3g; MgSO₄-7H₂O, 0.3g; FeSO₄-7H₂O, 3mg; thiamine hydrochloride, 10 µg; glucose, 20g; glycine, 0.1g; H₃BO₃, 2.9mg; MnCl₂-4H₂O, 1.8mg; ZnSO₄-7H₂O, 220µg; CuSO₄-5H₂O, 80µg; and NaMoO₄-2H₂O, 22.9mg. The second medium (Media 2) was derived from the flask media described in Example 1 and consisted of per liter: K₂HPO₄, 4.2g; NaH₂PO₄, 3.1g; MgSO₄-7H₂O, 0.24g; citric acid monohydrate, 0.25g; calcium chloride dehydrate, 25mg; glucose, 20g; yeast extract, 2g. The third medium (Media 3) was a hybrid and consisted of per liter: K₂HPO₄, 4.2g; NaH₂PO₄, 3.1g; MgSO₄-7H₂O, 0.24g; citric acid monohydrate, 0.25g; calcium chloride dehydrate, 25mg; glucose, 20g; yeast extract, 2g; H₃BO₃, 2.9mg; MnCl₂-4H₂O, 1.8 mg; ZnSO₄-7H₂O, 220µg; CuSO₄-5H₂O, 80µg; and NaMoO₄-2H₂O, 22.9mg. All three media formulations were prepared and autoclave sterilized in lab scale fermentor vessels for 30 minutes at 121°C. Sterile glucose was added to each vessel following cool down post autoclave sterilization.

Inoculum for each fermentor was *Chlorella protothecoides* (UTEX 250), prepared in two flask stages using the medium and temperature conditions of the fermentor inoculated. Each fermentor was inoculated with 10% (v/v) mid-log culture. The three lab scale fermentors were held at 28°C for the duration of the experiment. The microalgal cell growth in Media 1 was also evaluated at a temperature of 23°C. For all fermentor evaluations, pH was maintained at 6.6-6.8, agitations at 500rpm, and airflow at 1 vvm. Fermentation cultures were cultivated for 11 days. Biomass accumulation was measured by optical density at 750 nm and dry cell weight.

Lipid/oil concentration was determined using direct transesterification with standard gas chromatography methods. Briefly, samples of fermentation broth with biomass was blotted onto blotting paper and transferred to centrifuge tubes and dried in a vacuum oven at 65-70°C for 1 hour. When the samples were dried, 2mL of 5% H₂SO₄ in methanol was added to the tubes. The tubes were then heated on a heat block at 65-70°C for 3.5hours, while being vortexed and sonicated intermittently. 2ml of heptane was then added and the tubes were shaken vigorously. 2Ml of 6% K₂CO₃ was added and the tubes were shaken vigorously to mix and then centrifuged at 800rpm for 2 minutes. The supernatant was then transferred to GC vials containing Na₂SO₄ drying agent and ran using standard gas chromatography methods. Percent oil/lipid was based on a dry cell weight basis. The dry cell weights for cells grown using: Media 1 at 23°C was 9.4g/L; Media 1 at 28°C was 1.0g/L, Media 2 at 28°C was 21.2g/L; and Media 3 at 28°C was 21.5g/L. The lipid/oil concentration for cells grown using: Media 1 at 23°C was 3g/L; Media 1 at 28°C was 0.4g/L; Media 2 at 28°C was 18 g/L; and Media 3 at 28°C was 19g/L. The percent oil based on dry cell weight for cells grown using: Media 1 at 23°C was 32%; Media 1 at 28°C was 40%; Media 2 at 28°C was 85%; and Media 3 at 28°C was 88%. The lipid profiles (in area %, after normalizing to the internal standard) for algal biomass generated using the three different media formulations at 28°C are summarized below in Table 6.

**Table 6. Lipid profiles for Chlorella protothecoides grown under different media conditions.**

| | **Media 1 28°C (in Area %)** | **Media 2 28°C (in Area %)** | **Media 3 28°C (in Area %)** |
|---|---|---|---|
| **C14:0** | 1.40 | 0.85 | 0.72 |
| **C16:0** | 8.71 | 7.75 | 7.43 |
| **C16:1** | -- | 0.18 | 0.17 |
| **C17:0** | -- | 0.16 | 0.15 |
| **C17:1** | -- | 0.15 | 0.15 |
| **C18:0** | 3.77 | 3.66 | 4.25 |
| **C18:1** | 73.39 | 72.72 | 73.83 |
| **C18:2** | 11.23 | 12.82 | 11.41 |
| **C18:3 alpha** | 1.50 | 0.90 | 1.02 |
| **C20:0** | -- | 0.33 | 0.37 |
| **C20:1** | -- | 0.10 | 0.39 |
| **C20:1** | -- | 0.25 | -- |
| **C22:0** | -- | 0.13 | 0.11 |

### Culturing Oleaginous Yeast To Achieve High Oil Content

Yeast strain *Rhodotorula glutinis* (DSMZ-DSM 70398) was obtained from the Deutsche Sammlung von Mikroorganismen und Zellkulturen GmbH (German Collection of Microorganism and Cell Culture, Inhoffenstraße 7B, 38124 Braunschweig, Germany. Cryopreserved cells were thawed and added to 50 mL YPD media (described above) with 1x DAS vitamin solution (1000x: 9g/L tricine; 0.67g/L thiamine-HCl; 0.01 g/L d-biotin; 0.008 cyannocobalamin; 0.02 calcium pantothenate; and 0.04 g/L p-Aminobenzoic acid) and grown at 30°C with 200 rpm agitation for 18-24 hours until an OD reading was over 5 OD (A600). The culture was then transferred to 7-L fermentors and switched to YP1 medium (8.5 g/L Difco Yeast Nitrogen Base without Amino Acids and Ammonium Sulfate, 3 g/L Ammonium Sulfate, 4 g/L yeast extract) with 1x DAS vitamin solution. The cultures were sampled twice per day and assayed for OD (A600), dry cell weight (DCW) and lipid concentration. When the cultures reached over 50g/L DCW, the cultures were harvested. Based on dry cell weight, the yeast biomass contained approximately 50% oil. Two samples of yeast biomass were subjected to direct transesterification and analyzed via GC/FID for a lipid profile. The results are expressed in Area Percent, and shown in Table 7, below.

**Table 7. Lipid profile of transesterified yeast biomass samples.**

| | C10:0 | C12:0 | C14:0 | C15:0 | C16:0 | C16:1 | C17:0 | C18:0 | C18:1 | C18:2 | C18:3α | ≥C20 |
|---|---|---|---|---|---|---|---|---|---|---|---|---|
| Sample 1 | 0.03 | 0.21 | 3.36 | 0.25 | 33.26 | 0.76 | 0.20 | 6.88 | 42.68 | 9.28 | 1.33 | 1.1 |
| Sample 2 | 0.02 | 0.10 | 2.18 | 0.12 | 29.94 | 0.49 | 0.16 | 8.17 | 48.12 | 7.88 | 0.84 | 1.45 |

### Cultivation of Rhodococcus opacus to Achieve High Oil Content

A seed culture of *Rhodococcus opacus* PD630 (DSM 44193, Deutsche Sammlung von Mikroorganismen und Zellkuttwen GmbH) was generated using 2ml of a cryo-preserved stock inoculated into 50 ml of MSM media with 4% sucrose (see Schlegel, et al., (1961) Arch Mikrobiol 38, 209-22) in a 250 ml baffle flask. The seed culture was grown at 30°C with 200 rpm agitation until it reached an optical density of 1.16 at 600 nm. 10ml of the seed flask was used to inoculate cultures for lipid production under two different nitrogen conditions: 10mM NH₄Cl and 18.7mM NH₄Cl (each in duplicate). The growth cultures were grown at 30°C with 200 rpm agitation for 6 days. Cells grown in the 10 mM NH₄Cl condition reached a maximal 57.2% (average) lipid by DCW after 6 days of culture. Cells grown in the 18.7 mM NH₄Cl condition reached a maximal 51.8% (average) lipid by DCW after 5 days in culture.

A sample of *Rhodococcus opacus* biomass was subjected to direct transesterification and analyzed via GC/FID for a lipid profile. The results were: C14:0 (2.33); C15:0 (9.08); C16:0 (24.56); C16:1 (11.07); C17:0 (10.50); 2 double bond equivalent (2DBE) C17 species (19.90); C18:0 (2.49); C18:1 (17.41); C18:2 (0.05); C19:0 (0.75) and 2DBE C19 species (1.87).

### EXAMPLE 2

### Diversity of Lipid Chains in Microalgal Species

Lipid samples from a subset of strains grown in Example 1, and listed in Table 5, were analyzed for lipid profile using HPLC. Results are shown below in Table 8.

**Table 8. Diversity of lipid chains in microalgal species.**

| **Microalgal Strain** | **C:14:0** | **C:16:0** | **C:16:1** | **C:18:0** | **C:18:1** | **C:18:2** | **C:18:3** | **C:20:0** | **C:20:1** |
|---|---|---|---|---|---|---|---|---|---|
| *C. protothecoicles (UTEX 250)* | 0.57 | 10.30 | 0 | 3.77 | 70.52 | 14.24 | 1.45 | 0.27 | 0 |
| *C. protothecoides (UTEX 25)* | 0.61 | 8.70 | 0.30 | 2.42 | 71.98 | 14.21 | 1.15 | 0.20 | 0.24 |
| *C. kessleri (UTEX 397)* | 0.68 | 9.82 | 0 | 2.83 | 65.78 | 12.94 | 1.46 | 0 | 0 |
| *C. kessleri (UTEX 2229)* | 1.47 | 21.96 | 0 | 4.35 | 22.64 | 9.58 | 5.2 | 3.88 | 3.3 |
| *Prototheca stagnora (UTEX 327)* | 0 | 12.01 | 0 | 0 | 50.33 | 17.14 | 0 | 0 | 0 |
| *Proto theca moriformis (UTEX 1441)* | 1.41 | 29.44 | 0.70 | 3.05 | 57.72 | 12.37 | 0.97 | 0.33 | 0 |
| *Prototheca moriformis (UTEX 1435)* | 1.09 | 25.77 | 0 | 2.75 | 54.01 | 11.90 | 2.44 | 0 | 0 |

### EXAMPLE 3

### Drum Drying Microalgal Biomass

An F-tank batch of *Chlorella protothecoides* (UTEX 250) (about 4,542 L (about 1,200 gallons)) was used to generate biomass for extraction processes. The batch was allowed to run for approximately 100 hours, while controlling the glucose levels at 16 g/L, after which time the corn syrup feed was terminated. Residual glucose levels dropped to approximately 0 g/L two hours later. The final broth volume was 4,240 L (1,120 gallons). . Both in-process contamination checks and a thorough analysis of a final broth sample failed to show any signs of contamination. The microalgal biomass contained 38% oil based on dry cell weight (DCW).

The microalgal biomass was then dried on an atmospheric double drum dryer. The broth was fed through a nozzle onto the two steam-heated drums that were counter-rotating toward each other. The broth was mechanically spread by the action of the counter-rotating drums into thin split sheets on both hot cylinders. The adhering thin sheets of broth were rapidly dried conductively by the high heat flux of the condensing steam inside the drums. The steam pressure ranged from 3.1 to 7.2 bar (45 to 105 psig) and the drum rotational speed was adjusted to between 2 to 20 rpm. The moisture content of the biomass after drum drying was between 3-10% (by weight).

### Heat-conditioning Microbial Biomass

*Chlorella protothecoides* was produced and drum dried using the methods described in Example 6. Drum dried microalgal biomass was heat conditioned using a 4-deck vertical stacked conditioner (model 424, French Oil Mill Machinery, Piqua, Ohio). Each deck held up to 0.0793 m³ (2.8 cubic feet) of material. The vertical stacked conditioner was preheated using 3.1 to 6.9 bar (45 to 100 psig) steam for about an hour before heat conditioning of the biomass. After pre-heating, the microalgal biomass was loaded onto the top deck of the vertical stacked conditioner and guided to a chute leading to the next deck below by a sweeper arm on each deck, which was mounted to a common vertical shaft powered by an electric motor. A 150 pound load of drum dried microalgal biomass filled the vertical conditioner, and in order to ensure uniform heat-conditioning, the biomass was circulated by opening the vertical stacked conditioner's bottom discharge gate valve and returning the biomass to the top of the vertical conditioner. The biomass temperature in each deck was monitored and controlled between 82 and 121 °C (180 and 250°F) in order to prevent scorching. Heat-conditioning residence times were varied from 10 to 60 minutes to obtain biomass of varying moisture content. The heat-conditioned biomass was unloaded into covered polyethylene carts and immediately pressed in an oilseed press. Samples of biomass before and after heat-conditioning were analyzed for moisture and oil content.

### Oil Extraction from Microalgae using a Benchtop Taby Pressen

Drum dried *Chlorella protothecoides* (UTEX 250) biomass made according to the methods above was dried such that the resulting moisture content was about 5-5.5%. The microalgal biomass contained 48.5% oil based on dry cell weight (DCW). The biomass was fed through a Taby Pressen Type 70 oil press with a 2.2 Hp motor and 70mm screw diameter. The press was preheated to a barrel temperature of 100°C. No oil was extracted under these conditions. Another press run was performed using the same lot of drum-dried biomass with the moisture adjusted down to 0.5% moisture by weight using a forced air oven at 70°C for 30 minutes as a conditioning step prior to feeding it into the press. The press barrel was heated to 82°C and the drum-dried, conditioned microalgal biomass was fed through the bench-top press. Approximately 68% of the available oil (by weight) was recovered and the pressed cake, or spent biomass, was then solvent extracted to recover the residual oil. After multiple experiments with microalgal biomass of varying oil content (between 40-55% oil DCW), the pressed cake had approximately 30% oil as measured by analytical methods every time.

The analytical method for determining percent lipid/oil in a sample was based on a modified Soxlet method. Briefly, 1 gram of sample was weighed out and subjected to acid hydrolysis followed by petroleum ether solvent extraction. Both acid hydrolysis and petroleum either extraction was accelerated by heat using the MARS Microwave accelerated reaction system. The petroleum ether solvent was then evaporated and the amount of extracted lipid was determined gravimetrically.

### Small Scale Solvent Extraction

Pressed cake generated from the bench top press was solvent extracted to recover the residual oil. Excess petroleum ether was added to the pressed cake (5:1 weight by volume) and mixed for a minimum of an hour at room temperature. The petroleum ether mixture was then passed through a Buchner funnel containing a 5 µm filter. The solids were collected from the filter and then subjected to 3 additional washes with petroleum ether of 2x volume each. The filtered petroleum ether mixture and washes were pooled and placed in a RotoVap (2 L) to distill the petroleum ether. The remaining oil was collected and weighed. Upon microscopic inspection of the petroleum ether extracted cake, there was no free oil detected in the cake after petroleum ether extraction. However, lipid vesicles were still seen in intact (unbroken) algae cells. This method will recover 100% of the free oil in the pressed cake, but not in intact algae cells. This small scale solvent extraction process can be used to determine the effectiveness of an expeller press at breaking or cracking the algae cells.

### EXAMPLE 4

### Oil Extraction from Microalgae Using a Lab Scale Komet Press

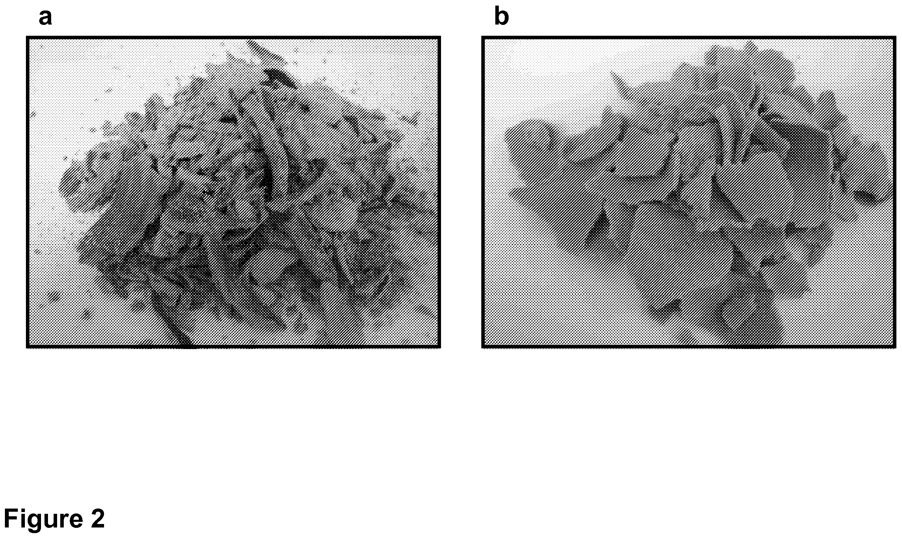

Thirty (30) kilograms of drum dried *Chlorella protothecoides* (UTEX 250) microalgal biomass containing about 48% oil by DCW with moisture content of about 5% was run through a Komet oilseed press with a 65 mm diameter in a pre-press conditioning step with the discharge cone completely disengaged. Under this low pressure pre-press condition, no oil was released; however, the dried microalgal biomass was converted from loose flakes into pre-pressed pellets. Figure 2a shows the drum-dried biomass material that was fed into the press. Figure 2b shows the pre-pressed pellets. The pre-pressed pellets were collected and run through the same press under full press conditions with the discharge cone completely engaged for maximum pressure. The result was a 69% recovery of the oil from the pellets.

Spent cake from the press was then subjected to solvent extraction using iso-hexane in a percolation type extractor. The iso-hexane extraction yielded an additional 1 kg of oil. The combination of the conditioned pressing followed by the hexane extraction recovered a total of 76% of the total available oil from the dried microalgal biomass. These results are summarized in Table 9 below.

**Table 9. Summary of results from Komet press run.**

| | |
|---|---|
| Total Biomass | 30 kilograms |
| Percent Oil (DCW) | 48% |
| Total Available Oil by Weight | 14.4 kilograms |
| Recovered Virgin Oil (Conditioned pressing) | 10 kilograms |
| % Oil Recovery from Pressing | 69% |
| Available Oil by Weight After Pressing | 4.4 kilograms |
| % Oil Recovery from Hexane Extraction | 23% of available oil from the pressed cake (approx. 1 kilogram oil) |
| Total Percentage of Oil Recovered | 76% |

### EXAMPLE 5

### Pilot-scale Pressing of Microalgae using a Press Aid

Microalgal biomass (*Chlorella protothecoides* UTEX 250) containing 38% oil by DCW was dried using a drum dryer with a resulting moisture content of about 3.5% (as measured by a moisture analyzer). An L-250 (8.89 cm) (3.5" diameter) French pilot scale oilseed screw press (French Oil Mill Machinery Company, Piqua, Ohio) was used in the following experiments. The core main barrel (or cage) had a diameter of 8.89 cm (3.5 inches) The screw consisted of alternating worms and collars set up for a compression ratio of 7 to 18. The main drive was powered by a 20 horsepower electric motor and the main shaft rotational speed was 20 rpm. The cage and shaft was preheated to between 82 and 127 °C (180°F and 260°F) by using indirect steam. A cone gap of 0.5 inches, measured between cone bracket and cone mounting plate was used. The cone gap was adjusted by using the 3-position directional valves and the hand pump of the hydraulic cone cylinder.

Dried switchgrass was used as a press aid for microalgae. Drum-dried algal biomass of 38% oil DCW was mixed with switchgrass to form a 20% switchgrass/biomass, 5% switchgrass/biomass, or biomass only samples. All three algal biomass samples were separately heat conditioned in a vertical stacked conditioner (as described above) for 30 minutes at 121°C. The press was heated to a barrel temperature of 93°C prior to the addition of the biomass. Approximately 39.5% of the total available oil (by weight) was recovered from pressing the 20% switchgrass/biomass and also yielded a good quality pressed cake for hexane extraction. Approximately 25% of the total available oil (by weight was recovered from pressing the 5% switchgrass/biomass and also yielded a good quality pressed cake for hexane extraction. The biomass only condition yielded lower oil recovery, about 5% of the total oil (by weight), and the pressed cake was of lower quality for hexane extraction.

Soybean hulls were also used as a press aid with microalgae. Drum-dried algal biomass of 38% oil DCW was mixed with soybean hulls to form a 20% soybean hull/biomass, 10% soybean hull/biomass or biomass only samples. All three algal biomass samples were separately heat conditioned in a vertical stacked conditioner for 30 minutes at 121°C. The press was heated to a barrel temperature of 93°C prior to the addition of the biomass. The 20% soybean hull/biomass mixture did not feed through the press; therefore, no oil was recovered. Approximately 22.5% of the total available oil (by weight) was recovered from pressing the 10% soybean hull/biomass and also yielded a good quality pressed cake for hexane extraction. The biomass only condition yielded no oil and clogged the screw assembly of the expeller press. In the 5%, 20% switchgrass and 10% soybean hulls conditions, there were about 25% solids by weight in the oil recovered. The results are summarized in Table 10 below, which show the total percent oil recovered by weight with the weight of the solids subtracted out.

**Table 10. Pressing microalgae with use of press aids.**

| Press Aid | Percent added | % oil recovered | Quality of pressed cake for solvent extraction |
|---|---|---|---|
| Switchgrass | 0 | ∼5% | Poor |
| Switchgrass | 5% | 24.9% | Good |
| Switchgrass | 20% | 39.5% | Good |
| Soybean hulls | 0 | none | N/A |
| Soybean hulls | 10% | 22.5% | Good |
| Soybean hulls | 20% | none | N/A |

### EXAMPLE 6

### Effects of Moisture Content on Oil Recovery

*Chlorella protothecoides* (UTEX 250) algal biomass of 38% oil DCW was drum dried according to the methods described in Example 3. The moisture content was measured at approximately 3 to 5% and was not conditioned prior to feeding into the screw press. 32.66 Kg (72 pounds) of the biomass was fed into 8.89 cm (3.5") oil seed screw press (French Oil Mill Company, Piqua, OH) preheated to 93 °C (200°F). Heavy footing or solids was observed to be pushed between the bars of the cage throughout all sections of the press. Approximately 5% oil was recovered (after solids were removed). Solvent extraction of the pressed cake recovered an additional 58% of the total available oil (approximately 7 kgs). Analysis of the solvent extracted cake showed that there was approximately 18.6% residual oil. Total recovery (press and solvent extraction) of oil was 62%, indicating that the press only broke or lysed 62% of the microalgal cells. The poor oil recovery (5%) and the heavy footing that was observed indicated that the conditions (e.g., moisture content of the microbial biomass) were not optimal.

A series of tests were performed to establish an optimal range of moisture content for dried microbial biomass that would yield the highest recovery of oil in an expeller press. Microalgal biomass containing 51.3% oil by DCW was dried using a drum dryer. A 8.89 cm (3.5") oilseed press (comparable to the L250 French press) was used and the setup was identical to that described in Example 5. The dried algal biomass was heat-conditioned in a vertical stacked conditioner at 121 °C (250°F). The time of heat-conditioning was varied to achieve the different moisture content levels. The press barrel was pre-heated to and maintained at 93 °C (200°F) during all of the experiments, unless otherwise noted. The heat-conditioned microalgal biomass was introduced slowly into the oilseed press, while monitoring the press electrical load. The feed rate was controlled by a variable-speed screw conveyor. The feed rate was determined by adding the weights of crude oil and pressed cake collected over 2-3 minutes. Samples of each of the pressed cake from the different conditions were analyzed using analytical methods described above to measure lipid/oil content.

A second set of experiments was done with the same lot of drum-dried microalgal biomass as above. One batch of microalgal biomass was conditioned in the vertical stacked conditioner at 93 °C (200°F) and had a final moisture content of 1.2%. Another batch of microalgal biomass was conditioned in the vertical stacked conditioner at 143 °C (290°F) and had a final moisture content of 0.7%. Both batches were pressed in the L250, 8.89 cm (3.5") diameter oilseed press with the barrel temperature at 93 °C (200°F). The batch with the 1.2% moisture content yielded 54.3% oil recovery (by weight), which is comparable to the results from the above batch of 1.2% moisture content biomass, which yielded 48.2% oil recovery. The 0.7% moisture content condition yielded the best results, producing a good quality cake for subsequent solvent extraction and a yield of 73% oil recovery. However, the oil was slightly darker than the oil produced from the biomass that was conditioned at 93 °C (200°F). Table 11 below summarizes the test moisture content and percent oil recovered (after subtracting out the solids) from these two sets of experiments.

**Table 11. Effects of moisture content on oil recovery.**

| Moisture content | % oil recovered | % oil in pressed cake | Oiling rate (g/min) | Quality of pressed cake for solvent extraction |
|---|---|---|---|---|
| 0.2% | 49.1% | 8% | 355 | Poor-burnt; powdery |
| 1% | 66.9% | 29% | 596 | Good |
| 1.2% | 48.2 | 33% | 518 | Good |
| 2.4% | 34.9% | 39% | 277 | Good |
| | | | | |
| 1.2%;conditioning temp 93 °C (200°F) | 54.3% | 32% | 520 | Good |
| 0.7%;conditioning temp 143 °C (290°F) | 73% | 16% | 697 | Good; Oil was slightly darker than 93 °C (200°F) runs |

The results from these two sets of experiments indicated that the optimum moisture content to achieve the highest percent oil recovery and a good quality pressed cake for subsequent solvent extraction in this Example is between 0.7% and 1.2%. This range of moisture content in the microalgal biomass also produced less solids in the oil (less than 25% by weight). Other similar experiments showed that a moisture content below 0.5% resulted in heavy footing (solids) in the oil (over 40% by weight), which impacts overall yield, and produced a burnt, powdery, poor quality cake that was not suitable for subsequent solvent extraction (See Figure 3a). At a moisture content higher than 1.2%, the overall yield of oil was lower, but the pressed cake produced was of very good quality for subsequent solvent extraction (See Figure 3b). This result indicates that moisture content plays a major role in the quality and texture of the pressed cake from microbial biomass, and there is an inhibition of percent oil yield if the moisture content is too high.

### EXAMPLE 7

### Prototheca in Lab Press with Lipid Profile of Pressed, Extracted, and Combined Materials

Approximately 2 kg of drum-dried *Prototheca moriformis* algal biomass with 60% lipid DCW was pressed using a bench-top Taby Type 70 press (see Example 8 above for press conditions). The dried biomass was heat conditioned in a forced air oven at 100 °C (212°F) for 30 minutes. The press was preheated to 93 °C (200°F) and 235.5 grams of oil was recovered (19%), after solids were removed. The oil was collected and analyzed for lipid profile (expressed as area%), chlorophyll and carotenoid content using standard methods. The residual oil in the pressed cake was recovered via a batch extraction with petroleum ether (as described in Example 3). A total of 311 grams of oil was extracted out of 961 grams of pressed cake. The extracted oil was also analyzed for lipid profile (expressed as area %), chlorophyll and carotenoids, which are summarized below. Overall, the lipid profile, chlorophyll and carotenoid content from pressed oil and solvent extracted oil were very similar.

**Table 12. Lipid profile of oil extracted from Prototheca moriformis.**

| | Pressed oil (Area %) | Solvent extracted oil (Area %) |
|---|---|---|
| C12:0 | 0.05 | 0.05 |
| C14:0 | 1.36 | 1.37 |
| C14:1 | 0.02 | 0.02 |
| C15:0 | 0.04 | 0.04 |
| C16:0 | 19.90 | 20.11 |
| C16:1 | 0.85 | 0.85 |
| C18:0 | 4.11 | 4.15 |
| C18:1 | 64.81 | 64.56 |
| C18:2 | 7.83 | 7.83 |
| C20:0 | 0.03 | 0.03 |

**Table 13. Carotenoid and chlorophyll content in oil extracted from Prototheca morifomis.**

| | Pressed oil (mcg/ml) | Solvent extracted oil (mcg/ml) |
|---|---|---|
| cis-Lutein | 0.041 | 0.042 |
| trans-Lutein | 0.140 | 0.112 |
| trans-Zeaxanthin | 0.045 | 0.039 |
| cis-Zeaxanthin | 0.007 | 0.013 |
| t-alpha-Cryptoxanthin | 0.007 | 0.010 |
| t-beta-Cryptoxanthin | 0.009 | 0.010 |
| t-alpha-Carotene | 0.003 | 0.001 |
| c-alpha-Carotene | none detected | none detected |
| t-beta-Carotene | 0.010 | 0.009 |
| 9-cis-beta-Carotene | 0.004 | 0.002 |
| Lycopene | none detected | none detected |
| Total Carotenoids | 0.267 | 0.238 |
| Chlorophyll | <0.01 mg/kg | <0.01 mg/kg |

Additionally, elemental analysis was also performed on pressed oil and solvent extracted oil from *Prototheca moriformis* using ICP mass spectrometry. The results are summarized below in Table 14.

**Table 14. Elemental analysis of oil extracted from Prototheca moriformis.**

| | Pressed oil (ppm) | Solvent extracted oil (ppm) |
|---|---|---|
| Lithium | <2 | <2 |
| Beryllium | <2 | <2 |
| Boron | <2 | <2 |
| Sodium | 12 | 12 |
| Magnesium | <2 | 2 |
| Aluminum | 8 | 8 |
| Phosphorus | <2 | <2 |
| Potassium | 12 | 26 |
| Calcium | 11 | <2 |
| Scandium | <2 | <2 |
| Titanium | <2 | <2 |
| Vanadium | <2 | <2 |
| Chromium | <2 | <2 |
| Manganese | <2 | <2 |
| Iron | <2 | <2 |
| Cobalt | <2 | <2 |
| Nickel | <2 | <2 |
| Copper | <2 | <2 |
| Zinc | <2 | <2 |
| Gallium | <2 | <2 |
| Germanium | <2 | <2 |
| Arsenic | <2 | <2 |
| Selenium | <2 | <2 |
| Rubidium | <2 | <2 |
| Strontium | <2 | <2 |
| Yttrium | <2 | <2 |
| Zirconium | <2 | <2 |
| Niobium | <2 | <2 |
| Molybdenum | <2 | <2 |
| Ruthenium | <2 | <2 |
| Rhodium | <2 | <2 |
| Palladium | <2 | <2 |
| Silver | <2 | <2 |
| Cadmium | <2 | <2 |
| Indium | <2 | <2 |
| Tin | <2 | <2 |
| Antimony | <2 | <2 |
| Tellurium | <2 | <2 |
| Cesium | <2 | <2 |
| Barium | <2 | <2 |
| Lanthanum | <2 | <2 |
| Cerium | <2 | <2 |
| Praseodymium | <2 | <2 |
| Neodymium | <2 | <2 |
| Samarium | <2 | <2 |
| Europium | <2 | <2 |
| Gadolinium | <2 | <2 |
| Terbium | <2 | <2 |
| Dysprosium | <2 | <2 |
| Holmium | <2 | <2 |
| Erbium | <2 | <2 |
| Thulium | <2 | <2 |
| Ytterbium | <2 | <2 |
| Lutetium | <2 | <2 |
| Hafnium | <2 | <2 |
| Tantalum | <2 | <2 |
| Tungsten | <2 | <2 |
| Rhenium | <2 | <2 |
| Iridium | <2 | <2 |
| Platinum | <2 | <2 |
| Gold | <2 | <2 |
| Thallium | <2 | <2 |
| Lead | <2 | <2 |
| Bismuth | <2 | <2 |
| Thorium | <2 | <2 |
| Uranium | <2 | <2 |
| Iodine | 28 | <4 |
| Sulfur (ICP) | <5 | <5 |
| Mercury | <2 | <2 |
| Chloride | <8 | <6 |

### EXAMPLE 8

### Pilot Scale Pressing of Prototheca moriformis

*Prototheca moriformis* (UTEX 1435) containing approximately 66% oil (by dry cell weight) was drum dried using the methods described in Example 3. After drum drying, the moisture content of the biomass was about 2.7%. The dried biomass was then heat-conditioned using methods described in Example 3. The moisture content of the biomass after heat-conditioning was approximately 0.6-1.4%. The algal biomass was then fed into 8.89 cm (3.5") oil seed screw press (French Oil Mill Company, Piqua OH) with the cage preheated to 91-104 °C (195-220°F). Heavy footing was observed to be pushed between the bars of the cage, indicating that the conditions were not optimal. Approximately 47.9% oil (based on weight and theoretical calculation of available oil in the biomass) was recovered with fines (solids). The solids were removed by centrifugation and the total oil yield was 31.9% after clarification. Analysis of the pressed cake showed that there was approximately 22% (by weight of the pressed cake) residual oil. Another batch of biomass that was similarly prepared was also run through the press with similar yield in oil recovery (57.3% including solids in the oil).

### EXAMPLE 9

### Use of Press Aids with Oil Extraction from Prototheca moriformis

A series of tests were performed with different press aids on the lab-scale bench top Taby press in order to see if the addition of a press aid can increase oil yields. Different press aids were added to the fermentation broth after harvesting the biomass. The press aid/biomass were then dried together on a drum dryer and then heat-conditioned. This press aid/biomass mixture was then fed into a lab-scale Taby press under conditions described above in Example 3. A condition with no added press aid was used as a negative control.

For the negative control, 3L of fermentation broth containing biomass with 63.4% oil was dried on a drum dryer. The dried biomass was fed through the bench top press and yielded minimal amount of oil. The next condition was 3L of fermentation broth with 150g (5% by wet weight of the fermentation broth) cellulosic filter aid (PB20, EP Minerals, Nevada). The mixture was then dried on a drum dryer and had a moisture content of 2.25% after drum drying. The cellulose/biomass was then heat conditioned in a 110°C oven for 20-30 minutes, to a final moisture content of 1.2%. This conditioned cellulose/biomass was then fed into the lab scale press. Based on theoretical calculations of available oil, there was approximately 145grams of available oil in the biomass. Approximately 148 grams of oil was recovered using the lab scale press, making total oil recovery about 100%.

The next conditioned tested was the addition of 5% coarse-ground soy hulls (by wet weight). 150 grams of soy hulls was mixed with 3L of fermentation broth containing *Prototheca moriformis* biomass. During the mixing process, it was observed that the coarse-ground soy hulls tended to settle out of solution, so constant mixing was required. The mixture was then dried on the drum dryer and had a moisture content of 6.5% after drum drying. The dried soyhull/biomass was then heat conditioned in a 110°C oven for 20-30 minutes, to a final moisture content of 2.5%. The heat-conditioned soyhull/biomass was then fed through the lab scale screw press. Out of the calculated 162 grams of available oil, 46 grams of oil was recovered from the lab scale screw press, making total oil recovery at 28%. These experiments were repeated with another lot of *Prototheca moriformis* fermentation broth and 2% coarse ground soyhulls added, 1% soyhulls added, 2% cellulose added and 1% cellulose added. A negative control with just drum dried fermentation broth was also tested. Minimal amount of oil was recovered from the negative control. From the 2% soyhull condition, 42% of the available oil was recovered and 30% of the available oil was recovered from the 1% soyhull condition. From the 2% cellulose condition, 40% of the available oil was recovered and 10% of the available oil was recovered from the 1% cellulose condition.

Additional experiments were performed using finely ground soy hulls and dry back addition to the fermentation broth as press aids. Because the course ground soy hulls have the tendency to settle out, the course ground soy hulls were finely ground using a coffee grinder on the finest setting. After grinding, the finely ground soy hulls had a powdery texture. Dryback (2x pressed cake of *Prototheca moriformis* biomass having 5% oil content) was also ground up for this experiment. The control condition was *Prototheca moriformis* biomass with approximately 60% oil, without the addition of press aid. 150 grams of either finely ground soy hulls (5%) or ground dry back (5%) was added to 3 L of fermentation broth containing *Prototheca moriformis* biomass. The algal biomass and press aid mixture was then dried on a drum dryer. The control (*Prototheca moriformis* biomass only) condition had a moisture content of 4% after drum drying, the 5% finely ground soy hull condition had a moisture content of 2.3% after drum drying, and the 5% dry back condition had a moisture content of 2.5% after drum drying. Each of the biomass was then dried in a 110°C oven for 20-30 minutes in order to heat condition the biomass. The final moisture content for the control biomass was 2%, the final moisture content for the 5% finely ground soy hull addition was 1.3% and the final moisture content for the 5% dry back addition was 1.01%. After heat conditioning, each of the biomass was then fed through a lab-scale Taby screw press under conditions described above in Example 3. The extracted oil and the pressed cake was collected and analyzed for an estimated yield. For the control condition, 6.7grams of oil was collected (after removal of solids from the oil), for an approximate yield of 2.8%. Heavy footing was observed through out the press and the pressed cake clogged the discharge end of the press. In the 5% finely ground soy hull added condition, 148.2 grams of oil was collected (after removal of solids from the oil), for an approximate yield of 79.2% recovery. There was minimal amount of footing during pressing and very low amount of solids were in the pressed oil before clarification. In the 5% dryback added condition, 195.9g of oil was collected (after removal of solids from the oil), for an approximate yield of 54.6%. There was minimal amount of footing during pressing and very small amount of solids were in the pressed oil before clarification. These results are consistent with the above results, where the addition of press aids to the fermentation broth (containing microalgae biomass) followed by co-drying on a drum drier produced biomass that had an increased oil yield when pressed on a screw press as compared to microalgal biomass with no press aid added.

### Pilot Scale Pressing of Prototheca moriformis Using a Press Aid

Pilot plant scale trial were performed to evaluate ground soy hulls as press aids when dry mixing with drum dried *Prototheca moriformis* microalgal biomass. Ground soy hulls were mixed with drum dried biomass at 10 and 20% w/w based on finished weight of the mix. Then the microalgal biomass/soy hull mix was heat conditioned in a French 424 vertical stacked conditioner before being pressed in a 8.89 cm (3.5") screw press (French Oil Mill Company, Piqua, OH). Oil and pressed cake were recovered and weighed to estimate yields.

A control batch of *Prototheca moriformis* biomass was prepared in a similar manner but without the inclusion of soyhulls. The control batch had an oil content of 52% (DCW) and a moisture content of 2.57% after drum drying. 31.75 kg (70 pounds) of the control batch was heat conditioned for 30 minutes in a vertical stacked conditioner at 91-106 °C (195-223°F) and the moisture content was reduced to 0.81-0.95%. 32.66 kg (72 pounds) of 10% soy hulls added biomass with an initial moisture content of 3.30% was conditioned at 91-106.4 °C (195-223.5°F) for 30 minutes and the moisture content was reduced to 1.06%. 31.75 kg (70 pounds) of the 20% soy hulls added biomass with an initial moisture content of 3.07% was heat conditioned at 98-108 °C (208-227°F) for 30 minutes and the moisture content was reduced to 1.47%. The heat conditioned biomass was then fed into the screw press. In the control batch, 13.61 kg (30 pounds) (out of the 31.75 kg (70 pounds) that was heat conditioned) was fed through the press before the press clogged. Approximately 1.81 kg (4.0 pounds) of oil was recovered (including solids) from the 13.61 kg (30 pounds) of biomass that was fed through, making the yield approximately 20.5%. In the 10% soy hull condition, 27.67 kg (61 pounds) (out of the 32.66 kg (72 pounds) that was heat conditioned) was fed through the press and approximately 3.18 kg (7.0 pounds) of oil was recovered (including solids), making the yield approximately 20%. In the 20% soy hulls test, all 31.75 kg (70 pounds) of the heat conditioned material was fed through the press, but minimal (unmeasured) amounts of oil was recovered.

With the success of the lab scale press experiments described above, the addition of press aids to the fermentation broth after harvesting the algal biomass was scaled up for a pilot scale oil screw press (8.89 cm (3.5") oil seed screw press (French Oil Mill Company, Piqua, OH)). *Prototheca moriformis* biomass was prepared under three different experimental conditions: a negative control with no cellulose (PB20) added, biomass with 25g/L cellulose added to the fermentation broth after harvesting, and biomass with 50g/L cellulose added to the fermentation broth after harvesting. Biomass from all three conditions was dried on a drum dryer. The negative control biomass had about 58% oil content (DCW) and a moisture content of 6.68%. 63.50 kg (140 pounds) of the negative control biomass was then conditioned in a vertical stacked conditioner at 107 °C (225°F) for 45 minutes and the moisture content after heat-conditioning was 2.5-3.5% The 25g/L cellulose added biomass had a moisture content of 5.30% after drum drying. 90.72 kg (200 pounds) of the 25g/L cellulose added biomass was then conditioned in a vertical stacked conditioner at 93 °C (200°F) for 45 minutes and the moisture content after heat-conditioning was 2.5-3.5%. The 50g/L cellulose added biomass had a moisture content of 4.35% after drum drying. 52.16 kg (115 pounds) of the 50g/L cellulose added biomass was then conditioned in a vertical stacked conditioner at 93 °C (200°F) for 45 minutes and the moisture content after heat-conditioning was 2.5-3.5%. Biomass from each experimental condition was then fed through the 8.89 cm (3.5") oil seed press. In the negative control condition, 14.65 kg (32.3 pounds) of oil (including solids or fines in the oil) was recovered. The pressed cake from the negative control condition was analyzed for residual oil content and the cake contained 42-52% (by weight) residual oil. In the 25g/L cellulose condition, 39.73 kg (87.6 pounds) of oil (including solids in the oil) was recovered. The pressed cake was analyzed for residual oil content and the cake contained 10-11% (by weight) residual oil. No oil was recovered from the 50g/L cellulose added condition. The biomass did not feed through the press and clogged the press after 5 minutes.

The results from this experiment were consistent with the results from the lab-scale bench top press. Although a modest amount of oil was recovered from the negative control condition, there was still a significant amount of residual oil left in the pressed cake. The 25g/L cellulose condition performed the best, yielding the most about of oil with the least amount of residual oil in the pressed cake. The 50g/L cellulose condition failed to yield any oil and clogged the press after 5 minutes of running. These results showed that the addition of a press-aid to the fermentation broth of the harvested microalgal biomass and the co-dried can increase the oil yield when pressed in an oil seed screw press.

### Pilot Scale Two-Step Full Press of Prototheca moriformis

Two-step full press of *Prototheca moriformis* biomass was undertaken whereby dried, conditioned *Prototheca moriformis* biomass was pressed in an expeller press and the spent biomass was then conditioned for a second time and pressed in an expeller press for a second time.

*Prototheca moriformis* (UTEX 1435) containing approximately 62% oil (by dry cell weight) was drum dried using the methods described above in Example 3. After drum drying, the moisture content of the biomass was about 2.7%. The dried biomass was then heat-conditioned using methods described in Example 3. The moisture content of the biomass after heat-conditioning was approximately 1.7-2.1%.

A first pass pressing was performed on the heat-conditioned biomass using a 8.89 cm (3.5") oil seed screw press (French Oil Mill Company, Piqua, OH) with the cage preheated to 90-104 °C (194-220°F). Approximately 77.1% of the oil (by weight) was recovered from the microalgal biomass during this first pass. Analysis of the pressed cake showed that there was about 21% by weight residual oil.

The pressed cake was heat-conditioned a second time so that the pressed cake was about 74-92 °C (166-197°F). The moisture content of the heat-conditioned pressed cake was approximately 1.8%. The heat-conditioned cake was then fed into the press with the cage preheated to 82-113 °C (180-235°F). Approximately 72.5% of the oil in the cake was recovered in this second pass, based on the weight of the oil recovered in this second, the pressed cake from the second press and the calculated available oil in the pressed cake after the first pass through the screw press. By adding the oil recovered in both passes through the press, the total oil recovery achieved with both passes was approximately 92.9%.

### Monosaccharide Composition of Delipidated Prototheca moriformis biomass

High oil *Prototheca moriformis* biomass was then harvested and dried using a drum dryer. The dried algal biomass was lysed and the oil extracted using an expeller press as described herein. The residual oil in the pressed biomass was then solvent extracted using petroleum ether. Residual petroleum ether was evaporated from the delipidated meal using a Rotovapor (Buchi Labortechnik AG, Switzerland). Glycosyl (monosaccharide) composition analysis was then performed on the delipidated meal using combined gas chromatography/mass spectrometry (GC/MS) of the per-O-trimethylsily (TMS) derivatives of the monosaccharide methyl glycosides produced from the sample by acidic methanolysis. A sample of delipidated meal was subjected to methanolysis in 1M HCl in methanol at 80°C for approximately 20 hours, followed by re-N-acetylation with pyridine and acetic anhydride in methanol (for detection of amino sugars). The samples were then per-O-trimethylsiylated by treatment with Tri-Sil (Pierce) at 80°C for 30 minutes (see methods in Merkle and Poppe (1994) Methods Enzymol. 230: 1-15 and York et al., (1985) Methods Enzymol. 118:3-40). GC/MS analysis of the TMS methyl glycosides was performed on an HP 6890 GC interfaced to a 5975b MSD, using a All Tech EC-1 fused silica capillary column (30m x 0.25 mm ID). The monosaccharides were identified by their retention times in comparison to standards, and the carbohydrate character of these are authenticated by their mass spectra. 20 micrograms per sample of inositol was added to the sample before derivatization as an internal standard. The monosaccharide profile of the delipidated *Prototheca moriformis* (UTEX 1435) biomass is summarized in Table 15 below.

**Table 15. Monosaccharide (glycosyl) composition analysis of Prototheca moriformis (UTEX 1435) delipidated biomass.**

| | **Mass (µg)** | **Mole % (of total carbohydrate)** |
|---|---|---|
| Arabinose | 0.6 | 1.2 |
| Xylose | n.d. | n.d. |
| Galacturonic acid (GalUA) | n.d. | n.d. |
| Mannose | 6.9 | 11.9 |
| Galactose | 14.5 | 25.2 |
| Glucose | 35.5 | 61.7 |
| N Acetyl Galactosamine (GalNAc) | n.d. | n.d. |
| N Acetyl Glucosamine (GlcNAc) | n.d. | n.d. |
| Heptose | n.d. | n.d. |
| 3 Deoxy-2-manno-2 Octulsonic acid (KDO) | n.d. | n.d. |
| **Sum** | **57** | **100** |

| | | |
|---|---|---|
| n.d. = none detected | | |

Two samples of delipidated *Prototheca moriformis* (UTEX 1435) biomass was also analyzed for dietary fiber content using AOAC Method 991.43. The dietary fiber content for the two samples was 22.89% and 33.06%.

### EXAMPLE 10

### Solvent Extraction of Pressed Cake from Microalgal Biomass

To maximize the total oil yield from the microalgal biomass, the pressed cake (as described in the previous Examples) was subjected to solvent extraction using a drum batch-type extractor and commercial hexane as the solvent. The pressed biomass was mixed with the commercial hexane in the extractor. Extraction of oil was performed in the drum extractor by washing the pressed cake three times with commercial hexane using a solvent to solids ratio of between 0.7:1 to 2:1. The temperature of the extractor was held at between 50 to 55 °C (122°F to 131°F), for a residence time of 1 hour for each wash and a slight vacuum of 2.54 to 5.08 cm (1 to 2 inches) of water. The drum extractor was rotated continuously during each wash to improving mixing the extraction efficiency.

The oil-hexane miscella leaving the extractor was filtered through a one micron filter and then evaporated to a minimum solvent content in a batch evaporation vessel. The solvent was removed by evaporation at 77 to 93 °C (170°F to 200°F) and a vacuum of 50.8 to 61 cm (20 to 24 inches) of Hg. 0.5% to 2% nitrogen was sparged to achieve low residual solvent in the crude oil. The desolventized crude oil was then packed in 18.9 L (5 gallon) containers. The wet spent meal ("marc") was desolventized in the same drum extractor vessel after the oil-hexane miscella was pumped out. Desolventization and drying of the marc was performed in the drum extractor by heating the biomass to 104 to 116°C (220°F to 240°F) using only indirect steam. The desolventized meal was packed in 166.56 L (44 gallon) fiber drums.

Solvent vapors from the drum extractor and the oil evaporator were condensed and recovered in the solvent-water tank where the water and solids were removed. The reclaimed solvent was stored and can be reused in future solvent extractions.

### EXAMPLE 11

### Drying and Oil Extraction from Oleaginous Yeast

Oleaginous yeast strain *Rhodotorula glutinis* (DSMZ-DSM 70398) was cultured according to the methods in Example 1 to produce oleaginous yeast biomass with approximately 50% lipid by DCW. The harvested yeast broth was dried using three different methods for comparison: (1) tray dried in a forced air oven at 75°C overnight; (2) dried on a drum dryer without concentration; and (3) the yeast broth was concentrated to 22% solids and the slurry was then dried on a drum dryer. Material from each of the three different drying conditions was heat conditioned and fed through a screw press for oil extraction. The press temperature was at 66°C (150°F) and the conditioned dried yeast biomass was held at about 88°C (190°F) until it was ready to be fed into the press.

The moisture content of the drum dried yeast broth without concentration was 5.4% and the drum dried yeast was then conditioned in an oven at 90°C for 20 minutes. The moisture content after conditioning was 1.4%. The conditioned drum dried yeast was then fed into a bench-top Taby screw press for oil extraction. This material oiled well, with minimal footing.

The moisture content of the drum dried concentrated yeast broth was 2.1% and the drum dried concentrated yeast was then conditioned in an oven at 90°C for 20 minutes. The moisture content after conditioning was 1.0%. The conditioned drum dried concentrated yeast was then fed into a bench-top Taby screw press for oil extraction. This material oiled well, with minimal footing.

### EXAMPLE 12

### Drying and Oil Extraction from Oleaginous Bacteria

Oleaginous bacteria strain *Rhodococcus opacus* PD630 (DSMZ-DSM 44193) was cultured according to the methods in Example 1 to produce oleaginous bacteria biomass with approximately 32% lipid by DCW.

The harvested *Rhodococcus opacus* broth was concentrated using centrifugation and then washed with deionized water and resuspended in 1.8L of deionized water. 50 grams of purified cellulose (PB20-Pre-co-Floc, EP Minerals, Nevada) was added to the resuspended biomass and the total solids was adjusted with deionized water to 20%. The *Rhodococcus* biomass was then dried on a drum drier and the moisture content of the *Rhodococcus* after drum drying was approximately 3%.

The drum-dried material was then heat conditioned in a oven at 130°C for 30 minutes with a resulting moisture content of approximately 1.2%. The heat conditioned biomass was then fed through a bench top Taby press (screw press) for oil extraction. The press temperature was at 98 °C (209°F) and the conditioned dried yeast biomass was held at about 116 °C (240°F) until it was ready to be fed into the press. Oil recovery was accompanied by heavy footing.

### EXAMPLE 13

### Genotyping Microalgal Strains

Microalgae samples from the 23 strains listed in Table 5 above were genotyped. Genomic DNA was isolated from algal biomass as follows. Cells (approximately 200 mg) were centifuged from liquid cultures 5 minutes at 14,000 x g. Cells were then resuspended in sterile distilled water, centrifuged 5 minutes at 14,000 x g and the supernatant discarded. A single glass bead ∼2mm in diameter was added to the biomass and tubes were placed at -80°C for at least 15 minutes. Samples were removed and 150 µl of grinding buffer (1% Sarkosyl, 0.25 M Sucrose, 50 mM NaCl, 20 mM EDTA, 100 mM Tris-HCl, pH 8.0, RNase A 0.5 ug/ul) was added. Pellets were resuspended by vortexing briefly, followed by the addition of 40 ul of 5M NaCl. Samples were vortexed briefly, followed by the addition of 66 µl of 5% CTAB (Cetyl trimethylammonium bromide) and a final brief vortex. Samples were next incubated at 65°C for 10 minutes after which they were centrifuged at 14,000 x g for 10 minutes. The supernatant was transferred to a fresh tube and extracted once with 300 µl of Phenol:Chloroform:Isoamyl alcohol 12:12:1, followed by centrifugation for 5 minutes at 14,000 x g. The resulting aqueous phase was transferred to a fresh tube containing 0.7 vol of isopropanol (∼190 µl), mixed by inversion and incubated at room temperature for 30 minutes or overnight at 4°C. DNA was recovered via centrifugation at 14,000 x g for 10 minutes. The resulting pellet was then washed twice with 70% ethanol, followed by a final wash with 100% ethanol. Pellets were air dried for 20-30 minutes at room temperature followed by resuspension in 50 µl of 10mM TrisCl, 1mM EDTA (pH 8.0).

Five µl of total algal DNA, prepared as described above, was diluted 1:50 in 10 mM Tris, pH 8.0. PCR reactions, final volume 20 µl, were set up as follows. Ten µl of 2 x iProof HF master mix (BIO-RAD) was added to 0.4 µl primer SZ02613 (5'-TGTTGAAGAATGAGCCGGCGAC-3' (SEQ ID NO:24) at 10mM stock concentration). This primer sequence runs from position 567-588 in Gen Bank accession no. L43357 and is highly conserved in higher plants and algal plastid genomes. This was followed by the addition of 0.4 µl primer SZ02615 (5'-CAGTGAGCTATTACGCACTC-3' (SEQ ID NO:25) at 10 mM stock concentration). This primer sequence is complementary to position 1112-1093 in Gen Bank accession no. L43357 and is highly conserved in higher plants and algal plastid genomes. Next, 5 µl of diluted total DNA and 3.2 µl dH₂O were added. PCR reactions were run as follows: 98°C, 45"; 98°C, 8"; 53°C, 12"; 72°C, 20" for 35 cycles followed by 72°C for 1 min and holding at 25°C. For purification of PCR products, 20 µl of 10 mM Tris, pH 8.0, was added to each reaction, followed by extraction with 40 µl of Phenol:Chloroform:isoamyl alcohol 12:12:1, vortexing and centrifuging at 14,000 x g for 5 minutes. PCR reactions were applied to S-400 columns (GE Healthcare) and centrifuged for 2 minutes at 3,000 x g. Purified PCR products were subsequently TOPO cloned into PCR8/GW/TOPO and positive clones selected for on LB/Spec plates. Purified plasmid DNA was sequenced in both directions using M13 forward and reverse primers. Sequences from the 23 microalgal strains are listed as SEQ ID NOs:1-23 in the attached Sequence Listing (see Table 5 for the correlation). Additionally, several *Prototheca* strains of microalgae (see Table 16, below) were also genotyped using the methods and primers described above. 23S rRNA genomic sequences are listed as SEQ ID NOs:26-34 in the attached Sequence Listing and are described below.

**Table 16. Prototheca microalgal strains.**

| Species | Strain | SEQ ID NO. |
|---|---|---|
| *Prototheca kruegani* | UTEX 329 | SEQ ID NO:26 |
| *Prototheca wickerhamii* | UTEX 1440 | SEQ ID NO:27 |
| *Prototheca stagnora* | UTEX 1442 | SEQ ID NO:28 |
| *Prototheca moriformis* | UTEX 288 | SEQ ID NO:29 |
| *Prototheca moriformis* | UTEX 1439; 1441; 1435; 1437 | SEQ ID NO:30 |
| *Prototheca wikerhamii* | UTEX 1533 | SEQ ID NO:31 |
| *Prototheca moriformis* | UTEX 1434 | SEQ ID NO:32 |
| *Prototheca zopfii* | UTEX 1438 | SEQ ID NO:33 |
| *Prototheca moriformis* | UTEX 1436 | SEQ ID NO:34 |

### Genotyping Oleaginous Yeast Strains

Genotyping of 48 different strains of oleaginous yeast was performed. Genomic DNA was isolated from each of the 48 different strains of oleaginous yeast biomass as follows. Cells (approximately 200 mg) were centrifuged from liquid cultures 5 minutes at 14,000 x g. Cells were then resuspended in sterile distilled water, centrifuged 5 minutes at 14,000 x g and the supernatant discarded. A single glass bead ∼2mm in diameter was added to the biomass and tubes were placed at -80°C for at least 15 minutes. Samples were removed and 150 µl of grinding buffer (1% Sarkosyl, 0.25 M Sucrose, 50 mM NaCl, 20 mM EDTA, 100 mM Tris-HCl, pH 8.0, RNase A 0.5 ug/ul) was added. Pellets were resuspended by vortexing briefly, followed by the addition of 40 ul of 5M NaCl. Samples were vortexed briefly, followed by the addition of 66 µl of 5% CTAB (Cetyl trimethylammonium bromide) and a final brief vortex. Samples were next incubated at 65°C for 10 minutes after which they were centrifuged at 14,000 x g for 10 minutes. The supernatant was transferred to a fresh tube and extracted once with 300 µl of Phenol:Chloroform:Isoamyl alcohol 12:12:1, followed by centrifugation for 5 minutes at 14,000 x g. The resulting aqueous phase was transferred to a fresh tube containing 0.7 vol of isopropanol (∼190 µl), mixed by inversion and incubated at room temperature for 30 minutes or overnight at 4°C. DNA was recovered via centrifugation at 14,000 x g for 10 minutes. The resulting pellet was then washed twice with 70% ethanol, followed by a final wash with 100% ethanol. Pellets were air dried for 20-30 minutes at room temperature followed by resuspension in 50 µl of 10mM TrisCl, 1mM EDTA (pH 8.0).

Five µl of total algal DNA, prepared as described above, was diluted 1:50 in 10 mM Tris, pH 8.0. PCR reactions, final volume 20 µl, were set up as follows. Ten µl of 2 x iProof HF master mix (BIO-RAD) was added to 0.4 µl primer SZ5434 forward primer (5' GTCCCTGCCCTTTGTACACAC -3' (SEQ ID NO: 35) at 10mM stock concentration) and 0.4 µl primer SZ5435 reverse primer (5'- TTGATATGCTTAAGTTCAGCGGG -3' (SEQ ID NO: 36) at 10 mM stock concentration). The primers were selected based on sequence conservation between three prime regions of 18S and five prime regions of fungal 26S rRNA genes. By reference, the forward primer is identical to nucleotides 1632-1652 of Genbank Ascension # AY550243 and the reverse primer is identical to nucleotides 464271-464293 of Genbank Ascension # NC_001144. Next, 5 µl of diluted total DNA and 3.2 µl dH₂O were added. PCR reactions were run as follows: 98°C, 45 seconds; 98°C, 8 seconds; 58°C, 12 seconds; 72°C, 36 seconds for 35 cycles followed by 72°C for 1 min and holding at 4°C. For purification of PCR products, 20 µl of 10 mM Tris, pH 8.0, was added to each reaction, followed by extraction with 40 µl of Phenol:Chloroform:isoamyl alcohol 12:12:1, vortexing and centrifuging at 14,000 x g for 5 minutes. PCR reactions were applied to S-400 columns (GE Healthcare) and centrifuged for 2 minutes at 3,000 x g. The resulting purified PCR products were cloned and transformed into *E. coli* using ZeroBlunt PCR4Blunt-TOPO vector kit (Invitrogen) according to manufacture's instructions. Sequencing reactions were carried out directly on ampicillin resistant colonies. Purified plasmid DNA was sequenced in both directions using M13 forward and reverse primers.

A list of the 48 strains of oleaginous yeast that were genotyped is summarized below in Table 17 along with the corresponding SEQ ID NOs.

**Table 17. Oleaginous yeast strains.**

| Strain Name | Strain Number | SEQ ID NO |
|---|---|---|
| *Rhodotorula glutinis* | DSMZ-DSM 70398 | SEQ ID NO:37 |
| *Lipomyces tetrasporus* | CBS 5911 | SEQ ID NO:70 |
| *Rhodotorula glutinis var. glutinis* | CBS 3044 | SEQ ID NO:38 |
| *Lipomyces tetrasporus* | CBS 8664 | SEQ ID NO:71 |
| *Lipomyces tetrasporus* | CBS 1808 | SEQ ID NO:39 |
| *Lipomyces tetrasporus* | CBS 1810 | SEQ ID NO:39 |
| *Lipomyces starkeyi* | CBS 1809 | SEQ ID NO:40 |
| *Trichosporon montevideense* | CBS 8261 | SEQ ID NO:72 |
| *Yarrowia lipolytica* | CBS 6331 | SEQ ID NO:41 |
| *Cryptococcus curvatus* | CBS 5324 | SEQ ID NO:42 |
| *Rhodotorula mucilaginosa var. mucilaginosa* | CBS 316 | SEQ ID NO:73 |
| *Cryptococcus curvatus* | CBS 570 | SEQ ID NO:42 |
| *Cryptococcus curvatus* | CBS 2176 | SEQ ID NO:42 |
| *Cryptococcus curvatus* | CBS 2744 | SEQ ID NO:42 |
| *Cryptococcus curvatus* | CBS 2754 | SEQ ID NO:42 |
| *Cryptococcus curvatus* | CBS 2829 | SEQ ID NO:42 |
| *Cryptococcus curvatus* | CBS 5163 | SEQ ID NO:42 |
| *Cryptococcus curvatus* | CBS 5358 | SEQ ID NO:42 |
| *Trichosporon sp.* | CBS 7617 | SEQ ID NO:43 |
| *Sporobolomyces alborubescens* | CBS 482 | SEQ ID NO:44 |
| *Rhodotorula glutinis var. glutinis* | CBS 324 | SEQ ID NO:45 |
| *Rhodotorula glutinis var. glutinis* | CBS 4476 | SEQ ID NO:46 |
| *Trichosporon behrend* | CBS 5581 | SEQ ID NO:47 |
| *Geotrichum histeridarum* | CBS 9892 | SEQ ID NO:48 |
| *Rhodotorula aurantiaca* | CBS 8411 | SEQ ID NO:49 |
| *Cryptococcus curvatus* | CBS 8126 | SEQ ID NO:74 |
| *Trichosporon domesticum* | CBS 8111 | SEQ ID NO:50 |
| *Rhodotorula toruloides* | CBS 8761 | SEQ ID NO:51 |
| *Rhodotorula terpendoidalis* | CBS 8445 | SEQ ID NO:52 |
| *Yarrowia lipolytica* | CBS 10144 | SEQ ID NO:53 |
| *Rhodotorula glutinis var. glutinis* | CBS 5805 | SEQ ID NO:54 |
| *Yarrowia lipolytica* | CBS 10143 | SEQ ID NO:55 |
| *Lipomyces tetrasporus* | CBS 5607 | SEQ ID NO:56 |
| *Yarrowia lipolytica* | CBS 5589 | SEQ ID NO:57 |
| *Lipomyces tetrasporus* | CBS 8724 | SEQ ID NO:58 |
| *Rhodosporidium sphaerocarpum* | CBS 2371 | SEQ ID NO:59 |
| *Trichosporon brassicae* | CBS 6382 | SEQ ID NO:60 |
| *Cryptococcus curvatus* | CBS 2755 | SEQ ID NO:61 |
| *Lipomyces tetrasporus* | CBS 7656 | SEQ ID NO:75 |
| *Lipomyces starkeyi* | CBS 7786 | SEQ ID NO:62 |
| *Yarrowia lipolytica* | CBS 6012 | SEQ ID NO:63 |
| *Trichosporon loubieri var. loubieri* | CBS 8265 | SEQ ID NO:64 |
| *Geotrichum vulgare* | CBS 10073 | SEQ ID NO:65 |
| *Rhodosporidium toruloides* | CBS 14 | SEQ ID NO:66 |
| *Rhodotorula glutinis var. glutinis* | CBS 6020 | SEQ ID NO:67 |
| *Lipomyces orientalis* | CBS 10300 | SEQ ID NO:76 |
| *Rhodotorula aurantiaca* | CBS 317 | SEQ ID NO:68 |
| *Torulaspora delbrueckii* | CBS 2924 | SEQ ID NO:69 |

The publications mentioned herein are cited for the purpose of describing and disclosing reagents, methodologies and concepts that may be used in connection with the present invention. Nothing herein is to be construed as an admission that these references are prior art in relation to the inventions described herein.

Although this invention has been described in connection with specific embodiments thereof, it will be understood that it is capable of further modifications following, in general, the principles of the invention and including such departures from the present disclosure as come within known or customary practice within the art to which the invention pertains and as may be applied to the essential features hereinbefore set forth.

### SEQUENCE LISTING

**SEQ ID NO:1**
   *Chlorella protothecoides* (UTEX 250) 
TGTTGAAGAATGAGCCGGCGACTTAGAAAAAGTGGCGTGGTTAAGGAAAAATTC
CGAAGCCTTAGCGAAAGCGAGTCTGAATAGGGCGATCAAATATTTTAATATTTAC
AATTTAGTCATTTTTTCTAGACCCGAACCCGGGTGATCTAACCATGACCAGGATG
AAACTTGGGTGATACCAAGTGAAGGTCCGAACCGACCGATGTTGAAAAATCGGC
GGATGAGTTGTGGTTAGCGGTGAAATACCAGTCGAACCCGGAGCTAGCTGGTTCT
CCCCGAAATGCGTTGAGGCGCAGCAGTACATCTAGTCTATCTAGGGGTAAAGCA
CTGTTTCGGTGCGGGCTGTGAAAACGGTACCAAATCGTGGCAAACTCTGAATACT
AGAAATGACGGTGTAGTAGTGAGACTGTGGGGGATAAGCTCCATTGTCAAGAGG
GAAACAGCCCAGACCACCAGCTAAGGCCCCAAAATGGTAATGTAGTGACAAAGG
AGGTGAAAATGCAAACACAACCAGGAGGTTGGCTTAGAAGCAGCCATCCTTTAA
AGAGTGCGTAATAGCTCACTG
**SEQ ID NO:2**
   *Chlorella protothecoides* (UTEX 25) 
TGTTGAAGAATGAGCCGGCGACTTAGAAAACGTGGCAAGGTTAAGGAAACGTAT
CCGGAGCCGAAGCGAAAGCAAGTCTGAACAGGGCGATTAAGTCATTTTTCTAG
ACCCGAACCCGGGTGATCTAACCATGACCAGGATGAAGCTTGGGTGACACCAAG
TGAAGGTCCGAACCGACCGATGTTGAAAAATCGGCGGATGAGTTGTGGTTAGCG
GTGAAATACCAGTCGAACTCGGAGCTAGCTGGTTCTCCCCGAAATGCGTTGAGGC
GCAGCGGTTCATAAGGCTGTCTAGGGGTAAAGCACTGTTTCGGTGCGGGCTGCG
AAAGCGGTACCAAATCGTGGCAAACTCTGAATACTAGATATGCTATTTATGGGCC
AGTGAGACGGTGGGGGATAAGCTTCATCGTCGAGAGGGAAACAGCCCAGATCAC
TAGCTAAGGCCCCAAAATGATCGTTAAGTGACAAAGGAGGTGAGAATGCAGAAA
CAACCAGGAGGTTTGCTTAGAAGCAGCCACCCTTTAAAGAGTGCGTAATAGCTC
ACTG
**SEQ ID NO:3**
   *Chlorella protothecoides* (CCAP 211/8D) 
TGTTGAAGAATGAGCCGGCGACTTAGAAAAAGTGGCGTGGTTAAGGAAAAATTC
CGAAGCCTTAGCGAAAGCGAGTCTGAATAGGGCGATCAAATATTTTAATATTTAC
AATTTAGTCATTTTTTCTAGACCCGAACCCGGGTGATCTAACCATGACCAGGATG
AAACTTGGGTGATACCAAGTGAAGGTCCGAACCGACCGATGTTGAAAAATCGGC
GGATGAGTTGTGGTTAGCGGTGAAATACCAGTCGAACCCGGAGCTAGCTGGTTCT
CCCCGAAATGCGTTGAGGCGCAGCAGTACATCTAGTCTATCTAGGGGTAAAGCA
CTGTTTCGGTGCGGGCTGTGAAAACGGTACCAAATCGTGGCAAACTCTGAATACT
AGAAATGACGGTGTAGTAGTGAGACTGTGGGGGATAAGCTCCATTGTCAAGAGG
GAAACAGCCCAGACCACCAGCTAAGGCCCCAAAATGGTAATGTAGTGACAAAGG
AGGTGAAAATGCAAACACAACCAGGAGGTTGGCTTAGAAGCAGCCATCCTTTAA
AGAGTGCGTAATAGCTCACTG
**SEQ ID NO:4**
   *Chlorella kessleri* (UTEX 397) 
TGTTGAAGAATGAGCCGGCGACTTAGAAAAAGTGGCGTGGTTAAGGAAAAATTC
CGAAGCCTTAGCGAAAGCGAGTCTGAATAGGGCGATCAAATATTTTAATATTTAC
AATTTAGTCATTTTTTCTAGACCCGAACCCGGGTGATCTAACCATGACCAGGATG
AAACTTGGGTGATACCAAGTGAAGGTCCGAACCGACCGATGTTGAAAAATCGGC
GGATGAGTTGTGGTTAGCGGTGAAATACCAGTCGAACCCGGAGCTAGCTGGTTCT
CCCCGAAATGCGTTGAGGCGCAGCAGTACATCTAGTCTATCTAGGGGTAAAGCA
CTGTTTCGGTGCGGGCTGTGAAAACGGTACCAAATCGTGGCAAACTCTGAATACT
AGAAATGACGGTGTAGTAGTGAGACTGTGGGGGATAAGCTCCATTGTCAAGAGG
GAAACAGCCCAGACCACCAGCTAAGGCCCCAAAATGGTAATGTAGTGACAAAGG
AGGTGAAAATGCAAACACAACCAGGAGGTTGGCTTAGAAGCAGCCATCCTTTAA
AGAGTGCGTAATAGCTCACTG
**SEQ ID NO:5**
   *Chlorella kessleri* (UTEX 2229) 
TGTTGAAGAATGAGCCGGCGACTTAGAAGAAGTGGCTTGGTTAAGGATAACTAT
CCGGAGCCAGAGCGAAAGCAAGTCTGAATAGGGCGCTTAAAGGTCACTTTTTCT
AGACCCGAACCCGGGTGATCTAACCATGACCAGGATGAAGCTTGGGTAACACCA
CGTGAAGGTCCGAACCGACCGATGTTGAAAAATCGGCGGATGAGTTGTGGTTAG
CGGTGAAATACCAATCGAACTCGGAGCTAGCTGGTTCTCCCCGAAATGCGTTGAG
GCGCAGCGGTTTATGAGGCTGTCTAGGGGTAAAGCACTGTTTCGGTGCGGGCTGC
GAAAGCGGTACCAAATCGTGGCAAACTCTGAATACTAGATATGCTATTCATGAG
CCAGTGAGACGGTGGGGGATAAGCTTCATCGTCAAGAGGGAAACAGCCCAGATC
ACCAGCTAAGGCCCCAAAATGGTCGTTAAGTGGCAAAGGAGGTGAGAATGCTGA
AACAACCAGGAGGTTTGCTTAGAAGCAGCCACCCTTTAAAGAGTGCGTAATAGC
TCACTG
**SEQ ID NO:6**
   *Chlorella kessleri* (UTEX 398) 
TGTTGAAGAATGAGCCGGCGACTTAGAAGAAGTGGCTTGGTTAAGGATAACTAT
CCGGAGCCAGAGCGAAAGCAAGTCTGAATAGGGCGCTTAAAGGTCACTTTTTCT
AGACCCGAACCCGGGTGATCTAACCATGACCAGGATGAAGCTTGGGTAACACCA
CGTGAAGGTCCGAACCGACCGATGTTGAAAAATCGGCGGATGAGTTGTGGTTAG
CGGTGAAATACCAATCGAACTCGGAGCTAGCTGGTTCTCCCCGAAATGCGTTGAG
GCGCAGCGGTTTATGAGGCTGTCTAGGGGTAAAGCACTGTTTCGGTGCGGGCTGC
GAAAGCGGTACCAAATCGTGGCAAACTCTGAATACTAGATATGCTATTCATGAG
CCAGTGAGACGGTGGGGGATAAGCTTCATCGTCAAGAGGGAAACAGCCCAGATC
ACCAGCTAAGGCCCCAAAATGGTCGTTAAGTGGCAAAGGAGGTGAGAATGCTGA
AACAACCAGGAGGTTTGCTTAGAAGCAGCCACCCTTTAAAGAGTGCGTAATAGC
TCACTG
**SEQ ID NO:7**
   *Parachlorella kessleri* (SAG 11.80) 
TGTTGAAGAATGAGCCGGCGACTTAGAAGAAGTGGCTTGGTTAAGGATAACTAT
CCGGAGCCAGAGCGAAAGCAAGTCTGAATAGGGCGCTTAAAGGTCACTTTTTCT
AGACCCGAACCCGGGTGATCTAACCATGACCAGGATGAAGCTTGGGTAACACCA
CGTGAAGGTCCGAACCGACCGATGTTGAAAAATCGGCGGATGAGTTGTGGTTAG
CGGTGAAATACCAATCGAACTCGGAGCTAGCTGGTTCTCCCCGAAATGCGTTGAG
 
GCGCAGCGGTTTATGAGGCTGTCTAGGGGTAAAGCACTGTTTCGGTGCGGGCTGC
GAAAGCGGTACCAAATCGTGGCAAACTCTGAATACTAGATATGCTATTCATGAG
CCAGTGAGACGGTGGGGGATAAGCTTCATCGTCAAGAGGGAAACAGCCCAGATC
ACCAGCTAAGGCCCCAAAATGGTCGTTAAGTGGCAAAGGAGGTGAGAATGCTGA
AACAACCAGGAGGTTTGCTTAGAAGCAGCCACCCTTTAAAGAGTGCGTAATAGC
TCACTG
**SEQ ID NO:8**
   *Parachlorella kessleri* (SAG 14.82) 
TGTTGAAGAATGAGCCGGCGACTTAGAAGAAGTGGCTTGGTTAAGGATAACTAT
CCGGAGCCAGAGCGAAAGCAAGTCTGAATAGGGCGCTTAAAGGTCACTTTTTCT
AGACCCGAACCCGGGTGATCTAACCATGACCAGGATGAAGCTTGGGTAACACCA
CGTGAAGGTCCGAACCGACCGATGTTGAAAAATCGGCGGATGAGTTGTGGTTAG
CGGTGAAATACCAATCGAACTCGGAGCTAGCTGGTTCTCCCCGAAATGCGTTGAG
GCGCAGCGGTTTATGAGGCTGTCTAGGGGTAAAGCACTGTTTCGGTGCGGGCTGC
GAAAGCGGTACCAAATCGTGGCAAACTCTGAATACTAGATATGCTATTCATGAG
CCAGTGAGACGGTGGGGGATAAGCTTCATCGTCAAGAGGGAAACAGCCCAGATC
ACCAGCTAAGGCCCCAAAATGGTCGTTAAGTGGCAAAGGAGGTGAGAATGCTGA
AACAACCAGGAGGTTTGCTTAGAAGCAGCCACCCTTTAAAGAGTGCGTAATAGC
TCACTG
**SEQ ID NO:9**
   *Parachlorella kessleri* (SAG 21.11 H9) 
TGTTGAAGAATGAGCCGGCGACTTAGAAAAAGTGGCGTGGTTAAGGAAAAATTC
CGAAGCCTTAGCGAAAGCGAGTCTGAATAGGGCGATCAAATATTTTAATATTTAC
AATTTAGTCATTTTTTCTAGACCCGAACCCGGGTGATCTAACCATGACCAGGATG
AAACTTGGGTGATACCAAGTGAAGGTCCGAACCGACCGATGTTGAAAAATCGGC
GGATGAGTTGTGGTTAGCGGTGAAATACCAGTCGAACCCGGAGCTAGCTGGTTCT
CCCCGAAATGCGTTGAGGCGCAGCAGTACATCTAGTCTATCTAGGGGTAAAGCA
CTGTTTCGGTGCGGGCTGTGAAAACGGTACCAAATCGTGGCAAACTCTGAATACT
AGAAATGACGGTGTAGTAGTGAGACTGTGGGGGATAAGCTCCATTGTCAAGAGG
GAAACAGCCCAGACCACCAGCTAAGGCCCCAAAATGGTAATGTAGTGACAAAGG
AGGTGAAAATGCAAACACAACCAGGAGGTTGGCTTAGAAGCAGCCATCCTTTAA
AGAGTGCGTAATAGCTCACTG
**SEQ ID NO:10**
   *Prototheca stagnora* (UTEX 327) 
TGTTGAAGAATGAGCCGGCGAGTTAAAAAAAATGGCATGGTTAAAGATATTTCT
CTGAAGCCATAGCGAAAGCAAGTTTTACAAGCTATAGTCATTTTTTTAGACCCG
AAACCGAGTGATCTACCCATGATCAGGGTGAAGTGTTGGTCAAATAACATGGAG
GCCCGAACCGACTAATGGTGAAAAATTAGCGGATGAATTGTGGGTAGGGGCGAA
AAACCAATCGAACTCGGAGTTAGCTGGTTCTCCCCGAAATGCGTTTAGGCGCAGC
AGTAGCAACACAAATAGAGGGGTAAAGCACTGTTTCTTTTGTGGGCTTCGAAAGT
TGTACCTCAAAGTGGCAAACTCTGAATACTCTATTTAGATATCTACTAGTGAGAC
CTTGGGGGATAAGCTCCTTGGTCAAAAGGGAAACAGCCCAGATCACCAGTTAAG
GCCCCAAAATGAAAATGATAGTGACTAAGGACGTGAGTATGTCAAAACCTCCAG
CAGGTTAGCTTAGAAGCAGCAATCCTTTCAAGAGTGCGTAATAGCTCACTG
**SEQ ID NO:11**
   *Prototheca moriformis* (UTEX 1441) 
TGTTGAAGAATGAGCCGGCGACTTAAAATAAATGGCAGGCTAAGAGAATTAATA
ACTCGAAACCTAAGCGAAAGCAAGTCTTAATAGGGCGCTAATTTAACAAAACAT
TAAATAAAATCTAAAGTCATTTATTTTAGACCCGAACCTGAGTGATCTAACCATG
GTCAGGATGAAACTTGGGTGACACCAAGTGGAAGTCCGAACCGACCGATGTTGA
AAAATCGGCGGATGAACTGTGGTTAGTGGTGAAATACCAGTCGAACTCAGAGCT
AGCTGGTTCTCCCCGAAATGCGTTGAGGCGCAGCAATATATCTCGTCTATCTAGG
GGTAAAGCACTGTTTCGGTGCGGGCTATGAAAATGGTACCAAATCGTGGCAAAC
TCTGAATACTAGAAATGACGATATATTAGTGAGACTATGGGGGATAAGCTCCAT
AGTCGAGAGGGAAACAGCCCAGACCACCAGTTAAGGCCCCAAAATGATAATGAA
GTGGTAAAGGAGGTGAAAATGCAAATACAACCAGGAGGTTGGCTTAGAAGCAGC
CATCCTTTAAAGAGTGCGTAATAGCTCACTG
**SEQ ID NO:12**
   *Prototheca moriformis* (UTEX 1435) 
TGTTGAAGAATGAGCCGGCGACTTAAAATAAATGGCAGGCTAAGAGAATTAATA
ACTCGAAACCTAAGCGAAAGCAAGTCTTAATAGGGCGCTAATTTAACAAAACAT
TAAATAAAATCTAAAGTCATTTATTTTAGACCCGAACCTGAGTGATCTAACCATG
GTCAGGATGAAACTTGGGTGACACCAAGTGGAAGTCCGAACCGACCGATGTTGA
AAAATCGGCGGATGAACTGTGGTTAGTGGTGAAATACCAGTCGAACTCAGAGCT
AGCTGGTTCTCCCCGAAATGCGTTGAGGCGCAGCAATATATCTCGTCTATCTAGG
GGTAAAGCACTGTTTCGGTGCGGGCTATGAAAATGGTACCAAATCGTGGCAAAC
TCTGAATACTAGAAATGACGATATATTAGTGAGACTATGGGGGATAAGCTCCAT
AGTCGAGAGGGAAACAGCCCAGACCACCAGTTAAGGCCCCAAAATGATAATGAA
GTGGTAAAGGAGGTGAAAATGCAAATACAACCAGGAGGTTGGCTTAGAAGCAGC
CATCCTTTAAAGAGTGCGTAATAGCTCACTG
**SEQ ID NO:13**
   *Chlorella minutissima* (UTEX 2341) 
TGTTGAAGAATGAGCCGGCGACTTAGAAAAAGTGGCGTGGTTAAGGAAAAATTC
CGAAGCCTTAGCGAAAGCGAGTCTGAATAGGGCGATCAAATATTTTAATATTTAC
AATTTAGTCATTTTTTCTAGACCCGAACCCGGGTGATCTAACCATGACCAGGATG
AAACTTGGGTGATACCAAGTGAAGGTCCGAACCGACCGATGTTGAAAAATCGGC
GGATGAGTTGTGGTTAGCGGTGAAATACCAGTCGAACCCGGAGCTAGCTGGTTCT
CCCCGAAATGCGTTGAGGCGCAGCAGTACATCTAGTCTATCTAGGGGTAAAGCA
CTGTTTCGGTGCGGGCTGTGAAAACGGTACCAAATCGTGGCAAACTCTGAATACT
AGAAATGACGGTGTAGTAGTGAGACTGTGGGGGATAAGCTCCATTGTCAAGAGG
GAAACAGCCCAGACCACCAGCTAAGGCCCCAAAATGGTAATGTAGTGACAAAGG
AGGTGAAAATGCAAACACAACCAGGAGGTTGGCTTAGAAGCAGCCATCCTTTAA
AGAGTGCGTAATAGCTCACTG
**SEQ ID NO:14**
   *Chlorella sp.* (UTEX 2068) 
TGTTGAAGAATGAGCCGGCGACTTAGAAAAAGTGGCGTGGTTAAGGAAAAATTC
CGAAGCCTTAGCGAAAGCGAGTCTGAATAGGGCGATCAAATATTTTAATATTTAC
AATTTAGTCATTTTTTCTAGACCCGAACCCGGGTGATCTAACCATGACCAGGATG
 
AAACTTGGGTGATACCAAGTGAAGGTCCGAACCGACCGATGTTGAAAAATCGGC
GGATGAGTTGTGGTTAGCGGTGAAATACCAGTCGAACCCGGAGCTAGCTGGTTCT
CCCCGAAATGCGTTGAGGCGCAGCAGTACATCTAGTCTATCTAGGGGTAAAGCA
CTGTTTCGGTGCGGGCTGTGAAAACGGTACCAAATCGTGGCAAACTCTGAATACT
AGAAATGACGGTGTAGTAGTGAGACTGTGGGGGATAAGCTCCATTGTCAAGAGG
GAAACAGCCCAGACCACCAGCTAAGGCCCCAAAATGGTAATGTAGTGACAAAGG
AGGTGAAAATGCAAACACAACCAGGAGGTTGGCTTAGAAGCAGCCATCCTTTAA
AGAGTGCGTAATAGCTCACTG
**SEQ ID NO:15**
   *Chlorella sp.* (CCAP 211/92) 
TGTTGAAGAATGAGCCGGCGACTTAGAAAACGTGGCAAGGTTAAGGACATGTAT
CCGGAGCCGAAGCGAAAGCAAGTCTGAATAGGGCGCCTAAGTCATTTTTCTAG
ACCCGAACCCGGGTGATCTAACCATGACCAGGATGAAGCTTGGGTGACACCAAG
TGAAGGTCCGAACCGACCGATGTTGAAAAATCGGCGGATGAGTTGTGGTTAGCG
GTGAAATACCAGTCGAACTCGGAGCTAGCTGGTTCTCCCCGAAATGCGTTGAGGC
GCAGCGGTTCATAAGGCTGTCTAGGGGTAAAGCACTGTTTCGGTGCGGGCTGCG
AAAGCGGTACCAAATCGTGGCAAACTCTGAATACTAGATATGCTATTTATGAGCC
AGTGAGACGGTGGGGGATAAGCTTCATCGTCGAGAGGGAAACAGCCCAGATCAC
TAGCTAAGGCCCCTAAATGATCGTTAAGTGACAAAGGAGGTGAGAATGCAGAAA
CAACCAGGAGGTTTGCTTAGAAGCAGCCACCCTTTAAAGAGTGCGTAATAGCTC
ACTG
**SEQ ID NO:16**
   *Chlorella sorokiniana* (SAG 211.40B) 
TGTTGAAGAATGAGCCGGCGACTTATAGGAAGTGGCAGGGTTAAGGAAGAATCT
CCGGAGCCCAAGCGAAAGCGAGTCTGAAAAGGGCGATTTGGTCACTTCTTATGG
ACCCGAACCTGGATGATCTAATCATGGCCAAGTTGAAGCATGGGTAACACTATGT
CGAGGACTGAACCCACCGATGTTGAAAAATCGGGGGATGAGCTGTGATTAGCGG
TGAAATTCCAATCGAATTCAGAGCTAGCTGGATCTCCCCGAAATGCGTTGAGGCG
CAGCGGCGACGATGTCCTGTCTAAGGGTAGAGCGACTGTTTCGGTGCGGGCTGC
GAAAGCGGTACCAAGTCGTGGCAAACTCCGAATATTAGGCAAAGGATTCCGTGA
GCCAGTGAGACTGTGGGGGATAAGCTTCATAGTCAAGAGGGAAACAGCCCAGAC
CATCAGCTAAGGCCCCTAAATGGCTGCTAAGTGGAAAAGGATGTGAGAATGCTG
AAACAACCAGGAGGTTCGCTTAGAAGCAGCTATTCCTTGAAAGAGTGCGTAATA
GCTCACTG
**SEQ ID NO:17**
   *Parachlorella beijerinkii* (SAG 2046) 
TGTTGAAGAATGAGCCGGCGACTTAGAAGAAGTGGCTTGGTTAAGGATAACTAT
CCGGAGCCAGAGCGAAAGCAAGTCTGAATAGGGCGCTTAAAGGTCACTTTTCT
AGACCCGAACCCGGGTGATCTAACCATGACCAGGATGAAGCTTGGGTAACACCA
CGTGAAGGTCCGAACCGACCGATGTTGAAAAATCGGCGGATGAGTTGTGGTTAG
CGGTGAAATACCAATCGAACTCGGAGCTAGCTGGTTCTCCCCGAAATGCGTTGAG
GCGCAGCGGTTTATGAGGCTGTCTAGGGGTAAAGCACTGTTTCGGTGCGGGCTGC
GAAAGCGGTACCAAATCGTGGCAAACTCTGAATACTAGATATGCTATTCATGAG
CCAGTGAGACGGTGGGGGATAAGCTTCATCGTCAAGAGGGAAACAGCCCAGATC
 
ACCAGCTAAGGCCCCAAAATGGTCGTTAAGTGGCAAAGGAGGTGAGAATGCTGA
AACAACCAGGAGGTTTGCTTAGAAGCAGCCACCCTTTAAAGAGTGCGTAATAGC
TCACTG
**SEQ ID NO:18**
   *Chlorella luteoviridis* (SAG 2203) 
TGTTGAAGAATGAGCCGGCGACTTATAGGGGGTGGCGTGGTTAAGGAAGTAATC
CGAAGCCAAAGCGAAAGCAAGTTTTCAATAGAGCGATTTTGTCACCCCTTATGGA
CCCGAACCCGGGTGATCTAACCTTGACCAGGATGAAGCTTGGGTAACACCAAGT
GAAGGTCCGAACTCATCGATCTTGAAAAATCGTGGGATGAGTTGGGGTTAGTTG
GTTAAATGCTAATCGAACTCGGAGCTAGCTGGTTCTCCCCGAAATGTGTTGAGGC
GCAGCGATTAACGAAATATTTTGTACGGTTTAGGGGTAAAGCACTGTTTCGGTGC
GGGCTGCGAAAGCGGTACCAAATCGTGGCAAACTCTGAATACTAAGCCTGTATA
CCGTTAGTCAGTGAGAGTATAGGGGATAAGCTCTATACTCAAGAGGGAAACAGC
CCAGATCACCAGCTAAGGCCCCAAAATGACAGCTAAGTGGCAAAGGAGGTGAAA
GTGCAGAAACAACCAGGAGGTTCGCTTAGAAGCAGCAACCCTTTAAAGAGTGCG
TAATAGCTCACTG
**SEQ ID NO:19**
   *Chlorella vulgaris* (CCAP 211/11K) 
TGTTGAAGAATGAGCCGGCGACTTAGAAGAAGTGGCTTGGTTAAGGATAACTAT
CCGGAGCCAGAGCGAAAGCAAGTCTGAATAGGGCGCTTAAAGGTCACTTTTTCT
AGACCCGAACCCGGGTGATCTAACCATGACCAGGATGAAGCTTGGGTAACACCA
CGTGAAGGTCCGAACCGACCGATGTTGAAAAATCGGCGGATGAGTTGTGGTTAG
CGGTGAAATACCAATCGAACTCGGAGCTAGCTGGTTCTCCCCGAAATGCGTTGAG
GCGCAGCGGTTTATGAGGCTGTCTAGGGGTAAAGCACTGTTTCGGTGCGGGCTGC
GAAAGCGGTACCAAATCGTGGCAAACTCTGAATACTAGATATGCTATTCATGAG
CCAGTGAGACGGTGGGGGATAAGCTTCATCGTCAAGAGGGAAACAGCCCAGATC
ACCAGCTAAGGCCCCAAAATGGTCGTTAAGTGGCAAAGGAGGTGAGAATGCTGA
AACAACCAGGAGGTTTGCTTAGAAGCAGCCACCCTTTAAAGAGTGCGTAATAGC
TCACTG
**SEQ ID NO:20**
   *Chlorella reisiglii* (CCAP 11/8) 
TGTTGAAGAATGAGCCGGCGACTTAGAAAAAGTGGCGTGGTTAAGGAAAAATTC
CGAAGCCTTAGCGAAAGCGAGTCTGAATAGGGCGATCAAATATTTTAATATTTAC
AATTTAGTCATTTTTTCTAGACCCGAACCCGGGTGATCTAACCATGACCAGGATG
AAACTTGGGTGATACCAAGTGAAGGTCCGAACCGACCGATGTTGAAAAATCGGC
GGATGAGTTGTGGTTAGCGGTGAAATACCAGTCGAACCCGGAGCTAGCTGGTTCT
CCCCGAAATGCGTTGAGGCGCAGCAGTACATCTAGTCTATCTAGGGGTAAAGCA
CTGTTTCGGTGCGGGCTGTGAAAACGGTACCAAATCGTGGCAAACTCTGAATACT
AGAAATGACGGTGTAGTAGTGAGACTGTGGGGGATAAGCTCCATTGTCAAGAGG
GAAACAGCCCAGACCACCAGCTAAGGCCCCAAAATGGTAATGTAGTGACAAAGG
AGGTGAAAATGCAAACACAACCAGGAGGTTGGCTTAGAAGCAGCCATCCTTTAA
AGAGTGCGTAATAGCTCACTG
**SEQ ID NO:21**
   *Chlorella ellipsoidea* (CCAP 211/42) 
TGTTGAAGAATGAGCCGGCGACTTATAGGGGGTGGCTTGGTTAAGGACTACAAT
CCGAAGCCCAAGCGAAAGCAAGTTTGAAGTGTACACACATTGTGTGTCTAGAGC
GATTTTGTCACTCCTTATGGACCCGAACCCGGGTGATCTATTCATGGCCAGGATG
AAGCTTGGGTAACACCAAGTGAAGGTCCGAACTCATCGATGTTGAAAAATCGTG
GGATGAGTTGTGAATAGGGGTGAAATGCCAATCGAACTCGGAGCTAGCTGGTTC
TCCCCGAAATGTGTTGAGGCGCAGCGATTCACGATCTAAAGTACGGTTTAGGGGT
AAAGCACTGTTTCGGTGCGGGCTGTTAACGCGGTACCAAATCGTGGCAAACTAA
GAATACTAAACTTGTATGCCGTGAATCAGTGAGACTAAGAGGGATAAGCTTCTTA
GTCAAGAGGGAAACAGCCCAGATCACCAGCTAAGGCCCCAAAATGACAGCTAAG
TGGCAAAGGAGGTGAGAGTGCAGAAACAACCAGGAGGTTTGCTTAGAAGCAGCC
ATCCTTTAAAGAGTGCGTAATAGCTCACTG
**SEQ ID NO:22**
   *Chlorella saccharophila* (CCAP 211/31) 
TGTTGAAGAATGAGCCGGCGACTTATAGGGGGTGGCTTGGTTAAGGACTACAAT
CCGAAGCCCAAGCGAAAGCAAGTTTGAAGTGTACACACGTTGTGTGTCTAGAGC
GATTTTGTCACTCCTTATGGACCCGAACCCGGGTGATCTATTCATGGCCAGGATG
AAGCTTGGGTAACACCAAGTGAAGGTCCGAACTCATCGATGTTGAAAAATCGTG
GGATGAGTTGTGAATAGGGGTGAAATGCCAATCGAACTCGGAGCTAGCTGGTTC
TCCCCGAAATGTGTTGAGGCGCAGCGATTCACGATCTAAAGTACGGTTTAGGGGT
AAAGCACTGTTTCGGTGCGGGCTGTTAACGCGGTACCAAATCGTGGCAAACTAA
GAATACTAAACTTGTATGCCGTGAATCAGTGAGACTAAGAGGGATAAGCTTCTTA
GTCAAGAGGGAAACAGCCCAGATCACCAGCTAAGGCCCCAAAATGACAGCTAAG
TGGCAAAGGAGGTGAGAGTGCAGAAACAACCAGGAGGTTTGCTTAGAAGCAGCC
ATCCTTTAAAGAGTGCGTAATAGCTCACTG
**SEQ ID NO:23**
   *Chlorella saccharophila* (CCAP 211/32) 
TGTTGAAGAATGAGCCGGCGACTTATAGGGGGTGGCTTGGTTAAGGACTACAAT
CCGAAGCCCAAGCGAAAGCAAGTTTGAAGTGTACACACATTGTGTGTCTAGAGC
GATTTTGTCACTCCTTATGGACCCGAACCCGGGTGATCTATTCATGGCCAGGATG
AAGCTTGGGTAACACCAAGTGAAGGTCCGAACTCATCGATGTTGAAAAATCGTG
GGATGAGTTGTGAATAGGGGTGAAATGCCAATCGAACTCGGAGCTAGCTGGTTC
TCCCCGAAATGTGTTGAGGCGCAGCGATTCACGATCTAAAGTACGGTTTAGGGGT
AAAGCACTGTTTCGGTGCGGGCTGTTAACGCGGTACCAAATCGTGGCAAACTAA
GAATACTAAACTTGTATGCCGTGAATCAGTGAGACTAAGAGGGATAAGCTTCTTA
GTCAAGAGGGAAACAGCCCAGATCACCAGCTAAGGCCCCAAAATGACAGCTAAG
TGGCAAAGGAGGTGAGAGTGCAGAAACAACCAGGAGGTTTGCTTAGAAGCAGCC
ATCCTTTAAAGAGTGCGTAATAGCTCACTG
**SEQ ID NO:24**
   SZ02613
   TGTTGAAGAATGAGCCGGCGAC
**SEQ ID NO:25**
   SZ02615
   CAGTGAGCTATTACGCACTC
**SEQ ID NO:26**
   UTEX 329 *Prototheca kruegani* 
TGTTGAAGAATGAGCCGGCGAGTTAAAAAGAGTGGCATGGTTAAAGAAAATACT
CTGGAGCCATAGCGAAAGCAAGTTTAGTAAGCTTAGGTCATTCTTTTTAGACCCG
AAACCGAGTGATCTACCCATGATCAGGGTGAAGTGTTAGTAAAATAACATGGAG
GCCCGAACCGACTAATGTTGAAAAATTAGCGGATGAATTGTGGGTAGGGGCGAA
AAACCAATCGAACTCGGAGTTAGCTGGTTCTCCCCGAAATGCGTTTAGGCGCAGC
AGTAGCAGTACAAATAGAGGGGTAAAGCACTGTTTCTTTTGTGGGCTTCGAAAGT
TGTACCTCAAAGTGGCAAACTCTGAATACTCTATTTAGATATCTACTAGTGAGAC
CTTGGGGGATAAGCTCCTTGGTCAAAAGGGAAACAGCCCAGATCACCAGTTAAG
GCCCCAAAATGAAAATGATAGTGACTAAGGATGTGGGTATGTCAAAACCTCCAG
CAGGTTAGCTTAGAAGCAGCAATCCTTTCAAGAGTGCGTAATAGCTCACTG
**SEQ ID NO:27**
   UTEX 1440 *Prototheca wickerhamii* 
TGTTGAAGAATGAGCCGGCGACTTAAAATAAATGGCAGGCTAAGAGATTTAATA
ACTCGAAACCTAAGCGAAAGCAAGTCTTAATAGGGCGTCAATTTAACAAAACTT
TAAATAAATTATAAAGTCATTTATTTTAGACCCGAACCTGAGTGATCTAACCATG
GTCAGGATGAAACTTGGGTGACACCAAGTGGAAGTCCGAACCGACCGATGTTGA
AAAATCGGCGGATGAACTGTGGTTAGTGGTGAAATACCAGTCGAACTCAGAGCT
AGCTGGTTCTCCCCGAAATGCGTTGAGGCGCAGCAATATATCTCGTCTATCTAGG
GGTAAAGCACTGTTTCGGTGCGGGCTATGAAAATGGTACCAAATCGTGGCAAAC
TCTGAATACTAGAAATGACGATATATTAGTGAGACTATGGGGGATAAGCTCCAT
AGTCGAGAGGGAAACAGCCCAGACCACCAGTTAAGGCCCCAAAATGATAATGAA
GTGGTAAAGGAGGTGAAAATGCAAATACAACCAGGAGGTTGGCTTAGAAGCAGC
CATCCTTTAAAGAGTGCGTAATAGCTCACTG
**SEQ ID NO:28**
   UTEX 1442 *Prototheca stagnora* 
TGTTGAAGAATGAGCCGGCGAGTTAAAAAAAATGGCATGGTTAAAGATATTTCT
CTGAAGCCATAGCGAAAGCAAGTTTTACAAGCTATAGTCATTTTTTTAGACCCG
AAACCGAGTGATCTACCCATGATCAGGGTGAAGTGTTGGTCAAATAACATGGAG
GCCCGAACCGACTAATGGTGAAAAATTAGCGGATGAATTGTGGGTAGGGGCGAA
AAACCAATCGAACTCGGAGTTAGCTGGTTCTCCCCGAAATGCGTTTAGGCGCAGC
AGTAGCAACACAAATAGAGGGGTAAAGCACTGTTTCTTTTGTGGGCTTCGAAAGT
TGTACCTCAAAGTGGCAAACTCTGAATACTCTATTTAGATATCTACTAGTGAGAC
CTTGGGGGATAAGCTCCTTGGTCAAAAGGGAAACAGCCCAGATCACCAGTTAAG
GCCCCAAAATGAAAATGATAGTGACTAAGGACGTGAGTATGTCAAAACCTCCAG
CAGGTTAGCTTAGAAGCAGCAATCCTTTCAAGAGTGCGTAATAGCTCACTG
**SEQ ID NO:29**
   UTEX 288 *Prototheca moriformis* 
TGTTGAAGAATGAGCCGGCGAGTTAAAAAGAGTGGCATGGTTAAAGATAATTCT
CTGGAGCCATAGCGAAAGCAAGTTTAACAAGCTAAAGTCACCCTTTTTAGACCCG
AAACCGAGTGATCTACCCATGATCAGGGTGAAGTGTTGGTAAAATAACATGGAG
GCCCGAACCGACTAATGGTGAAAAATTAGCGGATGAATTGTGGGTAGGGGCGAA
AAACCAATCGAACTCGGAGTTAGCTGGTTCTCCCCGAAATGCGTTTAGGCGCAGC
AGTAGCAACACAAATAGAGGGGTAAAGCACTGTTTCTTTTGTGGGCTTCGAAAGT
TGTACCTCAAAGTGGCAAACTCTGAATACTCTATTTAGATATCTACTAGTGAGAC
CTTGGGGGATAAGCTCCTTGGTCAAAAGGGAAACAGCCCAGATCACCAGTTAAG
GCCCCAAAATGAAAATGATAGTGACTAAGGATGTGGGTATGTTAAAACCTCCAG
CAGGTTAGCTTAGAAGCAGCAATCCTTTCAAGAGTGCGTAATAGCTCACTG
**SEQ ID NO:30**
   UTEX 1439, UTEX 1441 UTEX 1435 UTEX 1437 *Prototheca moriformis* 
TGTTGAAGAATGAGCCGGCGACTTAAAATAAATGGCAGGCTAAGAGAATTAATA
ACTCGAAACCTAAGCGAAAGCAAGTCTTAATAGGGCGCTAATTTAACAAAACAT
TAAATAAAATCTAAAGTCATTTATTTTAGACCCGAACCTGAGTGATCTAACCATG
GTCAGGATGAAACTTGGGTGACACCAAGTGGAAGTCCGAACCGACCGATGTTGA
AAAATCGGCGGATGAACTGTGGTTAGTGGTGAAATACCAGTCGAACTCAGAGCT
AGCTGGTTCTCCCCGAAATGCGTTGAGGCGCAGCAATATATCTCGTCTATCTAGG
GGTAAAGCACTGTTTCGGTGCGGGCTATGAAAATGGTACCAAATCGTGGCAAAC
TCTGAATACTAGAAATGACGATATATTAGTGAGACTATGGGGGATAAGCTCCAT
AGTCGAGAGGGAAACAGCCCAGACCACCAGTTAAGGCCCCAAAATGATAATGAA
GTGGTAAAGGAGGTGAAAATGCAAATACAACCAGGAGGTTGGCTTAGAAGCAGC
CATCCTTTAAAGAGTGCGTAATAGCTCACTG
**SEQ ID NO:31**
   UTEX 1533 *Prototheca wickerhamii* 
TGTTGAAGAATGAGCCGTCGACTTAAAATAAATGGCAGGCTAAGAGAATTAATA
ACTCGAAACCTAAGCGAAAGCAAGTCTTAATAGGGCGCTAATTTAACAAAACAT
TAAATAAAATCTAAAGTCATTTATTTTAGACCCGAACCTGAGTGATCTAACCATG
GTCAGGATGAAACTTGGGTGACACCAAGTGGAAGTCCGAACCGACCGATGTTGA
AAAATCGGCGGATGAACTGTGGTTAGTGGTGAAATACCAGTCGAACTCAGAGCT
AGCTGGTTCTCCCCGAAATGCGTTGAGGCGCAGCAATATATCTCGTCTATCTAGG
GGTAAAGCACTGTTTCGGTGCGGGCTATGAAAATGGTACCAAATCGTGGCAAAC
TCTGAATACTAGAAATGACGATATATTAGTGAGACTATGGGGGATAAGCTCCAT
AGTCGAGAGGGAAACAGCCCAGACCACCAGTTAAGGCCCCAAAATGATAATGAA
GTGGTAAAGGAGGTGAAAATGCAAATACAACCAGGAGGTTGGCTTAGAAGCAGC
CATCCTTTAAAGAGTGCGTAATAGCTCACTG
**SEQ ID NO:32**
   UTEX 1434 *Prototheca moriformis* 
TGTTGAAGAATGAGCCGGCGAGTTAAAAAGAGTGGCGTGGTTAAAGAAAATTCT
CTGGAACCATAGCGAAAGCAAGTTTAACAAGCTTAAGTCACTTTTTTTAGACCCG
AAACCGAGTGATCTACCCATGATCAGGGTGAAGTGTTGGTAAAATAACATGGAG
GCCCGAACCGACTAATGGTGAAAAATTAGCGGATGAATTGTGGGTAGGGGCGAA
AAACCAATCGAACTCGGAGTTAGCTGGTTCTCCCCGAAATGCGTTTAGGCGCAGC
AGTAGCAACACAAATAGAGGGGTAAAGCACTGTTTCTTTTGTGGGCTCCGAAAG
TTGTACCTCAAAGTGGCAAACTCTGAATACTCTATTTAGATATCTACTAGTGAGA
CCTTGGGGGATAAGCTCCTTGGTCGAAAGGGAAACAGCCCAGATCACCAGTTAA
 
GGCCCCAAAATGAAAATGATAGTGACTAAGGATGTGAGTATGTCAAAACCTCCA
GCAGGTTAGCTTAGAAGCAGCAATCCTTTCAAGAGTGCGTAATAGCTCACTG
**SEQ ID NO:33**
   UTEX 1438 *Prototheca zopfii* 
TGTTGAAGAATGAGCCGGCGAGTTAAAAAGAGTGGCATGGTTAAAGAAAATTCT
CTGGAGCCATAGCGAAAGCAAGTTTAACAAGCTTAAGTCACTTTTTTAGACCCG
AAACCGAGTGATCTACCCATGATCAGGGTGAAGTGTTGGTAAAATAACATGGAG
GCCCGAACCGACTAATGGTGAAAAATTAGCGGATGAATTGTGGGTAGGGGCGAA
AAACCAATCGAACTCGGAGTTAGCTGGTTCTCCCCGAAATGCGTTTAGGCGCAGC
AGTAGCAACACAAATAGAGGGGTAAAGCACTGTTTCTTTCGTGGGCTTCGAAAG
TTGTACCTCAAAGTGGCAAACTCTGAATACTCTATTTAGATATCTACTAGTGAGA
CCTTGGGGGATAAGCTCCTTGGTCAAAAGGGAAACAGCCCAGATCACCAGTTAA
GGCCCCAAAATGAAAATGATAGTGACTAAGGATGTGAGTATGTCAAAACCTCCA
GCAGGTTAGCTTAGAAGCAGCAATCCTTTCAAGAGTGCGTAATAGCTCACTG
**SEQ ID NO:34**
   UTEX 1436 *Prototheca moriformis* 
TGTTGAAGAATGAGCCGGCGACTTAGAAAAGGTGGCATGGTTAAGGAAATATTC
CGAAGCCGTAGCAAAAGCGAGTCTGAATAGGGCGATAAAATATATTAATATTTA
GAATCTAGTCATTTTTTCTAGACCCGAACCCGGGTGATCTAACCATGACCAGGAT
GAAGCTTGGGTGATACCAAGTGAAGGTCCGAACCGACCGATGTTGAAAAATCGG
CGGATGAGTTGTGGTTAGCGGTGAAATACCAGTCGAACCCGGAGCTAGCTGGTT
CTCCCCGAAATGCGTTGAGGCGCAGCAGTACATCTAGTCTATCTAGGGGTAAAGC
ACTGTTTCGGTGCGGGCTGTGAGAACGGTACCAAATCGTGGCAAACTCTGAATAC
TAGAAATGACGATGTAGTAGTGAGACTGTGGGGGATAAGCTCCATTGTCAAGAG
GGAAACAGCCCAGACCACCAGCTAAGGCCCCAAAATGGTAATGTAGTGACAAAG
GAGGTGAAAATGCAAATACAACCAGGAGGTTGGCTTAGAAGCAGCCATCCTTTA
AAGAGTGCGTAATAGCTCACTG
**SEQ ID NO:35**
   Forward primer SZ5434
   GTCCCTGCCCTTTGTACACAC
**SEQ ID NO:36**
   Reverse primer SZ5435
   TTGATATGCTTAAGTTCAGCGGG
**SEQ ID NO:37**
   *Rhodotorula glutinis* DSMZ-DSM 70398 and *Lipomyces tetrasporus* CBS 5911 
CGCCCGTCGCTACTACCGATTGAATGGCTTAGTGAGGCCTCCGGATTGGCTATTG
GGAGCTCGCGAGAGCACCTGACTGCCGAGAAGTTGTACGAACTTGGTCATTTAG
AGGAAGTAAAAGTCGTAACAAGGTTTCCGTAGGTGAACCTGCGGAAGGATCATT
AGTGAATATTAGGGTGTCCAACTTAACTTGGAGCCCGACCCTCACTTTCTAACCC
TGTGCATTTGTCTTGGGTAGTAGCTTGCGTCAGCGAGCGAATCCCATTTCACTTAC
AAACACAAAGTCTATGAATGTAACAAATTTATAACAAAACAAAACTTTCAACAA
 
CGGATCTCTTGGCTCTCGCATCGATGAAGAACGCAGCGAAATGCGATACGTAAT
GTGAATTGCAGAATTCAGTGAATCATCGAATCTTTGAACGCACCTTGCGCTCCAT
GGTATTCCGTGGAGCATGCCTGTTTGAGTGTCATGAATTCTTCAACCCACCTCTTT
CTTAGTGAATCAGGCGGTGTTTGGATTCTGAGCGCTGCTGGCTTCGCGGCCTAGC
TCGCTCGTAATGCATTAGCATCCGCAATCGAACTTCGGATTGACTCGGCGTAATA
GACTATTCGTTGAGGATTCTGGTCTCTGACTGGAGCCGGGTAAGGTTAAAGGGAG
CTACTAATCCTCATGTCTATCTTGAGATTAGACCTCAAATCAGGTAGGACTA
**SEQ ID NO:38**
   *Rhodotorula glutinis var. glutinis* CBS 3044 and *Lipomyces tetrasporus* CBS 8664 
CGCCCGTCGCTACTACCGATTGAATGGCTTAGTGAGGCCTCCGGATTGGCTATTG
GGAGCTCGCGAGAGCACCCGACTGCCGAGAAGTTGTACGAACTTGGTCATTTAG
AGGAAGTAAAAGTCGTAACAAGGTTTCCGTAGGTGAACCTGCGGAAGGATCATT
AGTGAATATTAGGGCGTCCAACTTAACTTGGAGCCCGAACTCTCACTTTCTAACC
CTGTGCATCTGTTTCTGGTCAGTAGCTCTCTCGGGAGTGAACGCCATTCACTTAA
AACACAAAGTCTATGAATGTATAAAATTATAACAAAACAAAACTTTCAACAAC
GGATCTCTTGGCTCTCGCATCGATGAAGAACGCAGCGAAATGCGATAAGTAATG
TGAATTGCAGAATTCAGTGAATCATCGAATCTTTGAACGCACCTTGCGCTCTCTG
GTATTCCGGAGAGCATGCCTGTTTGAGTGTCATGAAATCTTCAACCCTCTCTTTTC
TTAATGAATCGAGAGGTGCTTGGATCCTGAGCGCTGCTGGCTTCGGCCTAGCTCG
TTCGTAATGCATTAGCATCCGCAATCGAACTTCGGATTGACTTGGCGTAATAGAC
TATTCGCTGAGGATTCTGGTCTCGTACCAGAGCCGGGTTGGGTTAAAGGAAGCTT
CTAATCCTAAAAGTCTAACTTTGATTAGATCTCAAATCAGGTAGGACTA
**SEQ ID NO:39**
   *Lipomyces tetrasporus* CBS 1808 and *Lipomyces tetrasporus* CBS 1810 
CGCCCGTCGCTACTACCGATTGAATGGCTTAGTGAGGCCTTCGGACTGGCTCCAG
AAAATGGGAAACCATTATCAGGAGCTGGAAAGTTGGTCAAACTTGGTCATTTAG
AGGAAGTAAAAGTCGTAACAAGGTTTCCGTAGGTGAACCTGCGGAAGGATCATT
ACTGAGTATTTGTCTTTTAAAGACATCTCTCTATCCATAAACTCTTTTTCTAAAA
AGACATGATTTACACAATTAGTCTGAATGATTATATAAAAATCTTCAAAACTTTC
AACAACGGATCTCTTGGTTCTCGCATCGATGAAGAACGCAGCAAAATGCGATAA
GTATTGTGAATTGCAGGATTTTGTGAATCATCGAATTTTTGAACGCACATTGCAC
CTTCTGGTATTCCGGAGGGTATACCTGTTTGAGCGTCATTTATATACTCAAAACTT
TGTTTTGGTGATGGGCACATATCTGGTGAGAGCTAGATTTGCCTGAAATATAGTG
GTAGAGATTGCTACGAGTTATGCAAGTTAGCCAATGCTATTAAGTTAATTCGTTG
GTGAAGCATGCGGAGCTTTAGCGGTCGCCTTCCTTAACTATTGGAATTTTTCTAAT
TTTGACCTCAAATCAGGCAGGAGTA
**SEQ ID NO:40**
   *Lipomyces starkeyi* CBS 1809 and *Trichosporon montevideense* CBS 8261 
CGCCCGTCGCTACTACCGATTGAATGGCTTAGTGAGACCCTCGGATTGGCGTTAG
GAAGCCGGCAACGGCATCCTTTGGCCGAGAAGTTGGTCAAACTTGGTCATTTAGA
GGAAGTAAAAGTCGTAACAAGGTTTCCGTAGGTGAACCTGCGGAAGGATCATTA
GTGATTGCCTTTATAGGCTTATAACTATATCCACTTACACCTGTGAACTGTTCTAT
TACTTGACGCAAGTCGAGTATTTTTACAAACAATGTGTAATGAACGTCGTTTTAT
TATAACAAAATAAAACTTTCAACAACGGATCTCTTGGCTCTCGCATCGATGAAGA
 
ACGCAGCGAATTGCGATAAGTAATGTGAATTGCAGAATTCAGTGAATCATCGAA
TCTTTGAACGCAGCTTGCGCTCTCTGGTATTCCGGAGAGCATGCCTGTTTCAGTGT
CATGAAATCTCAACCACTAGGGTTTCCTAATGGATTGGATTTGGGCGTCTGCGAT
CTCTGATCGCTCGCCTTAAAAGAGTTAGCAAGTTTGACATTAATGTCTGGTGTAA
TAAGTTTCACTGGGTCCATTGTGTTGAAGCGTGCTTCTAATCGTCCGCAAGGACA
ATTACTTTGACTCTGGCCTGAAATCAGGTAGGACTA
**SEQ ID NO:41**
   *Yarrowia lipolytica* CBS 6331 
CGCCCGTCGCTACTACCGATTGAATGGTTTAGTGAGACCTTGGGAGGGCGAGATG
AGGGGGCAACCCCTTTTGAACATCCAAACTTGGTCAAACTTGATTATTTAGAGG
AAGTAAAAGTCGTAACAAGGTTTCCGTAGGTGAACCTGCGGAAGGATCATTATT
GATTTTATCTATTTCTGTGGATTTCTGGTATATTACAGCGTCATTTTATCTCAATTA
TAACTATCAACAACGGATCTCTTGGCTCTCACATCGATGAAGAACGCAGCGAACC
GCGATATTTTTTGTGACTTGCAGATGTGAATCATCAATCTTTGAACGCACATTGC
GCGGTATGGCATTCCGTACCGCACGGATGGAGGAGCGTGTTCCCTCTGGGATCGC
ATTGCTTTCTTGAAATGGATTTTTTAAACTCTCAATTATTACGTCATTTCACCTCCT
TCATCCGAGATTA
**SEQ ID NO:42**
   *Cryptococcus curvatus* CBS 5324, *Rhodotorula mucilaginosa var. mucilaginosa* CBS 316, *Cryptococcus curvatus* CBS 570, *Cryptococcus curvatus* CBS 2176, *Cryptococcus curvatus* CBS 2744, *Cryptococcus curvatus* CBS 2754, *Cryptococcus curvatus* CBS 2829, *Cryptococcus curvatus* CBS 5163, and *Cryptococcus curvatus* CBS 5358 
CGCCCGTCGCTACTACCGATTGAATGGCTTAGTGAGATTTCTGGATTGGCGTTAG
GAAGCCGGCAACGGCATCCTTTGGCTGAGAAGTTACTCAAACTTGGTCATTTAGA
GGAAGTAAAAGTCGTAACAAGGTTTCCGTAGGTGAACCTGCGGAAGGATCATTA
GTGATTTGCCTTCGGGCTAAACTATATCCATAACACCTGTGAACTGTTGATTGAC
TTCGGTCAATATTTTTACAAACATTGTGTAATGAACGTCATGTTATAATAACAAA
TATAACTTTCAACAACGGATCTCTTGGCTCTCGCATCGATGAAGAACGCAGCGAA
ATGCGATAAGTAATGTGAATTGCAGAATTCAGTGAATCATCGAATCTTTGAACGC
AACTTGCGCTCTCTGGTATTCCGGAGAGCATGCCTGTTTGAGTGTCATGAAATCT
CAACCATTAGGGTTTCTTAATGGCTTGGATTTGGACGTTTGCCAGTCAAATGGCT
CGTCTTAAAAGAGTTAGTGAATTTAACATTTGTCTTCTGGCGTAATAAGTTTCGCT
GGGCTGATAGTGTGAAGTTTGCTTCTAATCGTCCGCAAGGACAATTCTTGAACTC
TGGCCTCAAATCAGGTAGGACTA
**SEQ ID NO:43**
   *Trichosporon sp.* CBS 7617 
CGCCCGTCGCTACTACCGATTGAATGGCTTAGTGAGACCCTCGGATTGGCGTTAA
GAAGCCGGCAACGGCATCTTTGGCCGAGAAGTTGGTCAAACTTGGTCATTTAGA
GGAAGTAAAAGTCGTAACAAGGTTTCCGTAGGTGAACCTGCGGAAGGATCATTA
GTGAATTGCTCTTTGAGCGTTAAACTATATCCATCTACACCTGTGAACTGTTGATT
GACTTCGGTCAATTACTTTTACAAACATTGTGTAATGAACGTCATGTTATTATAAC
AAAAATAACTTTCAACAACGGATCTCTTGGCTCTCGCATCGATGAAGAACGCAGC
GAAATGCGATAAGTAATGTGAATTGCAGAATTCAGTGAATCATCGAATCTTTGAA
CGCAACTTGCGCTCTCTGGTATTCCGGAGAGCATGCCTGTTTGAGTATCATGAAA
 
TCTCAACCATTAGGGTTTCTTAATGGCTTGGATTTGGGCGCTGCCACTTGCCTGGC
TCGCCTTAAAAGAGTTAGCGTATTAACTTGTCGATCTGGCGTAATAAGTTTCGCT
GGTGTAGACTTGAGAAGTGCGCTTCTAATCGTCCTCGGACAATTCTTGAACTCTG
GTCTCAAATCAGGTAGGACTA
**SEQ ID NO:44**
   *Sporobolomyces alborubescens* CBS 482 
CGCCCGTCGCTACTACCGATTGAATGGCTTAGTGAGGCCTCCGGATTGGCTATTG
GGAGCTCGCGAGAGCACCCGACTGCCGAGAAGTTGTACGAACTTGGTCATTTAG
AGGAAGTAAAAGTCGTAACAAGGTTTCCGTAGGTGAACCTGCGGAAGGATCATT
AGTGAATATAGGACGTCCAACTTAACTTGGAGTCCGAACTCTCACTTTCTAACCC
TGTGCACTTGTTTGGGATAGTAACTCTCGCAAGAGAGCGAACTCCTATTCACTTA
TAAACACAAAGTCTATGAATGTATTAAATTTTATAACAAAATAAAACTTTCAACA
ACGGATCTCTTGGCTCTCGCATCGATGAAGAACGCAGCGAAATGCGATAAGTAA
TGTGAATTGCAGAATTCAGTGAATCATCGAATCTTTGAACGCACCTTGCGCTCCA
TGGTATTCCGTGGAGCATGCCTGTTTGAGTGTCATGAATACTTCAACCCTCCTCTT
TCTTAATGATTGAAGAGGTGTTTGGTTTCTGAGCGCTGCTGGCCTTTACGGTCTAG
CTCGTTCGTAATGCATTAGCATCCGCAATCGAATTTCGGATTGACTTGGCGTAAT
AGACTATTCGCTGAGGAATTCTAGTCTTCGGATTAGAGCCGGGTTGGGTTAAAGG
AAGCTTCTAATCAGAATGTCTACATTTTAAGATTAGATCTCAAATCAGGTAGGAC
TA
**SEQ ID NO:45**
   *Rhodotorula glutinis var. glutinis* CBS 324 
CGCCCGTCGCTACTACCGATTGAATGGCTTAGTGAGGCCTCCGGATTGGCTATTG
GGAGCTCGCGAGAGCACCCGACTGCCGAGAAGTTGTACGAACTTGGTCATTTAG
AGGAAGTAAAAGTCGTAACAAGGTTTCCGTAGGTGAACCTGCGGAAGGATCATT
AGTGAATCTAGGACGTCCAACTTAACTTGGAGTCCGAACTCTCACTTTCTAACCC
TGTGCATCTGTTTTAAAATTGGCTAGTAGCTCTTCGGAGCGAACCACCATTTTTCA
CTTATACAAACACAAAGTCTATGAATGTAAACAAATTTATAACAAAACAAAACT
TTCAACAACGGATCTCTTGGCTCTCGCATCGATGAAGAACGCAGCGAAATGCGAT
ACGTAATGTGAATTGCAGAATTCAGTGAATCATCGAATCTTTGAACGCACCTTGC
GCTCCTTGGTATTCCGAGGAGCATGCCTGTTTGAGTGTCATGAAATCTTCAACCC
ACCTCTTTCTTAGTGAATCTGGTGGTGCTTGGTTTCTGAGCGCTGCTCTGCTTCGG
CTTAGCTCGTTCGTAATGCATTAGCATCCGCAACCGAAACTTCGGATTGACTTGG
CGTAATAGACTATTCGCTGAGGATTCCAGACTTGTTCTGGAGCCGAGTTGGGTTA
AAGGAAGCTTCTAATCCTAAAGTCTATTTTTGATTAGATCTCAAATCAGGTAGG
ACTA
**SEQ ID NO:46**
   *Rhodotorula glutinis var. glutinis* CBS 4476 
CGCCCGTCGCTACTACCGATTGAATGGCTTAGTGAGGCTCCGGATTGGCTTCTG
GGAGCCGGCAACGGCACCTAGTCGCTGAGAAGTTGGACGAACTTGGTCATTTAG
AGGAAGTAAAAGTCGTAACAAGGTTTCCGTAGGTGAACCTGCGGAAGGATCATT
AATGAAATGCAAGGACGCTCTTTTTAGAGGTCCGACCCAATTCATTTTCTCACAC
TGTGCACACACTACTTTTTACACCATTTTTAACACTTGAAGTCTAAGAATGTAAA
CAGTCTCTTAATTGAGCATAAAATTTAAACAAAACTTTCAGCAACGGATCTCTTG
 
GCTCTCCCATCGATGAAGAACGCAGCGAAATGCGATACGTAATGTGAATTGCAG
AATTCAGTGAATCATCGAATCTTTGAACGCACCTTGCACTCTTTGGTATTCCGAA
GAGTATGTCTGTTTGAGTGTCATGAAACTCTCAACCCCCCTGTTTTGTAATGAACC
AGGCGTGGGCTTGGATTATGGCTGCTGCCGGCGTAATTGTCGACTCGGCTGAAAT
ACACGAGCTACCCATTTCATAAGAAATAGACGGTTTGACTCGGCGTAATAACATA
TTTCGCTGAGGACGTCACATTCTTTACCTAGTGGTGCTTCTAATGCGACATCTAAA
CTTTAAGCTTTAGACCTCAAATCAGTCAGGACTA
**SEQ ID NO:47**
   *Trichosporon behrend* CBS 5581 
CGCCCGTCGCTACTACCGATTGAATGGCTTAGTGAGACCCTCGGATTGGCGTTAG
GAAGCCGGCAACGGCATCCTTTGGCCGAGAAGTTGGTCAAACTTGGTCATTTAGA
GGAAGTAAAAGTCGTAACAAGGTTTCCGTAGGTGAACCTGCGGAAGGATCATTA
GTGATTGCCTTCATAGGCTTAAACTATATCCACATACACCTGTGAACTGTTCCAC
CACTTGACGCAAGTCGAGTGTTTTTACAAACAATGTGTAATGAACGTCGTTTTAT
TATAACAAAATAAAACTTTCAACAACGGATCTCTTGGCTCTCGCATCGATGAAGA
ACGCAGCGAATTGCGATAAGTAATGTGAATTGCAGAATTCAGTGAATCATCGAA
TCTTTGAACGCAGCTTGCGCTCTCTGGTATTCCGGAGAGCATGCCTGTTTCAGTGT
CATGAAATCTCAACCACTAGGGTTTCCTAATGGATTGGATTTGGGCGTCTGCGAT
CTCTGATCGCTCGCCTTAAAAGAGTTAGCAAGTTTGACATTAATGTCTGGTGTAA
TAAGTTTCACTGGGTCCATTGTGTTGAAGCGTGCTTCTAATCGTCCGCAAGGACA
ATTACTTTGACTCTGGCCTGAAATCAGGTAGGACTA
**SEQ ID NO:48**
   *Geotrichum histeridarum* CBS 9892 
CGCCCGTCGCTACTACCGATCGAATGGCTTAGTGAGGCTTCCGGATTGATTTGGG
AGAGAGGGCGACTTTTTCCTGGAACGAGAAGCTAGTCAAACTTGGTCATTTAGA
GGAAGTAAAAGTCGTAACAAGGTTTCCGTAGGTGAACCTGCGGAAGGATCATTA
GAAAAATGCGATATTAGTGGTTTATTTTGCTCGCCGAAAGGCAAACTTTTAACAT
ACCTACCTTTTTTTAACTATAAAAACTTTTAACAACGGATCTCTTGGTTCTCGCAT
CGATGAAGAACGCAGCGAATTGCGATACGTTTTGTGAATTGCAGAAGTGAATCA
TCAATCTTTGAACGCACATTGCGCCTGGTGGTATTCCGCCAGGCATACCTGTTTG
AGCGTTGTTCTCTCTGGGATTGTCTACTTTCCTCAAAGAAATTAAACAAACAAGT
TTGACACAACACCTCAACCTCAGATCAGGTAGGACTA
**SEQ ID NO:49**
   *Rhodotorula aurantiaca* CBS 8411 and *Cryptococcus curvatus* CBS 8126 
CGCCCGTCGCTACTACCGATTGAATGGCTTAGTGAGGCCTTCGGATTGGCTTCTG
GGAGCCGGCAACGGCACCTAGTCGCTGAGAAGTTTGACGAACTTGGTCATTTAG
AGGAAGTAAAAGTCGTAACAAGGTTTCCGTAGGTGAACCTGCGGAAGGATCATT
AATGAATTTTAGGACGTTCTTTTTAGAAGTCCGACCCTTTCATTTTCTTACACTGT
GCACACACTTCTTTTTTACACACACTTTTAACACCTTAGTATAAGAATGTAATAGT
CTCTTAATTGAGCATAAATAAAAACAAAACTTTCAGCAACGGATCTCTTGGCTCT
CGCATCGATGAAGAACGCAGCGAATTGCGATAAGTAATGTGAATTGCAGAATTC
AGTGAATCATCGAATCTTTGAACGCACCTTGCACTCTTTGGTATTCCGAAGAGTA
TGTCTGTTTGAGTGTCATGAAACTCTCAACCCCCCTATTTTGTAATGAGATGGGTG
TGGGCTTGGATTATGGTTGTCTGTCGGCGTAATTGCCGGCTCAACTGAAATACAC
 
GAGCAACCCTATTGAAATAAACGGTTTGACTTGGCGTAATAATTATTTCGCTAAG
GACGCTTTCTTCAAATATAAGAGGTGCTTCTAATTCGCTTCTAATAGCATTTAAGC
TTTAGACCTCAAATCAGTCAGGACTA
**SEQ ID NO:50**
   *Trichosporon domesticum* CBS 8111 
CGCCCGTCGCTACTACCGATTGAATGGCTTAGTGAGACCTCCGGATTGGCGTTGA
GAAGCCGGCAACGGCATCTCTTGGCTGAGAAGTTGGTCAAACTTGGTCATTTAGA
GGAAGTAAAAGTCGTAACAAGGTTTCCGTAGGTGAACCTGCGGAAGGATCATTA
GTGATTGCCTTAATTGGCTTAAACTATATCCATCTACACCTGTGAACTGTTTGATT
GAATCTTCGGATTCGATTTTATACAAACATTGTGTAATGAACGTCATTATATTATA
ACAAAAAAAAAACTTTCAACAACGGATCTCTTGGCTCTCGCATCGATGAAGAAC
GCAGCGAAATGCGATAAGTAATGTGAATTGCAGAATTCAGTGAATCATCGAATC
TTTGAACGCAACTTGCGCTCTCTGGTATTCCGGAGAGCATGCCTGTTTGAGTGTC
ATGAAATCTCAACCATTAGGGTTTCTTAATGGCTTGGATTTGGAGGTTTGCCAGT
CTGACTGGCTCCTCTTAAAAGAGTTAGCAAGTTGAACTATTGCTATCTGGCGTAA
TAAGTTTCGCTGGAATGGTATTGTGAAGCGTGCTTCTAATCGTCTTCGGACAATTT
TTTGACTCTGGCCTCAAATCAGGTAGGACTA
**SEQ ID NO:51**
   *Rhodotorula toruloides* CBS 8761 
CGCCCGTCGCTACTACCGATTGAATGGCTTAGTGAGGCCTCCGGATTGGCTATCG
GGAGCTCGCGAGAGCACCTGACTGCCGAGAAGTTGTACGAACTTGGTCATTTAG
AGGAAGTAAAAGTCGTAACAAGGTTTCCGTAGGTGAACCTGCGGAAGGATCATT
AGTGAATATTAGGGTGTCCAACTTAACTTGGAGCCCGACCCTCACTTTCTAACCC
TGTGCATTTGTCTTGGGTAGTAGCTCGTGTCAGCGAGCGAATCCCATTTCACTTAC
AAACACAAAGTCTATGAATGTAACAAATTTATAACAAACAAAACTTTCAACAAC
GGATCTCTTGGCTCTCGCATCGATGAAGAACGCAGCGAAATGCGATACGTAATGT
GAATTGCAGAATTCAGTGAATCATCGAATCTTTGAACGCACCTTGCGCTCCATGG
TATTCCGTGGAGCATGCCTGTTTGAGTGTCATGAATTCTTCAACCCACCTCTTTCT
TAGTGAATCAGGCGGTGTTTGGATTCTGAGCGTTGCTGGCTTCGCGGCCTAGCTC
GCTCGTAATGCATTAGCATCCGCAATCGAACTTCGGATTGACTCGGCGTAATAGA
CTATTCGCTGAGGATTCTGGTCTCTGACTGGAGCCGGGTAAGATTAAAGGAAGCT
ACTAATCCTCATGTCTATCTTTTGAGATTAGACCTCAAATCAGGTAGGACTA
**SEQ ID NO:52**
   *Rhodotorula terpendoidalis* CBS 8445 
CGCCCGTCGCTACTACCGATTGAATGGCTTAGTGAGGCCTCCGGACTGGCTATTG
GGATCTCGCGAGAGAACCTGACTGCTGGGAAGTTGTACGAACTTGGTCATTTAGA
GGAAGTAAAAGTCGTAACAAGGTTTCCGTAGGTGAACCTGCGGAAGGATCATTA
ATGAATATTAGGGTGCTCTTTTCATCAAAGAGGCCTGACCTTCATTCTTCTACCCT
GTGCACTATTCAAACATTCGGCAGTTGGTAATTTGGCTTGTAAAAGAGCCAGACG
ACTCTGCTGAATTCACTCTTAAACTCTAAAGTATAAGAATGTTACAAATAAAACA
AATAAAACTTTCAACAACGGATCTCTTGGCTCTCGCATCGATGAAGAACGCAGCG
AAATGCGATAAGTAATGTGAATTGCAGAATTCAGTGAATCATCGAATCTTTGAAC
GCACCTTGCGCTCGCTGGTATTCCGGCGAGCATGCCTGTTTGAGTGTCATGAAAA
CCTCAACCCTTCAATTCCTTGTTGAATTGTAAGGTGTTTGGATTCTGAATGTTTGC
 
TGGCTTGAAGGGCCCTTGGCTACTTCAAAAGCGAAGCTCATTCGTAATACATTAG
CATCTCAATTTCGAATATTCGGATTGACTCGGCGTAATAGACTTTATTCGCTGAG
GACACCTTCACAAGGTGGCCGAATTTCGAGGTAGAAGCTTCCAATTCGATCAAA
AGTCACTCTTAGTTTAGACCTCAGATCAGGCAGGACTA
**SEQ ID NO:53**
   *Yarrowia lipolytica* CBS 10144 
CGCCCGTCGCTACTACCGATTGAATGGTTTAGTGAGACCTTGGGAGGGCGAGATG
AGGGGGCAACCCCTTTTGAACATCCAAACTTGGTCAAACTTGATTATTTAGAGG
AAGTAAAAGTCGTAACAAGGTTTCCGTAGGTGAACCTGCGGAAGGATCATTATT
GATTTTATCTATTTCTGTGGATTTCTGGTATATTACAGCGTCATTTTATCTCAATTA
TAACTATCAACAACGGATCTCTTGGCTCTCACATCGATGAAGAACGCAGCGAACC
GCGATATTTTTTGTGACTTGCAGATGTGAATCATCAATCTTTGAACGCACATTGC
GCGGTATGGTATTCCGTACCGCACGGATGGAGGAGCGTGTTCCCTCTGGGATCGC
ATTGCTTTCTTGAAATGGATTTTTTAAACTCTCAATTATTACGTCATTTCACCTCCT
TCATCCGAGATTA
**SEQ ID NO:54**
   *Rhodotorula glutinis var. glutinis* CBS 5805 
CGCCCGTCGCTACTACCGATTGAATGGCTTAGTGAGGCCTCCGGATTGGCTATTG
GGAGCTCGCGAGAGCACCTGACTGCCGAGAAGTTGTACGAACTTGGTCATTTAG
AGGAAGTAAAAGTCGTAACAAGGTTTCCGTAGGTGAACCTGCGGAAGGATCATT
AGTGAATATTAGGGTGTCCAACTTAACTTGGAACCCGACCCTCACTTTCTAACCC
TGTGCATTTGTCTTGGGTAGTAGCTTGCGTCGGCGAGCGAATCCCATTTCACTTAC
AAACACAAAGTCTATGAATGTAACAAATTTATAACAAACAAAACTTTCAACAAC
GGATCTCTTGGCTCTCGCATCGATGAAGAACGCAGCGAAATGCGATACGTAATGT
GAATTGCAGAATTCAGTGAATCATCGAATCTTTGAACGCACCTTGCGCTCCATGG
TATTCCGTGGAGCATGCCTGTTTGAGTGTCATGAATTCTTCAACCCACCTATTTCT
TAGTGAATCAGGCGGTGTTTGGATTCTGAGCGCTGCTGGCCTCACGGCCTAGCTC
GCTCGTAATGCATTAGCATCCGCAATCGAACTTCGGATTGACTCGGCGTAATAGA
CTATTCGCTGAGGATTCTGGTCTCTGACTGGAGCCGGGTGAGATTAAAGGAAGCT
ACTAATCCTCATGTCTATCTTGAGATTAGACCTCAAATCAGGTAGGACTA
**SEQ ID NO:55**
   *Yarrowia lipolytica* CBS 10143 
GTCCCTGCCCTTTGTACACACCGCCCGTCGCTACTACCGATTGAATGGTTTAGTG
AGACCTTGGGAGGGCGAGATGAGGGGGCAACCCCTTCTGAACATCCAAACTTG
GTCAAACTTGATTATTTAGAGGAAGTAAAAGTCGTAACAAGGTTTCCGTAGGTGA
ACCTGCGGAAGGATCATTATTGATTTTATCTATTTCTGTGGATTTCTATTCTATTA
CAGCGTCATTTTATCTCAATTATAACTATCAACAACGGATCTCTTGGCTCTCACAT
CGATGAAGAACGCAGCGAACCGCGATATTTTTTGTGACTTGCAGATGTGAATCAT
CAATCTTTGAACGCACATTGCGCGGTATGGCATTCCGTACCGCACGGATGGAGGA
GCGTGTTCCCTCTGGGATCGCATTGCTTTCTTGAAATGGATTTTTTAAACTCTCAA
TTATTACGTCATTTCACCTCCTTCATCCGAGATTACCCGCTGAACTTAAGCATATC
AA
**SEQ ID NO:56**
   *Lipomyces tetrasporus* CBS 5607 
CGCCCGTCGCTACTACCGATTGAATGGCTTAGTGAGGCCTCCGGATTGGCTATTG
GGAGCTCGCGAGAGCACCTGACTGCTGAGAAGTTGTACGAACTTGGTCATTTAG
AGGAAGTAAAAGTCGTAACAAGGTTTCCGTAGGTGAACCTGCGGAAGGATCATT
AGTGAATCTAGGACGTCCAACTTAACTTGGAGTCCGAAATCTCACTTTCTAACCC
TGTGCATCTGTTAATTGGAATAGTAGCTCTTCGGAGTGAACCACCATTCACTTAT
AAAACACAAAGTCTATGAATGTATACAAATTTATAACAAAACAAAACTTTCAAC
AACGGATCTCTTGGCTCTCGCATCGATGAAGAACGCAGCGAAATGCGATACGTA
ATGTGAATTGCAGAATTCAGTGAATCATCGAATCTTTGAACGCACCTTGCGCTCC
TTGGTATTCCGAGGAGCATGCCTGTTTGAGTGTCATGAAATCTTCAACCCACCTC
TTTCTTAGTGAATCTGGTGGTGCTTGGTTTCTGAGCGCTGCTCTGCTTCGGCTTAG
CTCGTTCGTAATGCATTAGCATCCGCAACCGAACTTCGGATTGACTTGGCGTAAT
AGACTATTCGCTGAGGATTCTAGTTTACTAGAGCCGAGTTGGGTTAAAGGAAGCT
CCTAATCCTAAAGTCTATTTTTGATTAGATCTCAAATCAGGTAGGACTA
**SEQ ID NO:57**
   *Yarrowia lipolytica* CBS 5589 
CGCCCGTCGCTACTACCGATTGAATGGTTTAGTGAGACCTTGGGAGGGCGAGATG
AGGGGGGCAACCCCTTCTGAACATCCAAACTTGGTCAAACTTGATTATTTAGAGG
AAGTAAAAGTCGTAACAAGGTTTCCGTAGGTGAACCTGCGGAAGGATCATTATT
GATTTTATCTATTTCTGTGGATTTCTATTCTATTACAGCGTCATTTTATCTCAATTA
TAACTATCAACAACGGATCTCTTGGCTCTCACATCGATGAAGAACGCAGCGAACC
GCGATATTTTTTGTGACTTGCAGATGTGAATCATCAATCTTTGAACGCACATTGC
GCGGTATGGCATTCCGTACCGCACGGATGGAGGAGCGTGTTCCCTCTGGGATCGC
ATTGCTTTCTTGAAATGGATTTTTTTAAACTCTCAATTATTACGTCATTTCACCTCC
TTCATCCGAGATTA
**SEQ ID NO:58**
   *Lipomyces tetrasporus* CBS 8724 
CGCCCGTCGCTACTACCGATTGAATGGCTTAGTGAGGCCTTCGGACTGGCTCCAG
AAAATGGGAAACCATTATCAGGAGCTGGAAAGTTGGTCAAACTTGGTCATTTAG
AGGAAGTAAAAGTCGTAACAAGGTTTCCTTCCGTAGCACTTACTGAAGCTTTAGC
AGCCCGAAAAGGCGAATGCTAGCGACTATAAATAAATATGGCGTTCTTAAATGC
TAGTCTCTGATTAGAGGCGACATTGCCAAATTGCGGGGACATCCTAAAGATCTTG
ATACCAAGCTGGTAGTCGAAAGACGCCAGTGGCCGAGCTAACAGCCCTGGGTAT
GGTAATAATTCAAGATATGGAACAATGGGTAATCCGCAGCCAAGTCCTAAACTA
CGCAAGTAGCATGGATGCAGTTCACAGGCCAAATGGTGATGGGTAGATTACTAA
ATCTGCTTAAGATATGGTCGGTCCCGCTGTGAGAGCAGATGGGAAGCTACAAAG
CAGACTCGTGAGTTTGCGCAAACGTAACTAAAAACGTTCCGTAGGTGAACCTGC
GGAAGGATCATTACTGAGTATTTGTCTTTTAAAGACATCTCTCTATCCATAAACTC
TTTTTTCTAAAAAGACATGATTTACACAATTAGTCTGAATGATTATATAAAAATC
TTCAAAACTTTCAACAACGGATCTCTTGGTTCTCGCATCGATGAAGAACGCAGCA
AAATGCGATAAGTATTGTGAATTGCAGGATTTTGTGAATCATCGAATTTTTGAAC
GCACATTGCACCTTCTGGTATTCCGGAGGGTATACCTGTTTGAGCGTCATTTATAT
ACTCAAAACTTCGTTTTGGTGATGGGCACATATCTGGTGAGAGCTAGATTTGCCT
GAAATATAGTGGTAGAGATTGCTACGAGTTATGCAAGTTAGCCAATGCTATTAAG
 
TTAATTCGTTGGTGAAGCATGCGGAGCTTTAGTGATCGCCTTCCTTAACTATTGG
AATTTTTCTAATTTTGACCTCAAATCAGGCAGGAGTA
**SEQ ID NO:59**
   *Rhodosporidium sphaerocarpum* CBS 2371 
CGCCCGTCGCTACTACCGATTGAATGGCTTAGTGAGGCCTCCGGACCGGCTATTG
GGAGCTCGCGAGAGCACCCGACTGCTGGGAAGTTGTACGAACTTGGTCATTTAG
AGGAAGTAAAAGTCGTAACAAGGTTTCCGTAGGTGAACCTGCGGAAGGATCATT
AGTGAATATAGGACGTCCAACTTAACTTGGAGTCCGAACTCTCACTTTCTAACCC
TGTGCATTTGTTTGGGATAGTAGCCTCTCGGGGTGAACTCCTATTCACTCATAAA
CACAAAGTCTATGAATGTATTTAATTTATAACAAAATAAAACTTTCAACAACGGA
TCTCTTGGCTCTCGCATCGATGAAGAACGCAGCGAAATGCGATAAGTAATGTGA
ATTGCAGAATTCAGTGAATCATCGAATCTTTGAACGCACCTTGCGCTCCATGGTA
TTCCGTGGAGCATGCCTGTTTGAGTGTCATGAATACTTCAACCCTCCTCTTTTCTA
GTGAAAGAGAAGGTGCTTGGTTTCTGAGCGTTTTGCTGGCCTCACGGTCGAGCTC
GCTCGTAATGCATTAGCATCCGCAATCGAACTTCGGATTGACTTGGCGTAATAGA
CTATTCGCTGAGGAATTCTAATCTTCGGATTAGAGCCGGGTTGGGTTAAAGGAAG
CTTCTAATCCTAATGTCTATATTTTAGATTAGATCTCAAATCAGGTAGGACTA
**SEQ ID NO:60**
   *Trichosporon brassicae* CBS 6382 
CGCCCGTCGCTACTACCGATTGAATGGCTTAGTGAGACCTCCGGATTGGCGTTGA
GAAGCCGGCAACGGCATCTCTTGGCCGAGAAGTTGGTCAAACTTGGTCATTTAGA
GGAAGTAAAAGTCGTAACAAGGTTTCCGTAGGTGAACCTGCGGAAGGATCATTA
GTGATTGCCTTAATTGGCTTAAACTATATCCAACTACACCTGTGAACTGTTCGATT
GAATCTTCGATTCAATTTTACAAACATTGTGTAAAGAACGTCATTAGATCATAAC
AAAAAAAAACTTTTAACAACGGATCTCTTGGCTCTCGCATCGATGAAGAACGCA
GCGAAATGCGATAAGTAATGTGAATTGCAGAATTCAGTGAATCATCGAATCTTTG
AACGCAACTTGCGCTCTCTGGTATTCCGGAGAGCATGCCTGTTTGAGTGTCATGA
AATCTCACACATCAAGGTTTCTTGATGAAGTGGATTTGGAGGTTGCCAGTCTAAC
TGGCTCCTCTTAAAGGAGTTAGCATATTTGATTATTGCTGTCTGGCGTAATAAGTT
TCGCTAGTTTGGCATTTTGAAGTGTGCTTCTAATCGTCTTCGGACAATTTTTTGAC
TCTGGCCTCAAATCAGGTAGGACTA
**SEQ ID NO:61**
   *Cryptococcus curvatus* CBS 2755 and *Lipomyces tetrasporus* CBS 7656 
CGCCCGTCGCTACTACCGATTGAATGGCTTAGTGAGATTTCCGGATTGGCGTTAG
GAAGCCGGCAACGGCATCCTTTGGCTGAGAAGCTACTCAAACTTGGTCATTTAGA
GGAAGTAAAAGTCGTAACAAGGTTTCCGTAGGTGAACCTGCGGAAGGATCATTA
GTGATTTGCCTTCGGGCTAACTATATCCATAACACCTGTGAACTGTTGATTGACTT
CGGTCAATATTTTTACAAACATTGTGTAATGAACGTCATGTTATAATAACAAATA
TAACTTTCAACAACGGATCTCTTGGCTCTCGCATCGATGAAGAACGCAGCGAAAT
GCGATAAGTAATGTGAATTGCAGAATTCAGTGAATCATCGAATCTTTGAACGCAA
CTTGCGCTCTCTGGTATTCCGGAGAGCATGCCTGTTTGAGTGTCATGAAATCTCA
ACCATTAGGGTTTCTTAATGGCTTGGATTTGGACGTTTGCCAGTCAAATGGCTCG
TCTTAAAAGAGTTAGTGAATTTAACATTTGTCTTCTGGCGTAATAAGTTTCGCTGG
 
GCTGATAGTGTGAAGTTTGCTTCTAATCGTCCGCAAGGACAATTCTTGAACTCTG
GCCTCAAATCAGGTAGGACTA
**SEQ ID NO:62**
   *Lipomyces starkeyi* CBS 7786 
CGCCCGTCGCTACTACCGATTGAATGGCTTAGTGAGGCCTTCGGACTGGCTCCAG
AAAATGGGAAACCATTATCAGGAGCTGGAAAGTTGGTCAAACTTGGTCATTTAG
AGGAAGTAAAAGTCGTAACAAGGTTTCCGTAGGTGAACCTGCGGAAGGATCATT
ACTGAGTATTTGTCTTTTCAAGACATCTCTCTATCCATAAACTCTTTTTTTAAAA
AGACATGATTTATAACAATTAGTCTGAATGATTATTTTTAAATCTTCAAAACTTTC
AACAACGGATCTCTTGGTTCTCGCATCGATGAAGAACGCAGCAAATTGCGATAA
GTAATGTGAATTGCAGGATTTTGTGAATCATCGAATTTTTGAACGCACATTGCAC
CTTCTGGTATTCCGGAGGGTATACCTGTTTGAGCGTCATTTATATACTCAAAACTT
ACGTTTTGGTGATGGGCACGTATCTGGCTTCTAAGTTAGATTTGCCTGAAATATA
GCGGTAGAGGTCGCTAGAAGCGATGCAAGTTAGCCAATGCTATTAAAGTTAATT
CGTTGGTGACGCATGTTGAGCTTTTGGTGAAGTCTTCCTTAATTATTGGAATTTTT
TTCTAATTTTGACCTCAAATCAGGCAGGAGTA
**SEQ ID NO:63**
   *Yarrowia lipolytica* CBS 6012 
CGCCCGTCGCTACTACCGATTGAATGGTTTAGTGAGACCTTGGGAGGGCGAGATG
AGGGGGGCAACCCCTTTTGAACATCCAAACTTGGTCAAACTTGATTATTTAGAGG
AAGTAAAAGTCGTAACAAGGTTTCCGTAGGTGAACCTGCGGAAGGATCATTATT
GATTTTATCTATTTCTGTGGATTTCTATTCTATTACAGCGTCATTTTATCTCAATTA
TAACTATCAACAACGGATCTCTTGGCTCTCACATCGATGAAGAACGCAGCGAACC
GCGATATTTTTTGTGACTTGCAGATGTGAATCATCAATCTTTGAACGCACATTGC
GCGGTATGGCATTCCGTACCGCACGGATGGAGGAGCGTGTTCCCTCTGGGATCGC
ATTGCTTTCTTGAAATGGATTTTTTTAAACTCTCAATTATTACGTCATTTCACCTCC
TTCATCCGAGATTA
**SEQ ID NO:64**
   *Trichosporon loubieri var. loubieri* CBS 8265 
CGCCCGTCGCTACTACCGATTGAATGGCTTAGTGAGACCTCCGGATTGGCGTTGA
GAAGCCGGCAACGGCATCTCTTGGCCGAGAAGTTGGTCAAACTTGGTCATTTAGA
GGAAGTAAAAGTCGTAACAAGGTTTCCGTAGGTGAACCTGCGGAAGGATCATTA
GTGATTGCCATCTTGGCTTAAACTATATCCATCTACACCTGTGAACCGTTTGATTG
AATCTTCTGATTCAATTTACAAACATTGTGTAATGAACGTCATTAGATCATAAT
AAGAAAAACTTTCAACAACGGATCTCTTGGCTCTCGCATCGATGAAGAACGCA
GCGAAATGCGATAAGTAATGTGAATTGCAGAATTCAGTGAATCATCGAATCTTTG
AACGCAACTTGCGCTCTCTGGTATTCCGGAGAGCATGCCTGTTTGAGTGTCATGA
AATCTCAACCATTAGGGTTTCTTAATGGCTTGGATTTGGAGGTTGCCATTCTAAAT
GGCTCCTCTTAAAAGAGTTAGCGAGTTTAACTATTGCTATCTGGCGTAATAAGTT
TCGCTGGAATGGTATTGTGAAGCGCGCTTCTAATCGTCTTCGGACAATTTTTTGAC
TCTGGCCTCAAATCAGGTAGGACTA
**SEQ ID NO:65**
   *Geotrichum vulgare* CBS 10073 
CGCCCGTCGCTACTACCGATTGAATGGCTTAGTGAGGCTTCCGGATTGATTAGTT
GGAGAGGGAGACTTTCTGACTGAACGAGAAGCTAGTCAAACTTGGTCATTTAG
AGGAAGTAAAAGTCGTAACAAGGTTTCCGTAGGTGAACCTGCGGAAGGATCATT
AAAGATTTAATATTAATTGTGAAATTAAAACGATATTAACAAAAAATCATACAAT
CAATTATAAAAAAAATCAAAACTTTTAACAATGGATCTCTTGGTTCTCGTATCGA
TGAAGAACGCAGCGAAACGCGATATTTCTTGTGAATTGCAGAAGTGAATCATCA
GTTTTTGAACGCACATTGCACTTTGGGGTATCCCCCAAAGTATACTTGTTTGAGC
GTTGTTTCTCTCTTGGAATTGCATTGCTTTTCTAAAAAATCGAATCAAATTCGTTT
GAAACATCCATTCTTCAACCTCAGATCAAGTAGGATTA
**SEQ ID NO:66**
   *Rhodosporidium toruloides* CBS 14 
CGCCCGTCGCTACTACCGATTGAATGGCTTAGTGAGGCCTCCGGATTGGCTATTG
GGAGCTCGCGAGAGCACCTGACTGCCGAGAAGTTGTACGAACTTGGTCATTTAG
AGGAAGTAAAAGTCGTAACAAGGTTTCCGTAGGTGAACCTGCGGAAGGATCATT
AGTGAATATTAGGGTGTCCAACTTAACTTGGAGCCCGACCCTCACTTTCTAACCC
TGTGCATTTGTCTTGGGTAGTAGCTTGCGTCAGCGAGCGAATCCCATTTCACTTAC
AAACACAAAGTCTATGAATGTAACAAATTTATAACAAAACAAAACTTTCAACAA
CGGATCTCTTGGCTCTCGCATCGATGAAGAACGCAGCGAAATGCGATACGTAAT
GTGAATTGCAGAATTCAGTGAATCATCGAATCTTTGAACGCACCTTGCGCTCCAT
GGTATTCCGTGGAGCATGCCTGTTTGAGTGTCATGAATTCTTCAACCCACCTCTTT
CTTAGTGAATCAGGCGGTGTTTGGATTCTGAGCGCTGCTGGCTTCGCGGCCTAGC
TCGCTCGTAATGCATTAGCATCCGCAATCGAACTTCGGATTGACTCGGCGTAATA
GACTATTCGCTGAGGATTCTGGTCTCTGACTGGAGCCGGGTAAGGTTAAAGGGA
GCTACTAATCCTCATGTCTATCTTGAGATTAGACCTCAAATCAGGTAGGACTA
**SEQ ID NO:67**
   *Rhodotorula glutinis var. glutinis* CBS 6020 and *Lipomyces orientalis* CBS 10300 
CGCCCGTCGCTACTACCGATTGAATGGCTTAGTGAGGCCTCCGGATTGGCTATTG
GGAGCTCGCGAGAGCACCTGACTGCTGAGAAGTTGTACGAACTTGGTCATTTAG
AGGAAGTAAAAGTCGTAACAAGGTTTCCGTAGGTGAACCTGCGGAAGGATCATT
AGTGAATCTAGGACGTCCAACTTAACTTGGAGTCCGAACTCTCACTTTCTAACCC
TGTGCATCTGTTAATTGGAATAGTAGCTCTTCGGAGTGAACCACCATTCACTTAT
AAAACACAAAGTCTATGAATGTATACAAATTTATAACAAAACAAAACTTTCAAC
AACGGATCTCTTGGCTCTCGCATCGATGAAGAACGCAGCGAAATGCGATACGTA
ATGTGAATTGCAGAATTCAGTGAATCATCGAATCTTTGAACGCACCTTGCGCTCC
TTGGTATTCCGAGGAGCATGCCTGTTTGAGTGTCATGAAATCTTCAACCCACCTC
TTTCTTAGTGAATCTGGTGGTGCTTGGTTTCTGAGCGCTGCTCTGCTTCGGCTTAG
CTCGTTCGTAATGCATTAGCATCCGCAACCGAACTTCGGATTGACTTGGCGTAAT
AGACTATTCGCTGAGGATTCTAGTTTACTAGAGCCGAGTTGGGTTAAAGGAAGCT
CCTAATCCTAAAGTCTATTTTTGATTAGATCTCAAATCAGGTAGGACTA
**SEQ ID NO:68**
   *Rhodotorula aurantiaca* CBS 317 
CGCCCGTCGCTACTACCGATTGAATGGCTTAGTGAGATTTCCGGATTGGCGTTAG
GAAGCCGGCAACGGCATCCTTTGGCTGAGAAGCTACTCAAACTTGGTCATTTAAA
 
GGAAGTAAAAGTCGTAACAAGGTTTCCGTAGGTGAACCTGCGGAAGGATCATTA
GTGATTTGCCTTCGGGCTAACTATATCCATAACACCTGTGAACTGTTGATTGACTT
CGGTCAATATTTTTACAAACATTGTGTAATGAACGTCATGTTATAATAACAAATA
TAACTTTCAACAACGGATCTCTTGGCTCTCGCATCGATGAAGAACGCAGCGAAAT
GCGATAAGTAATGTGAATTGCAGAATTCAGTGAATCATCGAATCTTTGAACGCAA
CTTGCGCTCTCTGGTATTCCGGAGAGCATGCCTGTTTGAGTGTCATGAAATCTCA
ACCATTAGGGTTTCTTAATGGCTTGGATTTGGACGTTTGCCAGTCAAATGGCTCG
TCTTAAAAGAGTTAGTGAATTTAACATTTGTCTTCTGGCGTAATAAGTTTCGCTGG
GCTGATAGTGTGAAGTTTGCTTCTAATCGTCCGCAAGGACAATTCTTGAACTCTG
GCCTCAAATCAGGTAGGACTA
**SEQ ID NO:69**
   *Torulaspora delbrueckii* CBS 2924

CGCCCGTCGCTAGTACCGATTGAATGGCTTAGTGAGGCCTCAGGATCTGCTTAGA
GAAGGGGGCAACTCCATCTCAGAGCGGAGAATCTGGTCAAACTTGGTCATTTAG
AGGAACTAAAAGTCGTAACAAGGTTTCCGTAGGTGAACCTGCGGAAGGATCATT
AGAGAAATCTATATGAATGAAGTTAGAGGACGTCTAAAGATACTGTAAGAGAGG
ATCAGGTTCAAGACCAGCGCTTAATTGCGCGGTTGCGGCTTGGTTCGCCTTTTGC
GGAACATGTCTTTTCTCGTTGTTAACTCTACTTCAACTTCTACAACACTGTGGAGT
TTTCTACACAACTTTTCTTCTTTGGGAAGATACGTCTTGTGCGTGCTTCCCAGAGG
TGACAAACACAAACAACTTTTTATTATTATAAACCAGTCAAAACCAATTTCGTTA
TGAAATTAAAAATATTTAAAACTTTCAACAACGGATCTCTTGGTTCTCGCATCGA
TGAAGAACGCAGCGAAATGCGATACGTAATGTGAATTGCAGAATTCCGTGAATC
ATCGAATCTTTGAACGCACATTGCGCCCCTTGGTATTCCAGGGGGCATGCCTGTT
TGAGCGTCATTTCCTTCTCAAACAATCATGTTTGGTAGTGAGTGATACTCTGTCAA
GGGTTAACTTGAAATTGCTAGCCTGTTATTTGGTTGTGATTTTGCTGGCTTGGATG
ACTTTGTCCAGTCTAGCTAATACCGAATTGTCGTATTAGGTTTTACCAACTTCGGC
AGACTGTGTGTTGGCTCGGGCGCTTTAAAGACTTTGTCGTAAACGATTTATCGTTT
GTTTGAGCTTTTCGCATACGCAATCCGGGCGAACAATACTCTCAAAGTTTGACCT
CAAATCAGGTAGGAATA

## Claims

1. A method for extracting lipids from microbial biomass comprising
a. drying the microbial biomass to produce dried microbial biomass having a moisture content of from 3% to 15%, less than 6%, less than 5%, less than 4%, from 0.1% to 5%, from 0.1% to 3%, or from 0.5% to 3.5% by weight and constituting at least 20% oil by weight;
b. conditioning the dried microbial biomass to produce conditioned feedstock by heating the dry microbial biomass to a temperature in the range of 70°C to 150 °C, the conditioned feedstock having a moisture content of from 0.5% to 2.5% by weight of the microbial biomass; and
c. subjecting the conditioned feedstock to pressure sufficient to separate at least 5% of the oil in the biomass from other components,
leaving spent biomass of reduced oil content relative to the conditioned feedstock.

2. The method of claim 1, wherein a bulking agent is added to the dried microbial biomass prior to conditioning the dried microbial biomass.

3. The method of any one of the preceding claims, wherein the lipids are extracted by an expeller press.

4. The method of any one of the preceding claims, wherein the spent biomass contains a range of from less than 50% oil to less than 1% oil by weight, and wherein the oil content in the spent biomass is less than the oil content in the microbial biomass.

5. The method of claim 3, wherein the microbial biomass is subjected to one or more cycles of oscillating pressure, wherein each cycle consists of a lower pressure for a first period of time followed by a higher pressure for a second period of time, wherein, if more than one cycle is conducted, then the average pressure exerted on the biomass during the course of the last cycle is at least 2 fold higher than the average pressure exerted on the biomass during the course of the first or any earlier cycle.

6. The method of claim 3 or claim 5, wherein the expeller press is a continuously rotating worm shaft within a cage having a feeder at one end and a choke at an end opposite thereof, and having openings within the cage,
wherein the cage has an internal length that is between at least ten times to at least 20 times its internal diameter and comprises a plurality of elongated bars with at least some of the elongated bars separated by one or more spacers, the bars resting on a frame, wherein the one or more spacers between the bars form the openings, and oils are released through the openings to a collecting vessel fluidly coupled with the cage,
wherein the spacers between the elongated bars are of different thicknesses thereby allowing variation of the space between each elongated bar, wherein either the spacers or the gaps between the bars are from 0.13 mm to 0.76 mm (0.005 to 0.030 inches) thick,
wherein the biomass enters the cage through the feeder, and rotation of the worm shaft advances the biomass along the cage and applies pressure to the biomass disposed between the cage and the choke, the pressure lysing cells of the biomass and releasing oil through the openings of the cage, and spent biomass of reduced oil content is extruded from the choke end of the cage.

7. The method of claim 6, wherein the pressure increases by a factor of between 10 and 20 from the feeder end to the choke end of the cage and does not increase by more than 100% of the pressure at the feeder end of the cage per linear foot of the cage between the feeder and choke ends of the cage.

8. The method of any one of the preceding claims, further comprising extracting lipids from the spent biomass of reduced oil content with an organic solvent.

9. The method of claim 8, wherein the organic solvent is hexane.

10. The method of any one of the preceding claims, wherein the conditioned feedstock has a moisture content of between 0.5% and 2.0% by weight, optionally between 1.0% and 2.0% by weight, before subjecting the microbial biomass to pressure.

11. The method of any one of the preceding claims, wherein the conditioning step comprises heating the biomass for a period of time between 10 and 60 minutes.

12. The method of any one of the preceding claims, wherein a bulking agent is switchgrass, soybean hulls, dried rosemary, corn stover, cellulose, sugar cane bagasse, or spent microbial biomass that comprises between 40% and 90% polysaccharide and less than 10% oil, and is added to the microbial biomass prior to a step of dehydrating the microbial biomass to a moisture content of less than 6%.

13. The method of any one of the preceding claims, wherein the microbial biomass is selected from the group consisting of microalgal biomass, bacterial biomass, oleaginous yeast biomass, and non-yeast oleaginous fungal biomass and is derived from a culture that was cultivated through a process selected from the group consisting of a heterotrophic process, a photoautotrophic process, and a mixotrophic process.

14. The method of any one of the preceding claims, wherein the microbial biomass is microalgal biomass derived from a microalgae having a fatty acid profile of:
less than 2% C14:0; about 13-16% C16:0; about 1-4% C18:0; about 64-71% C18:1; about 10-15% C18:2; about 0.5-2% C18:3; and less than 1% carbon chain length 20 or longer;
about 1-2% C14:0; about 16-26% C16:0; about 2-6% C18:0; about 58-68% C18:1; and about 7-11% C18:2;
at least 4% C8-C14 , wherein the microalgae contains an exogenous gene encoding a thioesterase with a preference for one or more fatty acid chain lengths of 8, 10, 12 and 14 carbon atoms;
between 10 and 40% C8-C14; or
at least 15% C:16 fatty acids, at least 50% C18:1 fatty acids, at least 7% C18:2 fatty acids, and less than 3% C10:0-C14:0 fatty acids.

15. The method of any one of the preceding claims, wherein the extracted lipid has one or more properties selected from:
less than 0.01 milligram of chlorophyll per kilogram of lipid; or
one or more of the following: no more than 8 ppm chloride, no more than 2 ppm phosphorus, no more than 26 ppm potassium, no more than 12 ppm sodium, and no more than 5 ppm sulfur.

16. The method of any one of the preceding claims, wherein the microbial biomass is of the microalgal genus *Chlorella, Parachlorella* or *Prototheca,* optionally *Chlorella* or *Prototheca.*

17. The method of claim 16, wherein the microalgal biomass is of the species *Prototheca moriformis* or *Chlorella protothecoides.*

## Patentansprüche

1. Verfahren zum Extrahieren von Lipiden aus mikrobieller Biomasse, umfassend:
a. Trocknen der mikrobiellen Biomasse, so dass eine getrocknete mikrobielle Biomasse produziert wird, die einen Feuchtigkeitsgehalt von 3% bis 15%, weniger als 6%, weniger als 5%, weniger als 4%, von 0,1% bis 5%, von 0,1% bis 3% oder von 0,5% bis 3,5%, bezogen auf das Gewicht, aufweist, und mindestens 20 Gew.-% Öl ausmacht;
b. Aufbereiten der getrockneten Biomasse, so dass ein aufbereitetes Ausgangsmaterial produziert wird, durch Erwärmen der getrockneten mikrobiellen Biomasse auf eine Temperatur im Bereich von 70°C bis 150°C, wobei das aufbereitete Ausgangsmaterial einen Feuchtigkeitsgehalt von 0,5% bis 2,5% bezogen auf das Gewicht der mikrobiellen Biomasse aufweist; und
c. Unterwerfen des aufbereiteten Ausgangsmaterials einem Druck, der hinreicht, dass mindestens 5% des Öls in der Biomasse von anderen Komponenten getrennt werden,
so dass verbrauchte Biomasse mit reduziertem Ölgehalt im Vergleich zu dem aufbereiteten Ausgangsmaterial zurückbleibt.

2. Verfahren nach Anspruch 1, wobei vor dem Aufbereiten der getrockneten mikrobiellen Biomasse ein Füllstoff zu der getrockneten mikrobiellen Biomasse gegeben wird.

3. Verfahren nach einem der vorhergehenden Ansprüche, wobei die Lipide durch eine Expellerpresse extrahiert werden.

4. Verfahren nach einem der vorhergehenden Ansprüche, wobei die verbrauchte Biomasse einen Bereich von weniger als 50% Öl bis weniger als 1 Gew.-% Öl enthält, und wobei der Ölgehalt in der verbrauchten Biomasse geringer ist als der Ölgehalt in der mikrobiellen Biomasse.

5. Verfahren nach Anspruch 3, wobei die mikrobielle Biomasse einem oder mehr Zyklen eines oszillierenden Drucks ausgesetzt wird, wobei jeder Zyklus aus einem niedrigeren Druck für einen ersten Zeitraum besteht, gefolgt von einem höheren Druck für einen zweiten Zeitraum, wobei bei der Durchführung von mehr als einem Zyklus der Durchschnittsdruck, der während des Verlaufs des letzten Zyklus auf die Biomasse ausgeübt wird, mindestens doppelt so hoch ist wie der Durchschnittsdruck, der während des Verlaufs des ersten oder jedweden früheren Zyklus auf die Biomasse ausgeübt wird.

6. Verfahren nach Anspruch 3 oder Anspruch 5, wobei die Expellerpresse eine kontinuierlich rotierende Schneckenwelle innerhalb eines Käfigs ist, der an einem Ende eine Einspeisungseinrichtung und eine Drossel an seinem gegenüberliegenden Ende aufweist und Öffnungen innerhalb des Käfigs aufweist,
wobei der Käfig eine innere Länge hat, die zwischen dem mindestens zehnfachen und mindestens dem zwanzigfachen seines Innendurchmessers liegt und eine Vielzahl länglicher Stäbe umfasst, wobei zumindest einige der länglichen Stäbe durch mindestens einen Abstandshalter voneinander getrennt sind, wobei die Stäbe auf einem Gerüst ruhen, wobei der mindestens eine Abstandshalter zwischen den Stäben Öffnungen bildet und Öle durch die Öffnungen in einen Sammelbehälter entlassen werden, der in Fluidverbindung mit dem Käfig verbunden ist,
wobei die Abstandshalter zwischen den länglichen Stäben verschiedene Dicken aufweisen, die eine Veränderung des Raums zwischen jedem länglichen Stab ermöglichen, wobei entweder die Abstandshalter oder die Lücken zwischen den Stäben 0,13 mm bis 0,76 mm (0,005 bis 0,030 Zoll) dick sind,
wobei die Biomasse durch die Einspeisungseinrichtung in den Käfig eintritt, und die Rotation der Schneckenwelle die Biomasse den Käfig entlang vorbewegt und auf die zwischen dem Käfig und der Drossel angeordnete Biomasse Druck ausübt, wobei der Druck Zellen der Biomasse lysiert und Öl durch die Öffnungen des Käfigs freisetzt und verbrauchte Biomasse mit reduziertem Ölgehalt aus dem Drosselende des Käfigs extrudiert wird.

7. Verfahren nach Anspruch 6, wobei der Druck um einen Faktor zwischen 10 und 20 vom Einspeisungsende bis zum Drosselende des Käfigs ansteigt und nicht um mehr als 100% des Drucks am Einspeisungsende des Käfigs pro linearem Fuß des Käfigs zwischen dem Einspeisungs- und Drosselende des Käfigs ansteigt.

8. Verfahren nach einem der vorhergehenden Ansprüche, zudem das Extrahieren von Lipiden aus der verbrauchten Biomasse mit reduziertem Ölgehalt mit einem organischen Lösungsmittel.

9. Verfahren nach Anspruch 8, wobei das organische Lösungsmittel Hexan ist.

10. Verfahren nach einem der vorhergehenden Ansprüche, wobei das aufbereitete Ausgangsmaterial einen Feuchtigkeitsgehalt zwischen 0,5% und 2,0 Gew.-%, gegebenenfalls zwischen 1,0% und 2,0 Gew.-% aufweist, bevor die mikrobielle Biomasse einem Druck ausgesetzt wird.

11. Verfahren nach einem der vorhergehenden Ansprüche, wobei der Aufbereitungsschritt das Erwärmen der Biomasse für einen Zeitraum zwischen 10 und 60 Minuten umfasst.

12. Verfahren nach einem der vorhergehenden Ansprüche, wobei es sich bei dem Füllstoff um Rutenhirse, Sojabohnenhülsen, getrockneten Rosmarin, Maisstroh, Zellulose, Zuckerrohrbagasse oder verbrauchte Biomasse handelt, die zwischen 40% und 90% Polysaccharid und weniger als 10% Öl umfasst, und zu dem die mikrobielle Biomasse vor dem Schritt zugefügt wird, bei dem die mikrobielle Biomasse auf einen Feuchtigkeitsgehalt von weniger als 6% dehydratisiert wird.

13. Verfahren nach einem der vorhergehenden Ansprüche, wobei die mikrobielle Biomasse ausgewählt ist aus der Gruppe, bestehend aus Mikroalgen-Biomasse, Bakterien-Biomasse, öliger Hefe-Biomasse, öliger Nicht-Hefe-Pilz-Biomasse und aus einer Kultur stammt, die durch ein Verfahren kultiviert wurde, das ausgewählt ist aus der Gruppe, bestehend aus einem heterotrophen Verfahren, einem photoautotrophen Verfahren und einem mixotrophen Verfahren.

14. Verfahren nach einem der vorhergehenden Ansprüche, wobei die Mikroalgen-Biomasse eine Mikroalgen-Biomasse ist, die von Mikroalgen mit einem Fettsäureprofil von:
weniger als 2% C14:0; etwa 13-16% C16:0; etwa 1-4% C18:0; etwa 64-71% C18:1; etwa 10-15% C18:2; etwa 0,5-2% C18:3; und weniger als 1% Kohlenstoffkettenlänge 20 oder mehr;
etwa 1-2% C14:0; etwa 16-26% C16:0; etwa 2-6% C18:0; etwa 58-68% C18:1; und etwa 7 bis 11% C18:2;
mindestens 4% C8-C14, wobei die Mikroalge ein exogenes Gen enthält, das eine Thioesterase mit einer Präferenz für eine oder mehrere Fettsäurekettenlängen von 8, 10, 12 und 14 Kohlenstoffatomen codiert;
zwischen 10 und 40% C8-C14; oder
mindestens 15% C:16-Fettsäuren, mindestens 50% C18:1-Fettsäuren, mindestens 7% C18:2-Fettsäuren und weniger als 3% C10:0-C14:0-Fettsäuren, stammt.

15. Verfahren nach einem der vorhergehenden Ansprüche, wobei das extrahierte Lipid eine oder mehr Eigenschaften aufweist, ausgewählt aus:
weniger als 0,01 Milligramm Chlorophyll pro Kilogramm Lipid; oder
eine oder mehr der Folgenden: nicht mehr als 8 ppm Chlorid, nicht mehr als 2 ppm Phosphor, nicht mehr als 26 ppm Kalium, nicht mehr als 12 ppm Natrium und nicht mehr als 5 ppm Schwefel.

16. Verfahren nach einem der vorhergehenden Ansprüche, wobei die mikrobielle Biomasse von der Mikroalgengattung *Chlorella, Parachlorella* oder *Prototheca,* gegebenenfalls *Chlorella* oder *Prototheca* stammt.

17. Verfahren nach Anspruch 16, wobei die Mikroalgen-Biomasse von der Art *Prototheca moriformis* oder *Chlorella protothecoides* stammt.

## Revendications

1. Procédé d'extraction de lipides à partir d'une biomasse microbienne comprenant
a. le séchage de la biomasse microbienne pour produire une biomasse microbienne séchée ayant une teneur en humidité de 3 % à 15 %, inférieure à 6 %, inférieure à 5 %, inférieure à 4 %, de 0,1 % à 5 %, de 0,1 % à 3 %, ou de 0,5 % à 3,5 % en poids et constituant au moins 20 % d'huile en poids ;
b. le conditionnement de la biomasse microbienne séchée pour produire une matière première conditionnée par chauffage de la biomasse microbienne sèche à une température dans la plage de 70 °C à 150 °C, la matière première conditionnée ayant une teneur en humidité de 0,5 % à 2,5 % en poids de la biomasse microbienne ; et
c. la soumission de la matière première conditionnée à une pression suffisante pour séparer au moins 5 % de l'huile dans la biomasse des autres composants,
en laissant une biomasse épuisée ayant une teneur en huile réduite par rapport à la matière première conditionnée.

2. Procédé selon la revendication 1, dans lequel un agent diluant est ajouté à la biomasse microbienne séchée avant de conditionner la biomasse microbienne séchée.

3. Procédé selon l'une quelconque des revendications précédentes, dans lequel les lipides sont extraits par une presse d'extraction.

4. Procédé selon l'une quelconque des revendications précédentes, dans lequel la biomasse épuisée contient une plage de moins de 50 % d'huile à moins de 1 % d'huile en poids, et dans lequel la teneur en huile dans la biomasse épuisée est inférieure à la teneur en huile dans la biomasse microbienne.

5. Procédé selon la revendication 3, dans lequel la biomasse microbienne est soumise à un ou plusieurs cycles de pression oscillante, dans lequel chaque cycle est constitué d'une basse pression pendant une première durée suivie d'une haute pression pendant une deuxième durée, dans lequel, si plus d'un cycle est conduit, la pression moyenne exercée sur la biomasse au cours du dernier cycle est au moins 2 fois plus élevée que la pression moyenne exercée sur la biomasse au cours du premier cycle ou de tout cycle précédent.

6. Procédé selon la revendication 3 ou la revendication 5, dans lequel la presse d'extraction est un arbre à vis sans fin tournant en continu dans une cage comportant une trémie à une extrémité et un étranglement à une extrémité opposée de celle-ci, et comportant des ouvertures dans la cage,
dans lequel la cage a une longueur interne qui est entre au moins dix fois et au moins 20 fois son diamètre interne et comprend une pluralité de barres allongées avec au moins une partie des barres allongées séparées par un ou plusieurs éléments d'espacement, les barres reposant sur un cadre, dans lequel les un ou plusieurs éléments d'espacement entre les barres forment les ouvertures, et les huiles sont libérées à travers les ouvertures vers un récipient de collecte en raccordement fluidique avec la cage,
dans lequel les éléments d'espacement entre les barres allongées sont de différentes épaisseurs de façon à permettre la variation de l'espace entre chaque barre allongée, dans lequel les éléments d'espacement ou les écartements entre les barres sont de 0,13 mm à 0,76 mm (0,005 à 0,030 pouce) d'épaisseur,
dans lequel la biomasse pénètre dans la cage à travers la trémie, et la rotation de l'arbre à vis sans fin fait avancer la biomasse le long de la cage et applique une pression à la biomasse disposée entre la cage et l'étranglement, la pression causant la lyse des cellules de la biomasse et libérant l'huile à travers les ouvertures de la cage, et la biomasse épuisée ayant une teneur en huile réduite est extrudée à partir de l'extrémité d'étranglement de la cage.

7. Procédé selon la revendication 6, dans lequel la pression augmente d'un facteur compris entre 10 et 20 de l'extrémité de trémie à l'extrémité d'étranglement de la cage et n'augmente pas de plus de 100 % de la pression à l'extrémité de trémie de la cage par pied linéaire de la cage entre les extrémités de trémie et d'étranglement de la cage.

8. Procédé selon l'une quelconque des revendications précédentes, comprenant en outre l'extraction de lipides à partir de la biomasse épuisée ayant une teneur en huile réduite avec un solvant organique.

9. Procédé selon la revendication 8, dans lequel le solvant organique est l'hexane.

10. Procédé selon l'une quelconque des revendications précédentes, dans lequel la matière première conditionnée a une teneur en humidité comprise entre 0,5 % et 2,0 % en poids, facultativement entre 1,0 % et 2,0 % en poids, avant de soumettre la biomasse microbienne à une pression.

11. Procédé selon l'une quelconque des revendications précédentes, dans lequel l'étape de conditionnement comprend le chauffage de la biomasse pendant une durée comprise entre 10 et 60 minutes.

12. Procédé selon l'une quelconque des revendications précédentes, dans lequel un agent diluant est du panic érigé, des coques de soja, du romarin séché, des tiges de maïs, de la cellulose, de la bagasse de canne à sucre ou une biomasse microbienne épuisée qui comprend entre 40 % et 90 % de polysaccharide et moins de 10 % d'huile, et est ajouté à la biomasse microbienne avant une étape de déshydratation de la biomasse microbienne à une teneur en humidité inférieure à 6 %.

13. Procédé selon l'une quelconque des revendications précédentes, dans lequel la biomasse microbienne est choisie dans le groupe constitué de biomasse de microalgue, biomasse bactérienne, biomasse de levure oléagineuse et biomasse fongique oléagineuse non-levure et est dérivée d'une culture qui a été cultivée par un procédé choisi dans le groupe constitué d'un procédé hétérotrophe, un procédé photo-autotrophe et un procédé mixotrophe.

14. Procédé selon l'une quelconque des revendications précédentes, dans lequel la biomasse microbienne est une biomasse de microalgue dérivée d'une microalgue ayant un profil d'acides gras de :
moins de 2 % de C14:0 ; environ 13 à 16 % de C16:0 ; environ 1 à 4 % de C18:0 ; environ 64 à 71 % de C18:1 ; environ 10 à 15 % de C18:2 ; environ 0,5 à 2 % de C18:3 ; et moins de 1 % d'une longueur de chaine de carbone de 20 ou plus ;
environ 1 à 2 % de C14:0 ; environ 16 à 26 % de C16:0 ; environ 2 à 6 % de C18:0 ; environ 58 à 68 % de C18:1 ; et environ 7 à 11 % de C18:2 ;
au moins 4 % de C8-C14, dans lequel la microalgue contient un gène exogène codant pour une thioestérase avec une préférence pour une ou plusieurs longueurs de chaîne d'acide gras de 8, 10, 12 et 14 atomes de carbone ;
entre 10 et 40 % de C8-C14 ; ou
au moins 15 % d'acides gras C:16, au moins 50 % d'acides gras C18:1, au moins 7 % d'acides gras C18:2, et moins de 3 % d'acides gras C10:0-C14:0.

15. Procédé selon l'une quelconque des revendications précédentes, dans lequel le lipide extrait possède une ou plusieurs propriétés choisies parmi :
moins de 0,01 milligramme de chlorophylle par kilogramme de lipide ; ou
un ou plusieurs des suivants : pas plus de 8 ppm de chlorure, pas plus de 2 ppm de phosphore, pas plus de 26 ppm de potassium, pas plus de 12 ppm de sodium, et pas plus de 5 ppm de soufre.

16. Procédé selon l'une quelconque des revendications précédentes, dans lequel la biomasse microbienne est du genre de microalgue *Chlorella, Parachlorella* ou *Prototheca,* facultativement *Chlorella* ou *Prototheca.*

17. Procédé selon la revendication 16, dans lequel la biomasse de microalgues est de l'espèce *Prototheca moriformis* ou *Chlorella protothecoides.*
